(19) Europäisches Patentamt
European Patent Office
Office européen des brevets

(11) Numéro de publication: **0 414 607 B1**

(12) **FASCICULE DE BREVET EUROPEEN**

(45) Date de publication de fascicule du brevet: **22.03.95**

(51) Int. Cl.[6]: **C07H 21/00**, C12N 15/28, C12N 15/11, A61K 31/70

(21) Numéro de dépôt: **90402329.8**

(22) Date de dépôt: **22.08.90**

(54) **Séquence d'oligonucléotides anti-sens, anti-ARN messager du TNF alpha, procédé de préparation, application à titre de médicaments et compositions pharmaceutiques.**

(30) Priorité: **23.08.89 FR 8911171**

(43) Date de publication de la demande:
**27.02.91 Bulletin 91/09**

(45) Mention de la délivrance du brevet:
**22.03.95 Bulletin 95/12**

(84) Etats contractants désignés:
**AT BE CH DE DK ES FR GB GR IT LI LU NL SE**

(56) Documents cités:
**EP-A- 0 090 789**
**WO-A-86/04606**
**WO-A-88/06625**

**GENE AMPLIF. ANAL., vol. 3, 1983, pages 1-26; M.H. CARUTHERS et al.:"Deoxyoligonucleotide synthesis via the phosphoramidite method"**

**NUCLEIC ACIDS RESEARCH, vol. 13, no. 17, septembre 1985, pages 6361-6373,Oxford, GB; G.E. NEDWIN et al.: "Human lymphotoxin and tumor necrosis factorgenes: structure, homology and chromosomal localization"**

**G.ZON Pharmac.Res.5/9, 539 (1988)**

(73) Titulaire: **ROUSSEL-UCLAF**
**35, Boulevard des Invalides**
**F-75007 Paris (FR)**

(72) Inventeur: **Braham, Abdel Karim**
**3, Allée du Pr. Frühling**
**F-93200 Saint Denis (FR)**
Inventeur: **Smets, Pierre**
**137, Avenue du Maréchal Leclerc**
**F-78670 Villennes Sur Seine (FR)**
Inventeur: **Zalisz, René**
**20, Delattre de Tassigny**
**F-95180 Menucourt (FR)**

(74) Mandataire: **Jacobi, Markus Alexander et al**
**Roussel Uclaf**
**111, Route de Noisy**
**B.P. 9**
**F-93230 Romainville (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

La présente invention concerne de nouvelles séquences d'oligonucléotides anti-sens, anti-ARN messager du TNF alpha, le procédé de préparation, l'application à titre de médicaments de ces nouvelles séquences et les compositions pharmaceutiques les renfermant.

On sait que le TNF (Tumor Necrosis Factor) secreté par les macrophages est responsable de véritables désastres métaboliques que peuvent engendrer un choc endotoxinique infectieux. L'activité biologique du TNF est décrite par WO-86/04606 et par WO-88/06625. L'utilisation des oligonucléotides dans la chimiothérapie est enseignée par G. Zon (Pharmaceutical Res. 5/9, 539 (1988). En cherchant à bloquer la source de production de TNF la demanderesse a été amenée à rechercher des séquences d'oligonucléotides anti-sens, anti-ARN messager qui puissent ainsi bloquer la production du TNF.

En étudiant diverses séquences, la demanderesse a trouvé qu'une séquence particulière d'oligonucléotides présentait cette propriété.

L'invention a ainsi pour objet une nouvelle séquence d'oligonucléotides anti-sens, anti-ARN messager du TNF alpha, caractérisée en ce qu'elle possède la structure de formule (I) suivante :

```
    5'                       3'
(d) TCA TGG TGT CCT TTG CAG - X                  (I)
    1                        18
```

dans laquelle X représente un atome d'hydrogène, ou une séquence de 1 à 17 oligonucléotides, sous forme libre, sous forme alcoylée, sous forme sulfurée ou sous forme de dérivé de la poly L-lysine.

Dans la formule générale (I) et dans ce qui suit,

- par séquence de 1 à 17 oligonucléotides on désigne toute séquence renfermant l'adenine et la guanine (bases puriques), la cytosine et la thymine (bases pyrimidiques),
- par forme alcoylée, on entend les dérivés alcoylés présentant un groupement alcoyle sur le phosphate et notamment un groupement méthyle, éthyle ou propyle. Ces groupements peuvent être présents sur tout ou partie des groupements phosphates,
- par forme sulfurée, on entend les dérivés sulfurés sur le phosphate, notamment les thioates et les dithioates. Ces groupements peuvent être présents sur tout ou partie des groupements phosphates.

Parmi les produits de l'invention, on peut citer les séquences d'oligonucléotides répondant à la formule (I) ci-dessus, dans laquelle X représente un atome d'hydrogène ou tout ou partie de la séquence de formule ($I_A$) :

```
  5'                  3'
- CC TCA TGC TTT CAG TAG              (IA)
  19                  35
```

sous forme libre, sous forme alcoylée, sous forme sulfurée, ou sous forme de dérivé de la poly L-lysine.

Parmi ces derniers, on retient de préférence les séquences d'oligonucléotides répondant à la formule (I) ci-dessus dans laquelle X représente un atome d'hydrogène, une séquence de formule - CC ou la séquence de formule ($I_A$), sous forme libre, sous forme alcoylée, sous forme sulfurée ou sous forme de dérivé de la poly L-lysine et notamment les séquences suivantes :

5' 3'
(d) TCA TGG TGT CCT TTG CAG CC
1 20

5' 3'
(d) TCA TGG TGT CCT TTG CAG
1 18

5' 3'
(d) TCA TGG TGT CCT TTG CAG CC TCA TGC TTT CAG TAG
1 35

L'invention a aussi pour objet un procédé de préparation des séquences d'oligonucléotides telles que définies par la formule (I) ci-dessus, procédé caractérisé en ce que l'on immobilise sur un support S le premier nucléotide en 3' de la chaîne à synthétiser de formule :

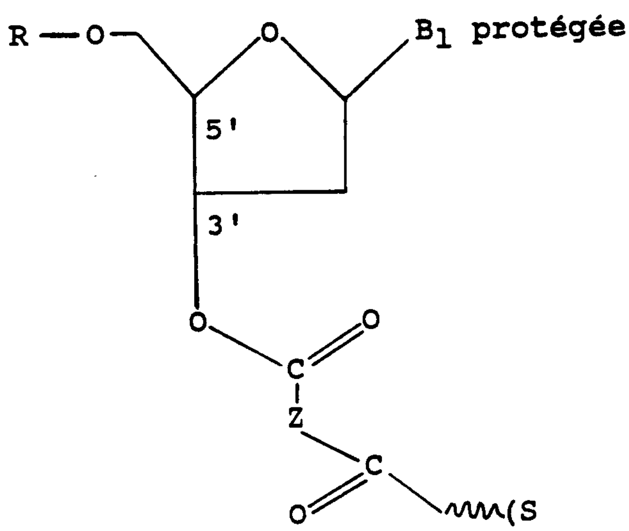

(II)

dans laquelle S représente le support, Z représente un groupement hydrocarboné renfermant de 2 à 20 atomes de carbone, $B_1$ est une base purique ou pyrimidique correspondant au premier nucléotide dont la fonction amine est protégée et R est un groupement protecteur,
débloque l'hydroxyle en 5′ par un réactif acide pour obtenir le produit de formule :

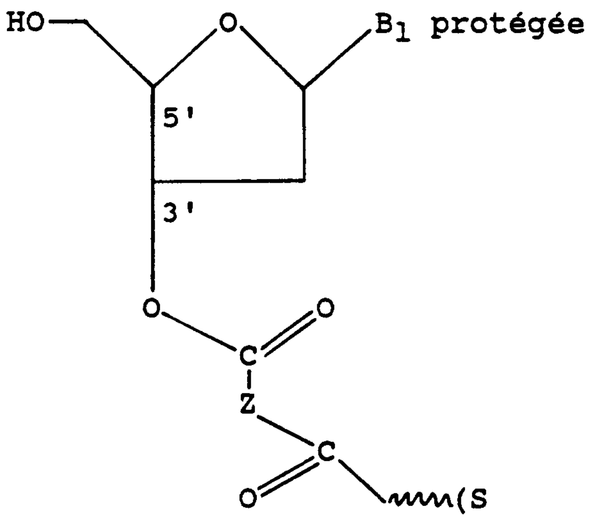

(III)

dans laquelle S, Z et $B_1$ ont la signification déjà indiquée, puis fait réagir ce dernier avec le monomère du second nucléotide de formule :

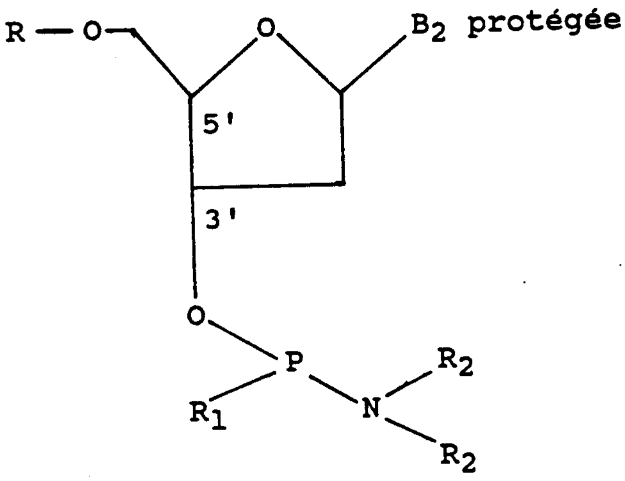

(IV)

dans laquelle R a la signification dejà indiquée, $B_2$ est une base purique ou pyrimidique correspondant au second nucléotide dont la fonction amine est protégée, $R_1$ représente un radical alcoyle ou un groupement $-OR'_1$ ou $-SR'_1$ dans lequel $R'_1$ représente un groupement protecteur, $R_2$ représente un groupement protecteur, pour obtenir un produit de formule :

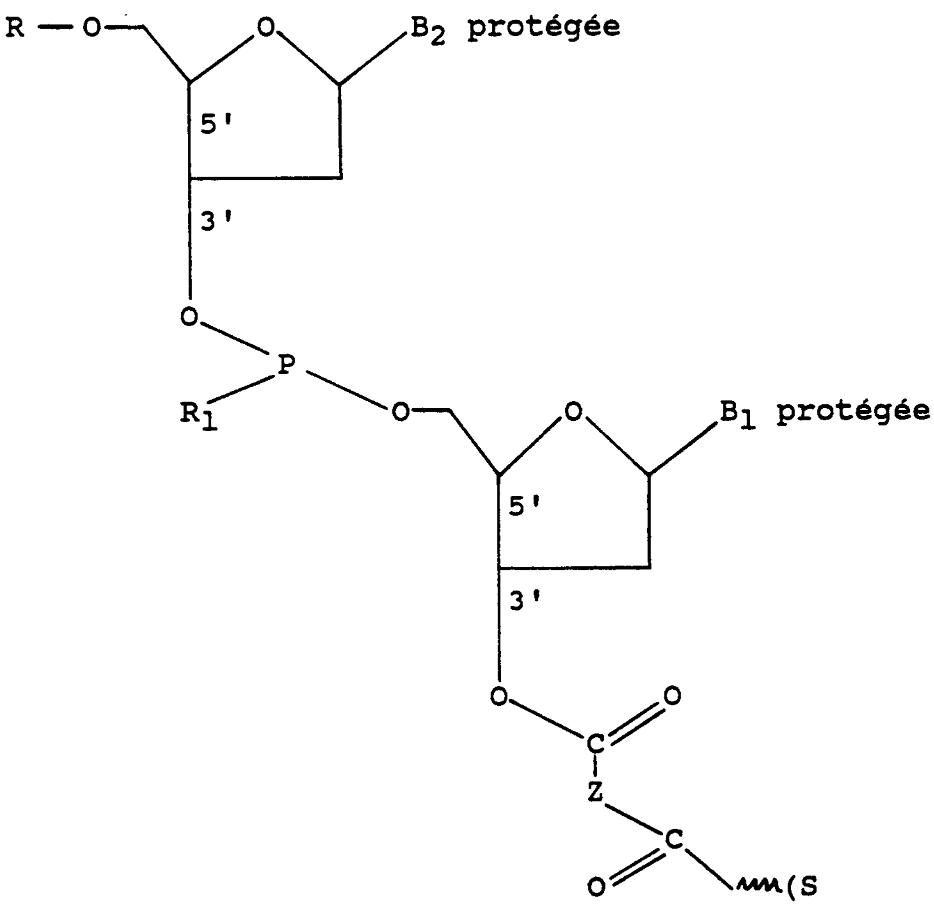

(V)

dans laquelle S, R, $R_1$, $B_1$ et $B_2$ ont la signification déjà indiquée, puis oxyde le produit de formule (V) obtenu pour obtenir un produit de formule :

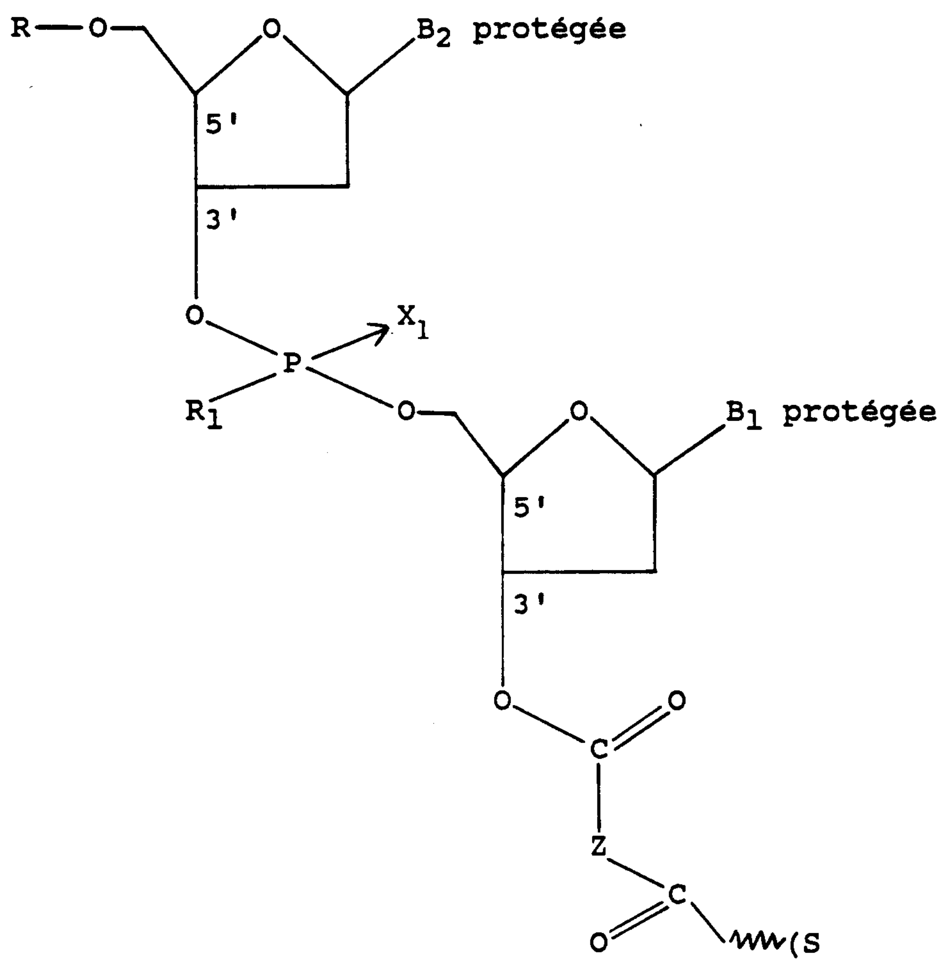

(VI)

dans laquelle S, R, $R_1$, $B_1$, $B_2$ et Z ont la signification déjà indiquée et $X_1$ représente un atome d'oxygène ou de soufre, puis, comme ci-dessus, au départ du produit de formule (II) effectue un nouveau cycle avec ce produit de formule (VI) et le monomère d'un nouveau nucléotide jusqu'à obtention de l'enchaînement désiré pour obtenir finalement un produit de formule :

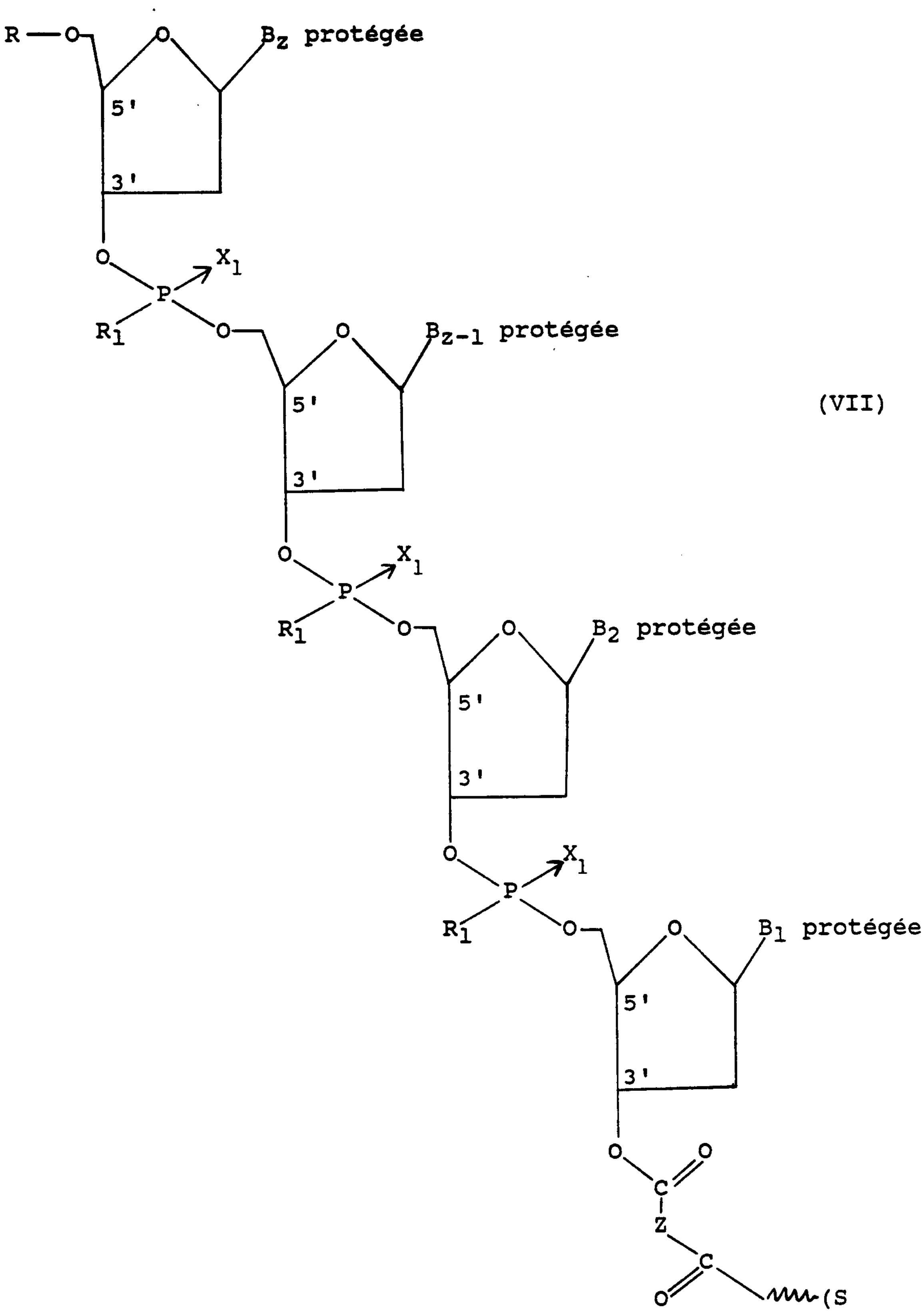

(VII)

dans laquelle S, R, $R_1$, $B_1$, $B_2$, $X_1$ et Z ont la signification déjà indiquée et $B_z$ représente le dernier nucléotide de la séquence, puis déprotège l'oligonucléotide, le sépare du support et purifie le produit de formule (I) ainsi obtenu.

Dans des conditions préférentielles de mise en oeuvre de l'invention, le procédé de préparation ci-dessus décrit, est caractérisé en ce que :

- le déblocage de l'hydroxyle en 5′ du produit de formule (II) est effectué par un réactif acide tel que l'acide acétique, l'acide di ou trichloracétique,
- la réaction de couplage du produit de formule (III) avec le produit de formule (IV) est activée au moyen du tétrazole en opérant dans l'acétonitrile,
- l'oxydation du phosphite de formule (V) en phosphate de formule (VI) est effectuée au moyen de l'iode en solution dans un mélange eau/lutidine/tétrahydrofuranne ou dans un mélange eau/pyridine/tétrahydrofuranne,
- l'oxydation du phosphite de formule (V) pour obtenir la forme sulfurée (VI) est effectuée au moyen du soufre en solution dans un mélange sulfure de carbone, de pyridine et de triéthylamine anhydre,
- en fin de synthèse la déprotection du groupement hydroxyle terminal en 5′ est effectuée au moyen d'un acide tel que l'acide acétique, l'acide di ou trichloracétique,
- la déprotection de l'oligonucléotide de formule (VII) est effectuée au moyen de l'ammoniaque concentré en chauffant modérément le milieu réactionnel.

Pour la mise en oeuvre du procédé ci-dessus décrit, on utilise de préférence le synthétiseur automatique "Applied Biosystems Modele 381A".

Dans la mise en oeuvre du procédé, objet de l'invention, le support S est un support solide qui peut être constitué par de la silice ou du verre poreux. On peut également utiliser les supports décrits dans le brevet européen n° 0 208 599.

Selon l'invention, Z est un groupement hydrocarboné $-(CH_2)_n-$ dans lequel n est un nombre entier pouvant varier de 2 à 20. On utilise de préférence des produits dans lesquels Z est un radical $-(CH_2)_2-$.

Dans les formules (II) à (VII) les bases $B_1$, $B_2$ .... $B_z$ sont des bases puriques ou pyrimidiques dont les fonctions amines sont protégées. Les groupements protecteurs peuvent être par exemple des groupements benzoyle ou isobutyryle. Dans le cas de l'adénine et de la cytosine, on utilise de préférence le groupement benzoyle. Dans le cas de la guanine on utilise de préférence le groupement isobutyryle.

Le groupement protecteur R de la fonction hydroxyle en 5' est par exemple un radical trityle, monométhoxytrityle, diméthoxytrityle ou pixyle.

Le groupement protecteur $R'_1$ des fonctions hydroxyles des groupements phosphates peut être constitué par exemple par un radical méthyle, cyanoéthyle, ortho ou parachlorophényle. On utilise de préférence le groupement cyanoéthyle. Le groupement protecteur $R_2$ peut être constitué par un radical alcoyle tel que méthyle, éthyle, isopropyle ou par un radical morpholino ou pipéridino. On utilise de préférence le groupement isopropyle.

Lors de la mise en oeuvre du procédé, la fraction du produit de formule (III) qui n'aurait pas réagi est immédiatement transformée en ester pour éviter la formation d'une mauvaise séquence au couplage suivant. Cette réaction de "capping" est effectuée de préférence au moyen de l'anhydride acétique dans le mélange lutidine/tétrahydrofuranne et est catalysée par de la diméthylaminopyridine ou par du méthylimidazole.

Les dérivés de la poly L-lysine des séquences d'oligonucléotides telles ques définies ci-dessus peuvent être préparés par un procédé caractérisé en ce que l'on fait réagir un produit de formule (VIII) :

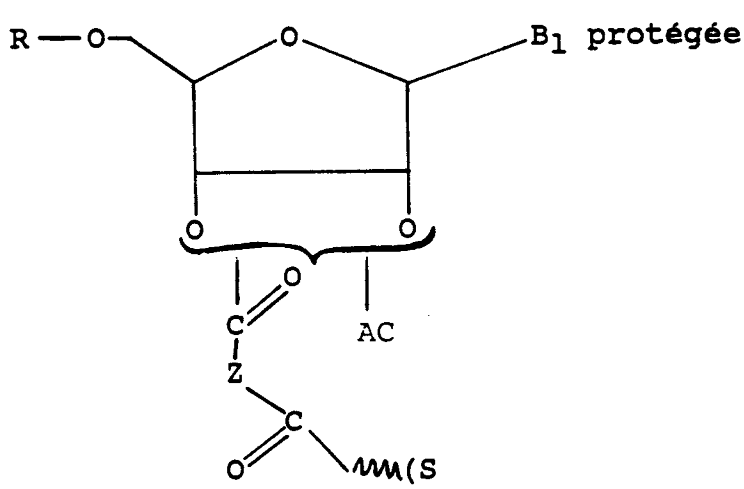

dans laquelle S, Z, R, $B_1$ ont la signification déjà indiquée, avec un produit de formule (IV), pour obtenir un produit de formule (IX) :

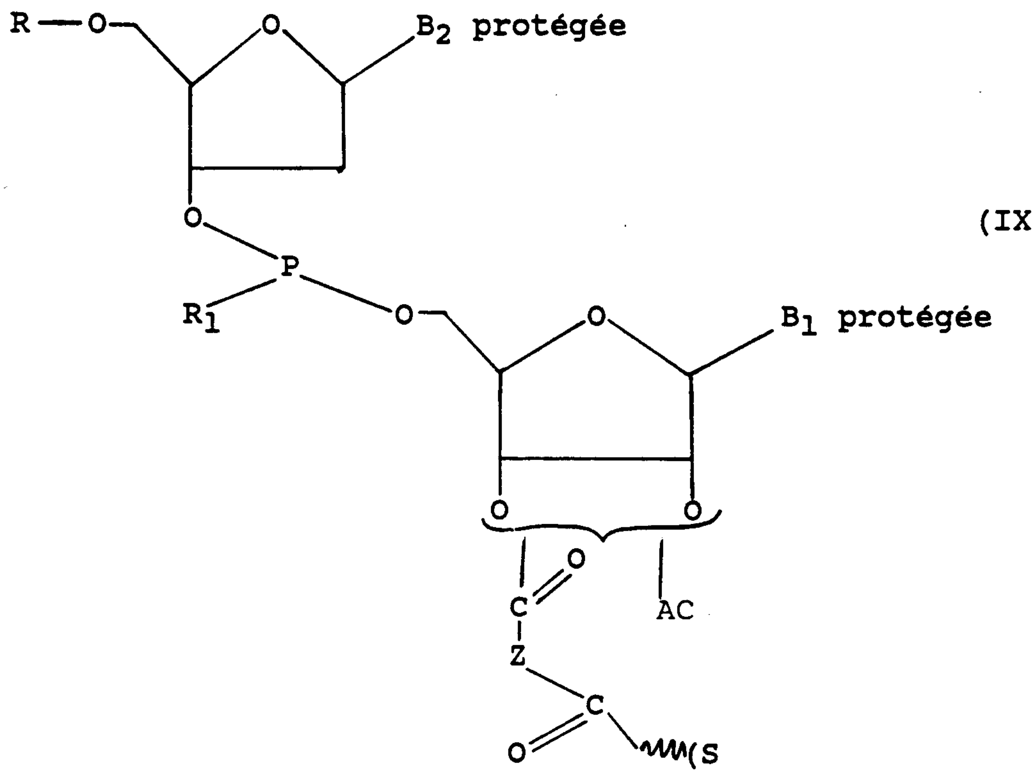

(IX)

dans laquelle S, R, Z, $R_1$, $B_1$ et $B_2$ ont la signification déjà indiquée, oxyde, élimine le support et le groupement activateur pour obtenir un produit de formule (X) :

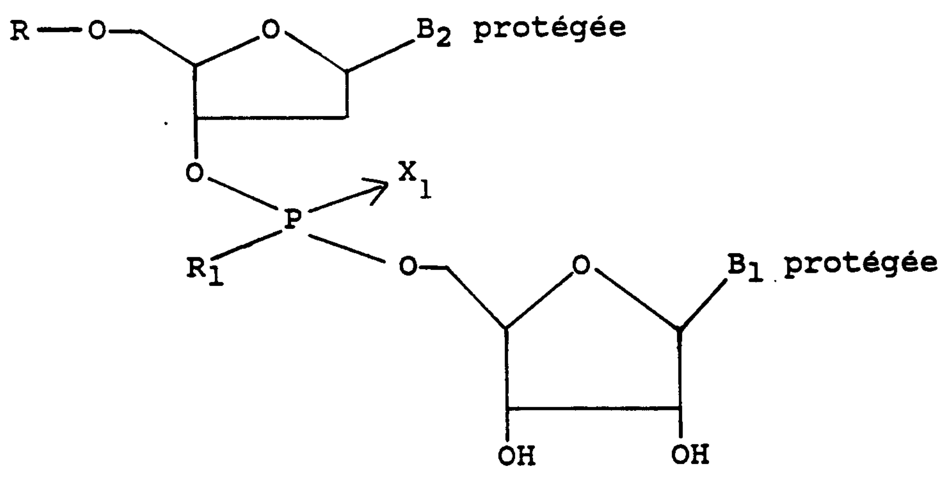

(X)

dans laquelle R, $R_1$, $B_1$ et $B_2$ ont la signification déjà indiquée, $X_1$ représente un atome d'oxygène ou de soufre, oxyde le ribose terminal puis fait réagir avec de la L-lysine en milieu réducteur, pour obtenir le produit de formule (XI) :

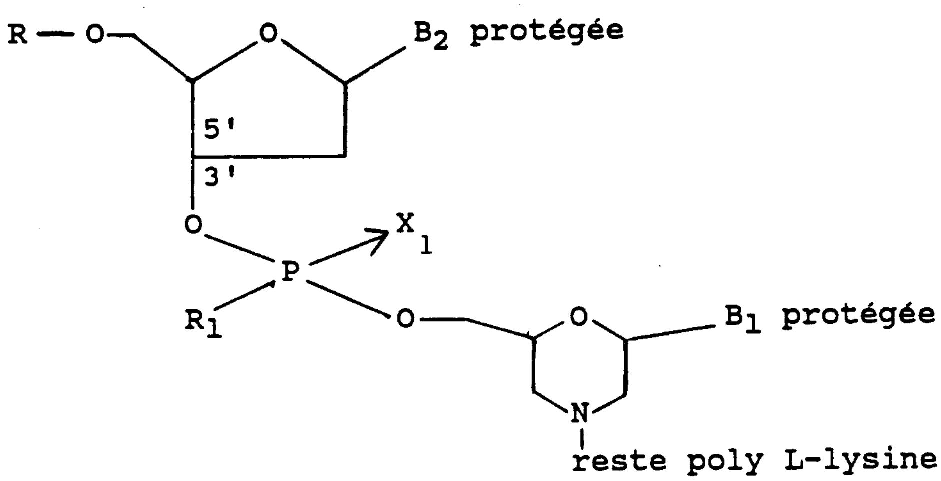

(XI)

dans laquelle R, $R_1$, $B_1$, $B_2$ et $X_1$ ont la signification déjà indiquée, puis poursuit la synthèse comme au départ du produit de formule (VI).

Les dérivés de la poly L-lysine peuvent notamment être préparés selon un procédé analogue au procédé décrit par J.P. LEONETTI et coll. GENE 72 (1988) 323-332.

Les nouvelles séquences d'oligonucléotides objet de la présente invention présentent de très intéressantes propriétés pharmacologiques ; elles possèdent en particulier la capacité de bloquer la production du TNF alpha par les cellules en se fixant spécifiquement sur l'ARN messager cible au niveau de la sequence [166-185] qui se trouve à cheval sur le codon AUG.

Ces propriétés sont illustrées plus loin dans la partie expérimentale.

Ces propriétés justifient l'utilisation des nouvelles séquences d'oligonucléotides telles que définies ci-dessus par la formule (I) à titre de médicaments.

La présente invention a ainsi également pour objet l'application à titre de médicaments des nouvelles séquences d'oligonucléotides anti-sens, anti-ARN messager du TNF alpha, caractérisée en ce qu'elles possèdent la structure de formule (I) suivante :

```
     5'                  3'
(d)  TCA TGG TGT CCT TTG CAG - X          (I)
     1                   18
```

dans laquelle X représente un atome d'hydrogène, ou une séquence de 1 à 17 oligonucléotides, sous forme libre, sous forme alcoylée, sous forme sulfurée ou sous forme de dérivé de la poly L-lysine.

Parmi les médicaments de l'invention, on retient notamment les médicaments caracterisés en ce qu'ils sont constitués par les séquences d'oligonucléotides répondant à la formule (I) ci-dessus dans laquelle X représente un atome d'hydrogène ou tout ou partie de la séquence de formule ($I_A$) :

```
 5'                      3'
 - CC TCA TGC TTT CAG TAG          (IA)
   19                    35
```

sous forme libre, sous forme alcoylée, sous forme sulfurée, ou sous forme de dérivé de la poly L-lysine.

Parmi ces derniers, on retient de préférence les médicaments renfermant les séquences d'oligonucléotides telles que définies ci-dessus, dans laquelle X représente un atome d'hydrogène, une séquence de formule - CC ou la séquence de formule ($I_A$), sous forme libre, sous forme alcoylée, sous forme sulfurée ou sous forme de dérivé de la poly L-lysine.

Parmi les médicaments préférés de l'invention, on retient tout particulièrement les oligonucléotides répondant à la formule :

5'                                    3'
(d) TCA TGG TGT CCT TTG CAG CC
1                                     20

5'                              3'
(d) TCA TGG TGT CCT TTG CAG
1                               18

5'                                                                        3'
(d) TCA TGG TGT CCT TTG CAG CC TCA TGC TTT CAG TAG
1                                                                         35

sous forme libre, sous forme alcoylée, sous forme sulfurée ou sous forme de dérivé de la poly L-lysine.

Ces médicaments trouvent par exemple leur emploi dans le traitement des chocs endotoxiniques infectieux de toutes origines et de tous les états pathologiques liés à une hypersécrétion de TNF alpha.

La dose usuelle, variable selon le produit utilisé, le sujet traité et l'affection en cause peut être par exemple de 1 microgramme à 30 mg par jour par voie intraveineuse chez l'homme.

L'invention a également pour objet les compositions pharmaceutiques qui renferment au moins une séquence d'oligonucléotide de principe actif.

A titre de principe actif, les séquences d'oligonucléotides répondant à la formule (I) ci-dessus, sous forme libre, sous forme alcoylée, sous forme sulfurée ou sous forme de dérivé de la poly L-lysine, peuvent être incorporées dans des compositions pharmaceutiques destinées à la voie digestive, parentérale ou locale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables, les aérosols ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif, des exemples de mise en oeuvre de l'invention.

**EXEMPLE 1 : Séquence (d) 5' TCA TGG TGT CCT TTG CAG 3' (18 mère anti-TNF alpha) sous forme libre.**

Appareillage :

On utilise un synthétiseur automatique "Applied Biosystems Modele 381 A". On charge l'appareil de tous les réactifs utiles. Les produits de formule (IV) sont mis en solution dans l'acétonitrile ; ils sont sous légère pression d'argon ce qui leur permet d'être délivrés automatiquement par ouverture d'électrovannes au fur et à mesure des besoins.

On inscrit la séquence du nucléotide à synthétiser. On indique la quantité de produit sur laquelle on veut travailler.

On programme ou non la détritylation finale pour récupérer le produit de formule (I) sous forme hydroxylée ou sous forme tritylée en 5.

On place une petite colonne préremplie contenant le support fonctionnalisé (II) sur l'appareil (support de verre poreux CPG).

La synthèse s'effectue ensuite de bout en bout sans intervention manuelle.

Les solutions de détritylation sont récupérées automatiquement à chaque stade dans un collecteur de fractions couplé à l'appareil ; on peut ainsi connaitre les rendements de chaque stade.

Réactifs :

Tous les réactifs utilisés doivent être d'une grande pureté.

Les 4 phosphoramidites de formule (IV) ($B_2$ = adénine, cytosine, guanine ou thymine) sont conditionnés dans de petits flacons. Il suffit d'ajouter la quantité nécessaire de solvant avant de les introduire dans l'appareil.

Les autres solutions introduites dans l'appareil sont les suivantes :

- Tétrazole à 4 % dans l'acétonitrile pour l'activateur de couplage,
- Anhydride acétique/lutidine/tétrahydrofuranne 1/1/8 d'une part et diméthylaminopyridine/tétrahydrofuranne 7/93 d'autre part, mélangés in situ pour la réaction de "capping",
- Iode/eau/lutidine/tétrahydrofuranne 3/2/2/93 pour l'oxydation du phosphite de formule (V) en phosphate de formule (VI),
- Acide trichloracétique à 2 % dans le dichlorométhane pour la réaction de détritylation,
- Acétonitrile pur pour la dissolution des phosphoramidites.

Méthode :

La réaction est effectuée sur 0,95 micromole. Le produit est détritylé dans l'appareil à la fin de la synthèse.

Le rendement de la synthèse en produit brut est de 79 %. Le déblocage du produit de formule (VII) est effectué de la manière suivante :

1 - ammoniaque à 28 % / 1 heure à température ambiante,
2 - ammoniaque saturé / 60 °C / 5 heures.

Purification :

1 - Dessalage sur colonne de NAP10 (Pharmacia) Sephadex G50®.
2 - HPLC sur colonne de Partisil 10 SAX® gradient de 50 à 99 % de solvant A dans le mélange A + B en 20 mn.
A = tampon phosphate 0,3 M / $CH_3CN$ 7/3 pH 6,2
B = tampon phosphate $10^{-3}$M / $CH_3CN$ 7/3 pH 6,2
débit 2 $cm^3$/mn.
3) Dessalage sur colone de NAP 25 (Pharmacia) Sephadex G50®.

Analyse du produit purifié :

- HPLC sur Partisil 10 SAX® mêmes conditions que ci-dessus TR = 13,92 mn.
- HPLC sur phase inverse microbondapack C18® gradient 8,4 à 15 % de $CH_3CN$ dans acétate de triéthylammonium $10^{-2}$M pH 5,5 en 20 mn, débit 2 $cm^3$/mn, TR = 12,13 mn.

On obtient finalement 0,125 micromole de produit pur, rendement 13 %.

**5' 3'**

**EXEMPLE 2 : Séquence (d) TCA TGG TGT CCT TTG CAG (18 mère anti-TNF alpha) sous forme tout thioate.**

En opérant comme à l'exemple 1 mais en remplaçant le cycle d'oxydation à l'iode par une étape d'oxydation au soufre de 450 secondes, on a obtenu le produit cité en rubrique.

L'oxydation à l'iode est remplacée par une étape d'oxydation au soufre. La solution d'iode/eau/lutidine/tétrahydrofuranne est remplacée par une solution de :
3 g de soufre
28,5 $cm^3$ de sulfure de carbone
28,5 $cm^3$ de pyridine anhydre
3 $cm^3$ de triéthylamine anhydre.

Dans le cas d'une oxydation par le soufre, il est indispensable de rincer la colonne avant et après l'oxydation par des lavages répétés d'une solution de sulfure de carbone dans la pyridine 1/1 pour enlever tout résidu de soufre.
Pour le reste la méthode demeure inchangée.
Rendement de synthèse sur 1,24 micromole : 56,5 %.

Déblocage :

1 - ammoniaque à 28 % / 1 heure / température ambiante
2 - ammoniaque saturé 1 nuit à 50 °C.

Purification :

1 - dessalage NAP 25 (Sephadex)® éluant $H_2O$.
2 - HPLC contrôle microbondapack C18®
TR = 24,46
Gradient de 30 à 50 % de A en 20 mn, débit 2 $cm^3$/mn.
A = acétate de triéthylammonimum $10^{-2}$M/l, pH 5,5/$CH_3CN$ 7/3
B = acétate de triéthylammonium $10^{-2}$M/l, pH 5,5

Rendement produit pur : 40,3 %.

**EXEMPLE 3 : Séquence (d) 5' TCA TGG TGT CCT TTG CAG CC 3' (20 mère anti-TNF alpha) sous forme libre.**

En opérant comme à l'exemple 1, on a obtenu le produit cité en rubrique.

**EXEMPLE 4 : Séquence (d) 5' TCA TGG TGT CCT TTG CAG CC TCA TGC TTT CAG TAG 3' (35 mère anti-TNF alpha) sous forme libre.**

En opérant comme à l'exemple 1, on a obtenu le produit cité en rubrique

**EXEMPLE 5 :**

On a préparé un soluté injectable répondant à la formulation suivante :

| | |
|---|---|
| - Produit de l'exemple 1 | 10 microgrammes |
| - Excipient aqueux stérile | 2 ml |

**EXEMPLE 6 :**

On a préparé des comprimés répondant à la formule suivante :

| | |
|---|---|
| - Produit de l'exemple 2<br>- Excipient q.s.p. un comprimé terminé à | 20 microgrammes<br>100 mg |
| (composition de l'excipient : lactose, amidon, talc, stéarate de magnésium). | |

**EXEMPLE 7 :**

On a préparé un soluté injectable, répondant à la formulation suivante :

| | |
|---|---|
| - Produit de l'exemple 3<br>- Excipient aqueux stérile | 10 microgrammes<br>2 ml |

ETUDE DE LA SEQUENCE D'OLIGONUCLEOTIDE PREPAREE A L'EXEMPLE 3.

1 - Test biologique

1.1 - Incuber les monocytes humains (4 x $10^6$ cellules) avec 6 micromoles d'oligonucléotides anti-sens sous les 4 formes pendant 3 h.
1.2 - Rajouter ensuite le LPS (10 microgrammes/ml) et l'INF (50 x $10^3$ u/ml).
1.3 - Incuber 18 h.
1.4 - Reprendre le surnageant de culture.
1.5 - Rajouter ce surnageant aux cellules sensibles à la lyse par le TNF (L 929) pendant 24 h.
1.6 - Mesurer la viabilité cellulaire par colorimétrie avec crystal violet.

2 - Test immunologique

2.1 - Incuber les monocytes humains (4 x $10^6$ cellules) avec 6 micromoles d'oligonucléotides anti-sens sous les 4 formes pendant 3 h.
2.2 - Rajouter ensuite le LPS (10 microgrammes/ml) et l'INF (50 x $10^3$ u/ml) pour la stimulation de la production de TNF.
2.3 - Incuber 18 h.
2.4 - Reprendre le surnageant de culture.
2.5 - Faire un coating avec ce surnageant de culture.
2.6 - Rajouter un anti-corps anti TNF marqué à l'iode (pour RIA) ou à un enzyme (pour ELISA).
2.7 - Incuber pendant 3 h.
2.8 - Révélation :
a) rajouter le substrat d'enzyme (ELISA) et lire la densité optique.
b) doser la radioactivité (RIA).
2.9 - Déterminer la quantité de TNF produite par les monocytes.
2.10 - Calculer l'indice d'inhibition de la production du TNF.

3 - RESULTATS

Indice d'inhibition de la production du TNF alpha par les monocytes traités par les oligonucléotides anti-sens.

$$\text{Indice d'inhibition} = 1 - \frac{\text{V/ml en présence des oligos}}{\text{V/ml en absence des oligos}} \times 100 = 63,5 \pm 9$$

**Revendications**

**Revendications pour les Etats contractants suivants : AT, BE, CH, DE, DK, FR, GB, IT, LI, LU, NL, SE**

**1.** Séquence d'oligonucléotides anti-sens, anti-ARN messager du TNF alpha, caractérisée en ce qu'elle possède la structure de formule (I) suivante :

```
    5'                     3'
(d) TCA TGG TGT CCT TTG CAG - X                (I)
    1                      18
```

dans laquelle X représente un atome d'hydrogène, ou une séquence de 1 à 17 oligonucléotides, sous forme libre, sous forme alcoylée, sous forme sulfurée ou sous forme de dérivé de la poly L-lysine.

**2.** Séquence d'oligonucléotides répondant à la formule (I) de la revendication 1, caractérisée en ce que X représente un atome d'hydrogène ou tout ou partie de la séquence de formule ($I_A$) :

```
5'                     3'
 - CC TCA TGC TTT CAG TAG                (I_A)
   19                  35
```

sous forme libre, sous forme alcoylée, sous forme sulfurée, ou sous forme de dérivé de la poly L-lysine.

**3.** Séquence d'oligonucléotides telle que définie par la formule (I) de la revendication 1, caractérisée en ce que dans ladite formule (I), X représente un atome d'hydrogène, une séquence de formule - CC ou la séquence de formule ($I_A$), sous forme libre, sous forme alcoylée, sous forme sulfurée ou sous forme de dérivé de la poly L-lysine.

**4.** Séquence d'oligonucléotides selon l'une quelconque des revendications 1 à 3, caractérisée en ce qu'elle répond à la formule :

```
    5'                        3'
(d) TCA TGG TGT CCT TTG CAG CC
    1                         20
```

sous forme libre, sous forme alcoylée, sous forme sulfurée ou sous forme de dérivé de la poly L-lysine.

**5.** Séquence d'oligonucléotides selon l'une quelconque des revendications 1 à 3, caractérisée en ce qu'elle répond à la formule :

```
    5'                     3'
(d) TCA TGG TGT CCT TTG CAG
    1                      18
```

sous forme libre, sous forme alcoylée, sous forme sulfurée ou sous forme de dérivé de la poly L-lysine.

**6.** Séquence d'oligonucléotides selon l'une quelconque des revendications 1 à 3, caractérisée en ce qu'elle répond à la formule :

```
     5'                                              3'
(d)  TCA TGG TGT CCT TTG CAG CC TCA TGC TTT CAG TAG
     1                                               35
```

sous forme libre, sous forme alcoylée, sous forme sulfurée ou sous forme de dérivé de la poly L-lysine.

**7.** Procédé de préparation de la séquence d'oligonucléotides telle que définie par la formule (I) de la revendication 1, caractérisé en ce que l'on immobilise sur un support S le premier nucléotide en 3′ de la chaine à synthétiser de formule :

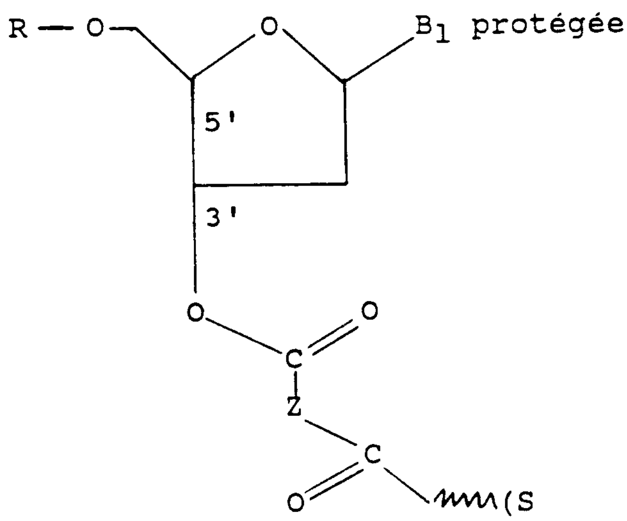

(II)

dans laquelle S représente le support, Z représente un groupement hydrocarboné renfermant de 2 à 20 atomes de carbone, $B_1$ est une base purique ou pyrimidique correspondant au premier nucléotide dont la fonction amine est protégée et R est un groupement protecteur,
débloque l'hydroxyle en 5′ par un réactif acide pour obtenir le produit de formule :

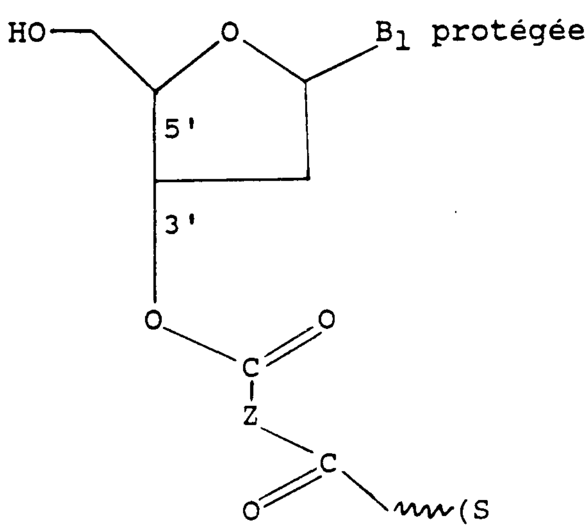

(III)

dans laquelle S, Z et $B_1$ ont la signification déjà indiquée, puis fait réagir ce dernier avec le monomère du second nucléotide de formule :

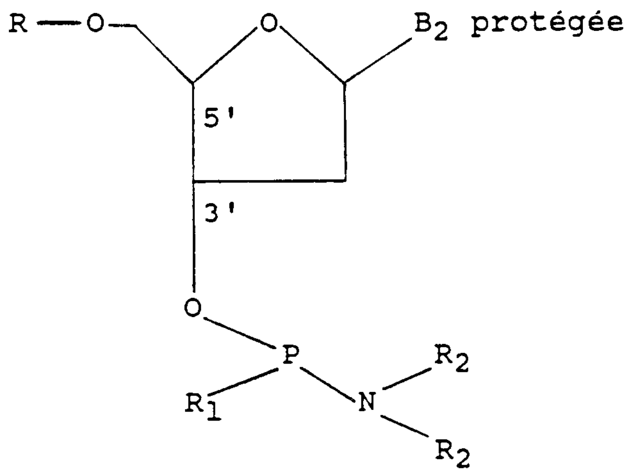

(IV)

dans laquelle R a la signification déjà indiquée, $B_2$ est une base purique ou pyrimidique correspondant au second nucléotide dont la fonction amine est protégée, $R_1$ représente un radical alcoyle ou un groupement $-OR'_1$ ou $-SR'_1$ dans lequel $R'_1$ représente un groupement protecteur, $R_2$ représente un groupement protecteur, pour obtenir un produit de formule :

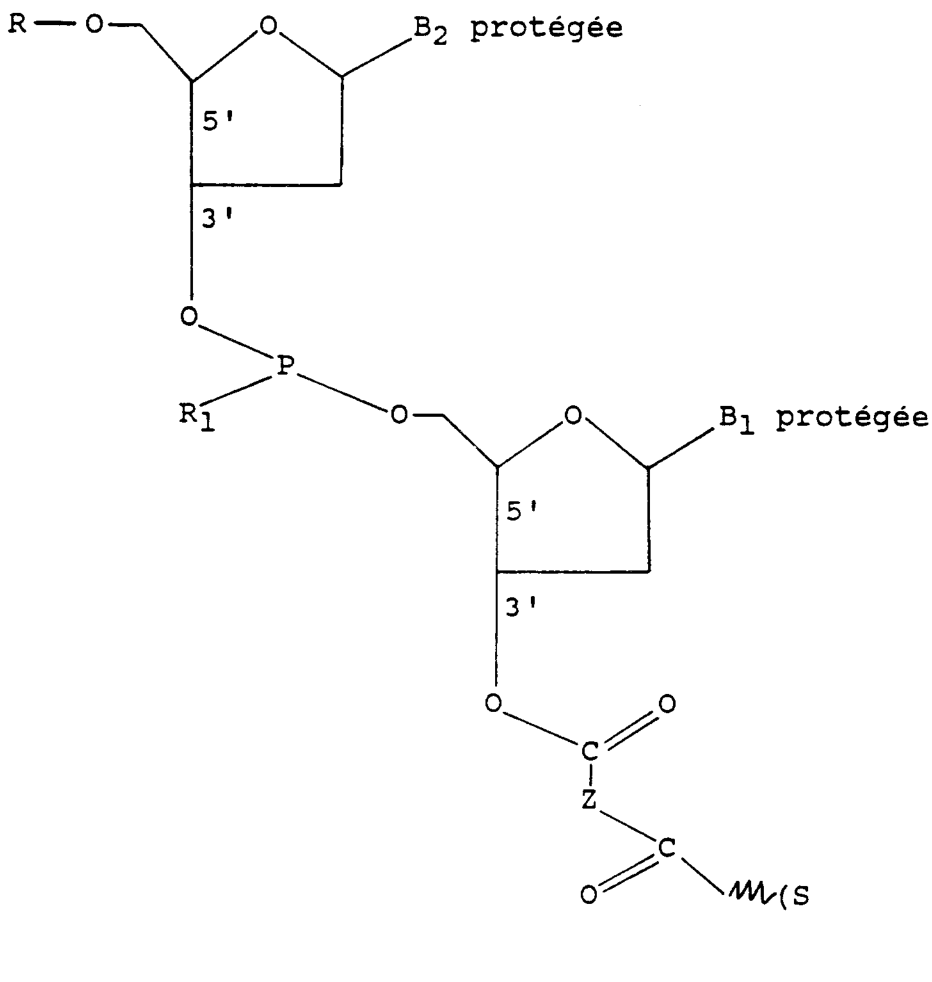

(V)

dans laquelle S, R, $R_1$, $B_1$ et $B_2$ ont la signification déjà indiquée, puis oxyde le produit de formule (V) obtenu pour obtenir un produit de formule :

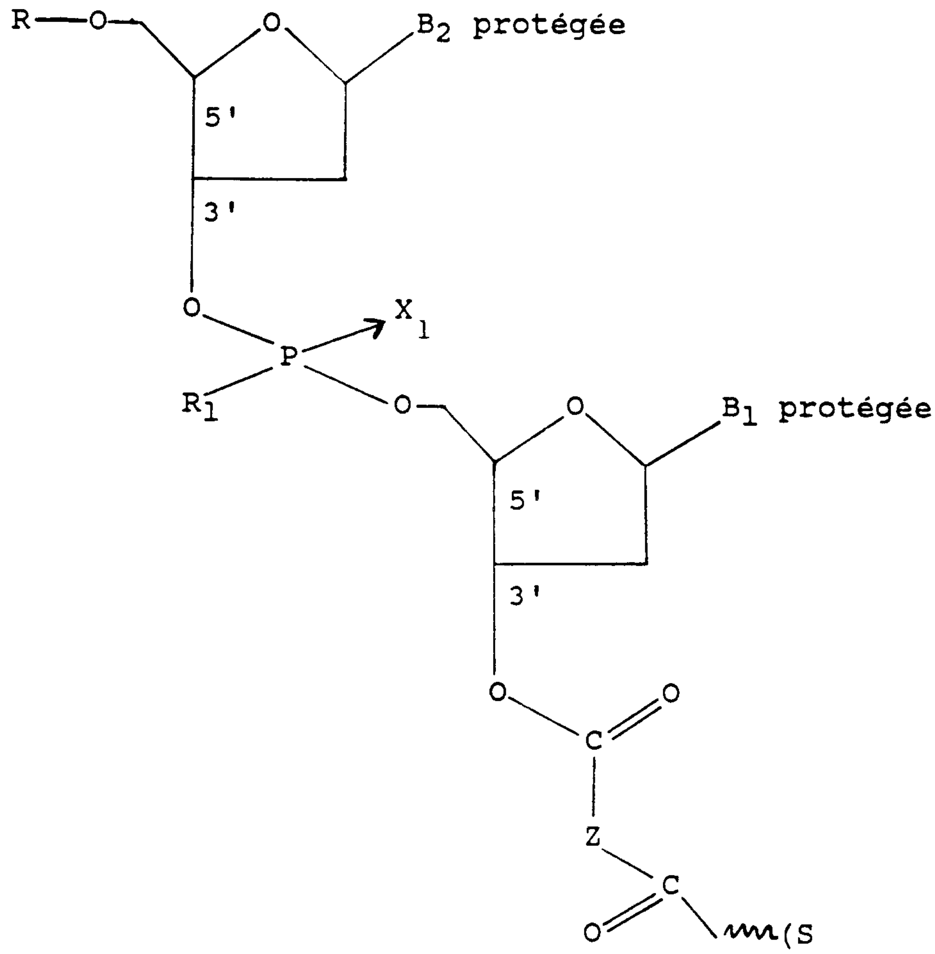

(VI)

dans laquelle S, R, $R_1$, $B_1$, $B_2$ et Z ont la signification déjà indiquée et $X_1$ représente un atome d'oxygène ou de soufre, puis, comme ci-dessus, au départ du produit de formule (II) effectue un nouveau cycle avec ce produit de formule (VI) et le monomère d'un nouveau nucléotide jusqu'à obtention de l'enchaînement désiré pour obtenir finalement un produit de formule :

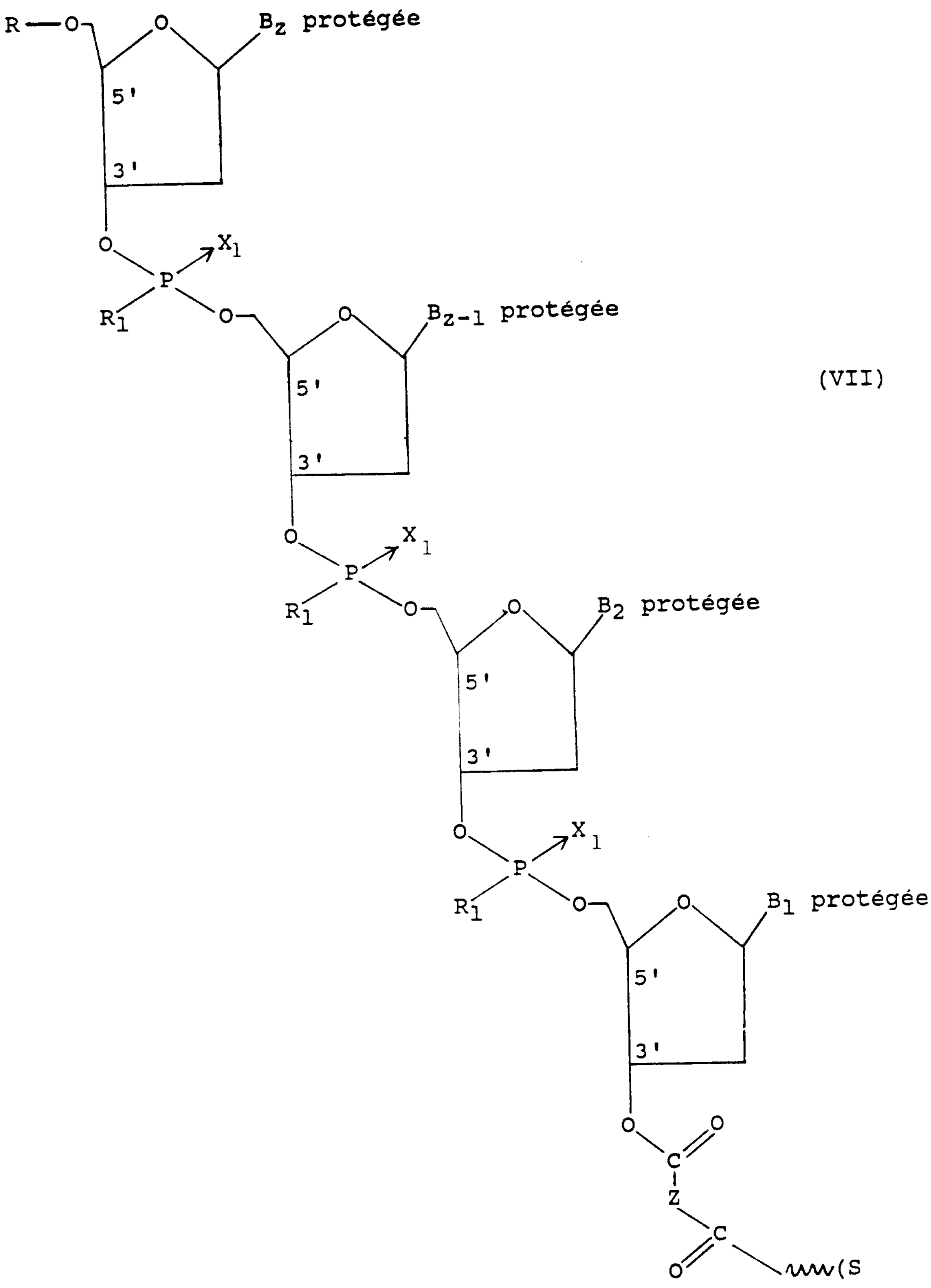

(VII)

dans laquelle S, R, $R_1$, $B_1$, $B_2$, $X_1$ et Z ont la signification déjà indiquée et $B_z$ représente le dernier nucléotide de la séquence, puis déprotège l'oligonucléotide, le sépare du support et purifie le produit de formule (I) ainsi obtenu.

**8.** Procédé selon la revendication 7, caractérisé en ce que :

- le déblocage de l'hydroxyle en 5′ du produit de formule (II) est effectué par un réactif acide tel que l'acide acétique, l'acide di ou trichloracétique,
- la réaction de couplage du produit de formule (III) avec le produit de formule (IV) est activée au moyen du tétrazole en opérant dans l'acétonitrile,
- l'oxydation du phosphite de formule (V) en phosphate de formule (VI) est effectuée au moyen de l'iode en solution dans un mélange eau/lutidine/tétrahydrofuranne ou dans un mélange eau/pyridine/tétrahydrofuranne,
- l'oxydation du phosphite de formule (V) pour obtenir la forme sulfurée (VI) est effectuée au moyen du soufre en solution dans un mélange sulfure de carbone, de pyridine et de triéthylamine anhydre,
- en fin de synthèse la déprotection du groupement hydroxyle terminal en 5′ est effectuée au moyen d'un acide tel que l'acide acétique, l'acide di ou trichloracétique,
- la déprotection de l'oligonucléotide de formule (VII) est effectuée au moyen de l'ammoniaque concentré en chauffant modérément le milieu réactionnel.

**9.** Procédé selon la revendicaion 7 pour la préparation des dérivés de la poly L-lysine des séquences d'oligonucléotides telles que définies par la formule (I), procédé caractérisé en ce que l'on fait réagir un produit de formule (VIII) :

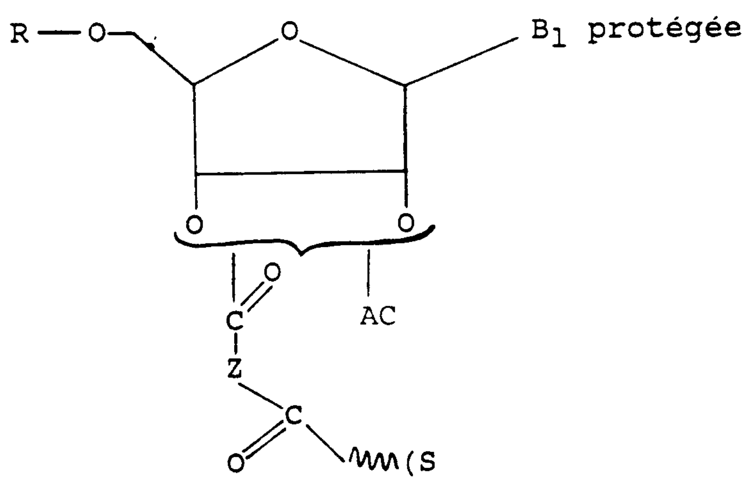

(VIII)

dans laquelle S, Z, R, $B_1$ ont la signification déjà indiquée, avec un produit de formule (IV), pour obtenir un produit de formule (IX) :

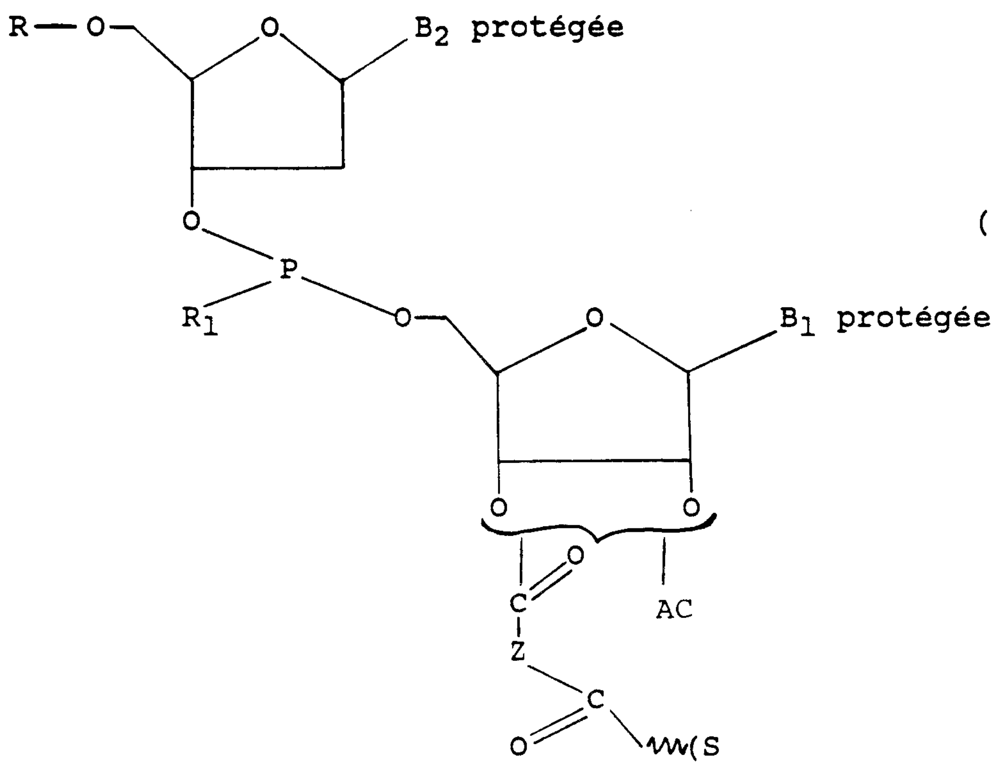

(IX)

dans laquelle S, R, Z, $R_1$, $B_1$ et $B_2$ ont la signification déjà indiquée, oxyde, élimine le support et le groupement activateur pour obtenir un produit de formule (X) :

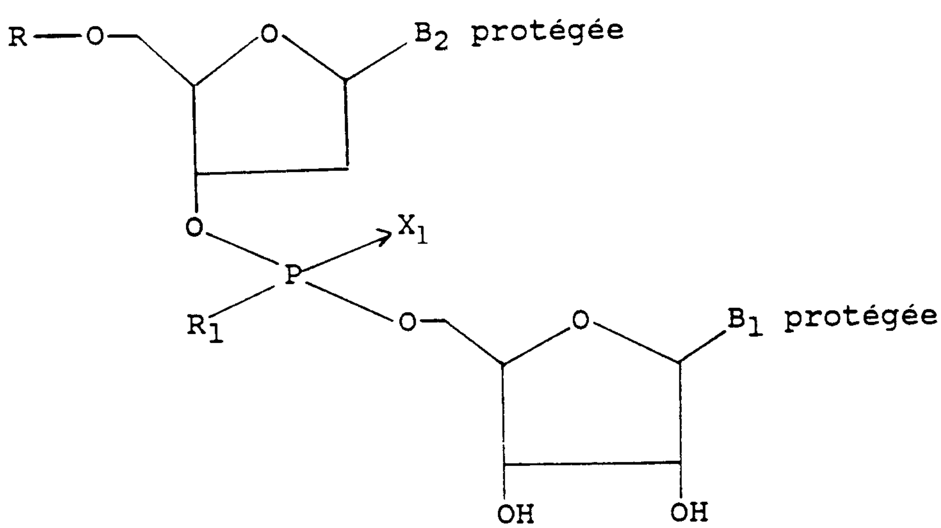

(X)

dans laquelle R, $R_1$, $B_1$ et $B_2$ ont la signification déjà indiquée, $X_1$ représente un atome d'oxygène ou de soufre, oxyde le ribose terminal puis fait réagir avec de la L-lysine en milieu réducteur, pour obtenir le produit de formule (XI) :

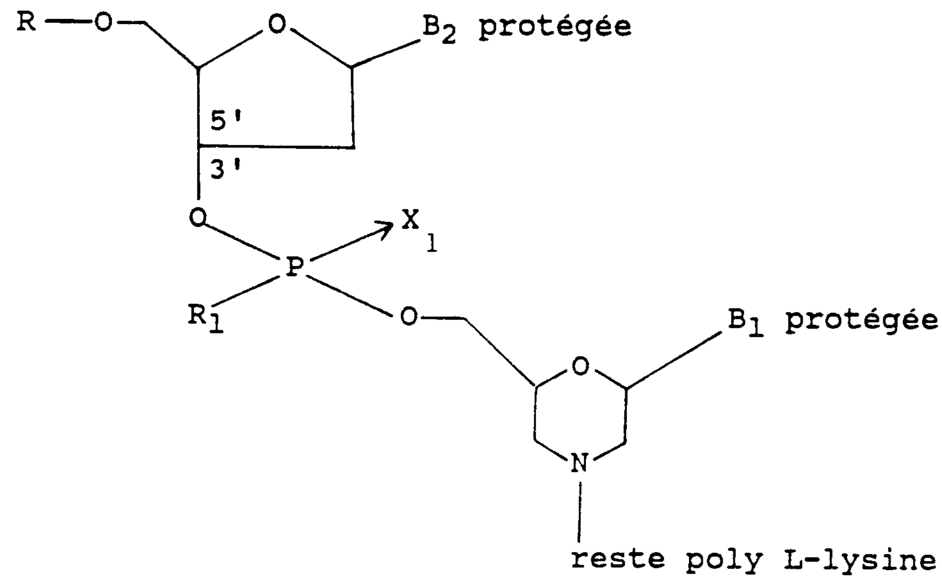

(XI)

dans laquelle R, $R_1$, $B_1$, $B_2$ et $X_1$ ont la signification déjà indiquée, puis poursuit la synthèse comme au départ du produit de formule (VI).

**10.** Médicaments, caractérisés en ce qu'ils sont constitués par les nouvelles séquences d'oligonucléotides telles que définies à la revendication 1.

**11.** Médicaments, caractérisés en ce qu'ils sont constitués par les nouvelles séquences d'oligonucléotides telles que définies à l'une quelconque des revendications 2 à 6.

**12.** Compositions pharmaceutiques, caractérisées en ce qu'elles renferment, à titre de principe actif, l'un ou moins des médicaments tels que définis à l'une quelconque des revendications 10 ou 11.

**Revendications pour l'Etat contractant suivant : ES**

**1.** Procédé de préparation de la séquence d'oligonucléotides anti-sens, anti-ARN messager du TNF alpha, possèdant la structure de formule (I) suivante :

```
     5'                      3'
(d)  TCA TGG TGT CCT TTG CAG - X          (I)
     1                       18
```

dans laquelle X représente un atome d'hydrogène, ou une séquence de 1 à 17 oligonucléotides, sous forme libre, sous forme alcoylée, sous forme sulfurée ou sous forme de dérivé de la poly L-lysine, caractérisé en ce que l'on immobilise sur un support S le premier nucléotide en 3′ de la chaîne à synthétiser de formule :

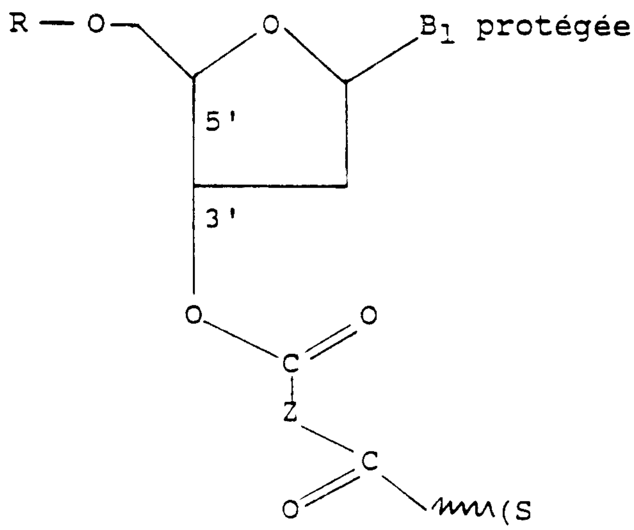

dans laquelle S représente le support, Z représente un groupement hydrocarboné renfermant de 2 à 20 atomes de carbone, $B_1$ est une base purique ou pyrimidique correspondant au premier nucléotide dont la fonction amine est protégée et R est un groupement protecteur,
débloque l'hydroxyle en 5' par un réactif acide pour obtenir le produit de formule :

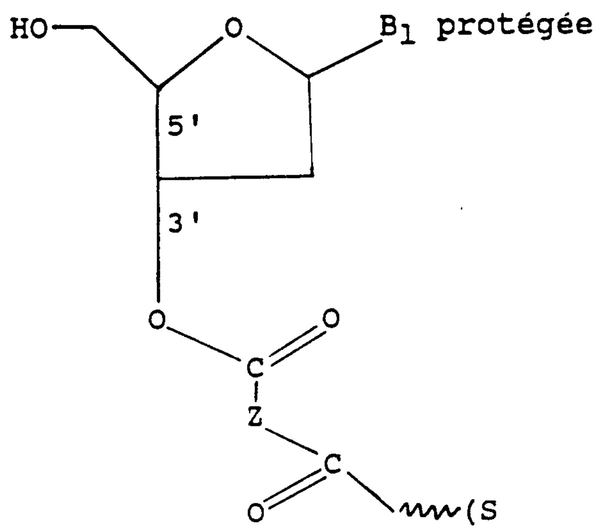

(III)

dans laquelle S, Z et $B_1$ ont la signification déjà indiquée, puis fait réagir ce dernier avec le monomère du second nucléotide de formule :

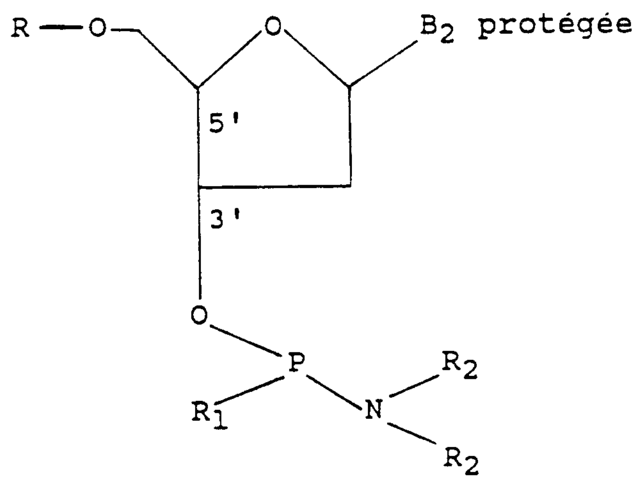

(IV)

dans laquelle R a la signification déjà indiquée, $B_2$ est une base purique ou pyrimidique correspondant au second nucléotide dont la fonction amine est protégée, $R_1$ représente un radical alcoyle ou un groupement $-OR'_1$ ou $-SR'_1$ dans lequel $R'_1$ représente un groupement protecteur, $R_2$ représente un groupement protecteur, pour obtenir un produit de formule :

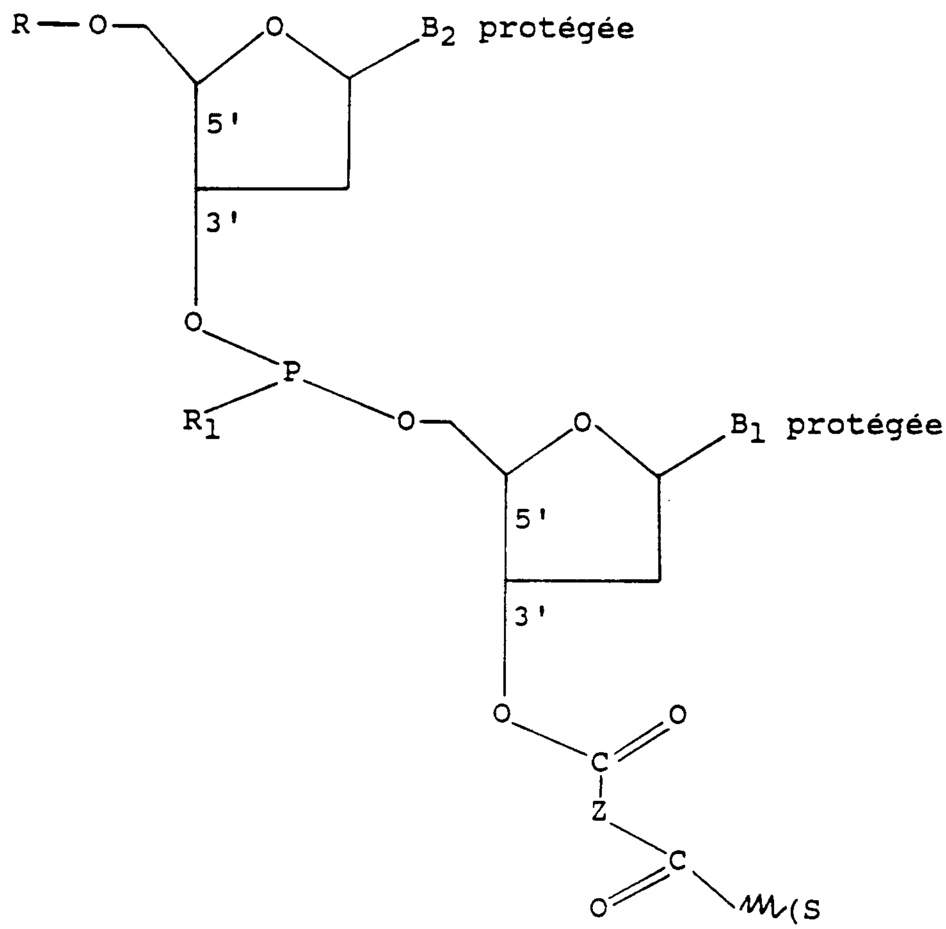

(V)

dans laquelle S, R, $R_1$, $B_1$ et $B_2$ ont la signification déjà indiquée, puis oxyde le produit de formule (V) obtenu pour obtenir un produit de formule :

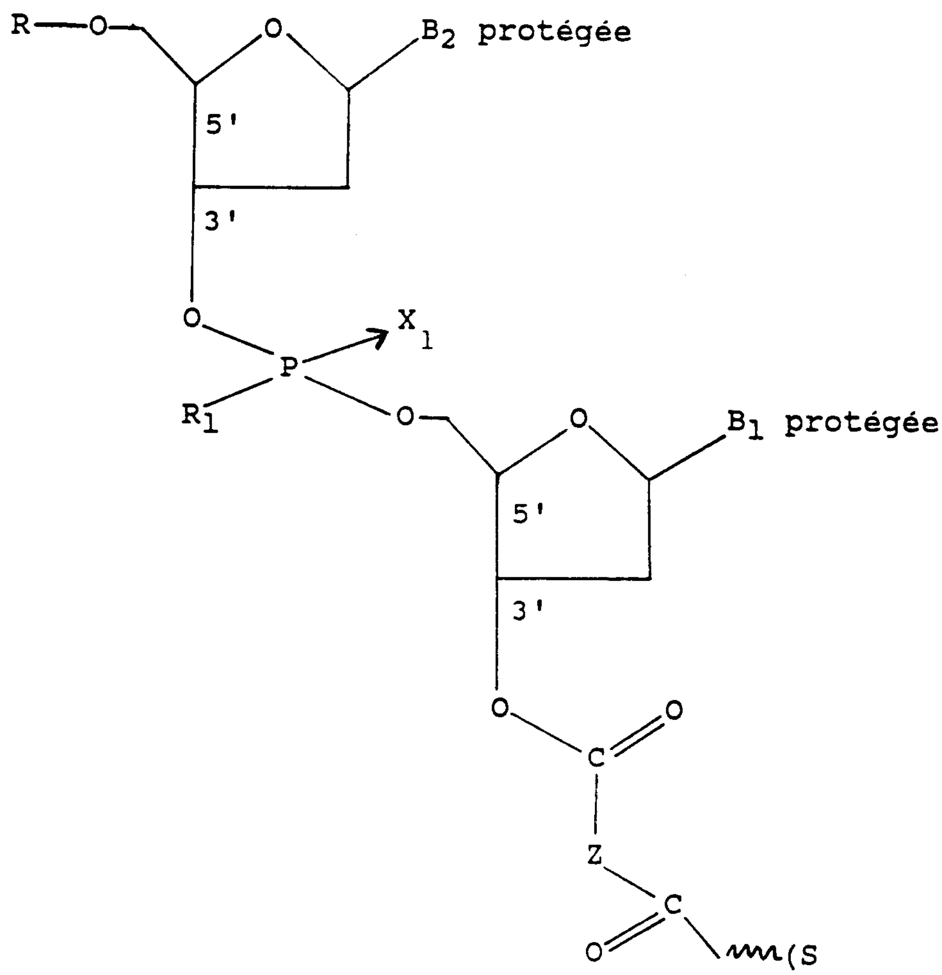

(VI)

dans laquelle S, R, $R_1$, $B_1$, $B_2$ et Z ont la signification déjà indiquée et $X_1$ représente un atome d'oxygène ou de soufre, puis, comme ci-dessus, au départ du produit de formule (II) effectue un nouveau cycle avec ce produit de formule (VI) et le monomère d'un nouveau nucléotide jusqu'à obtention de l'enchaînement désiré pour obtenir finalement un produit de formule :

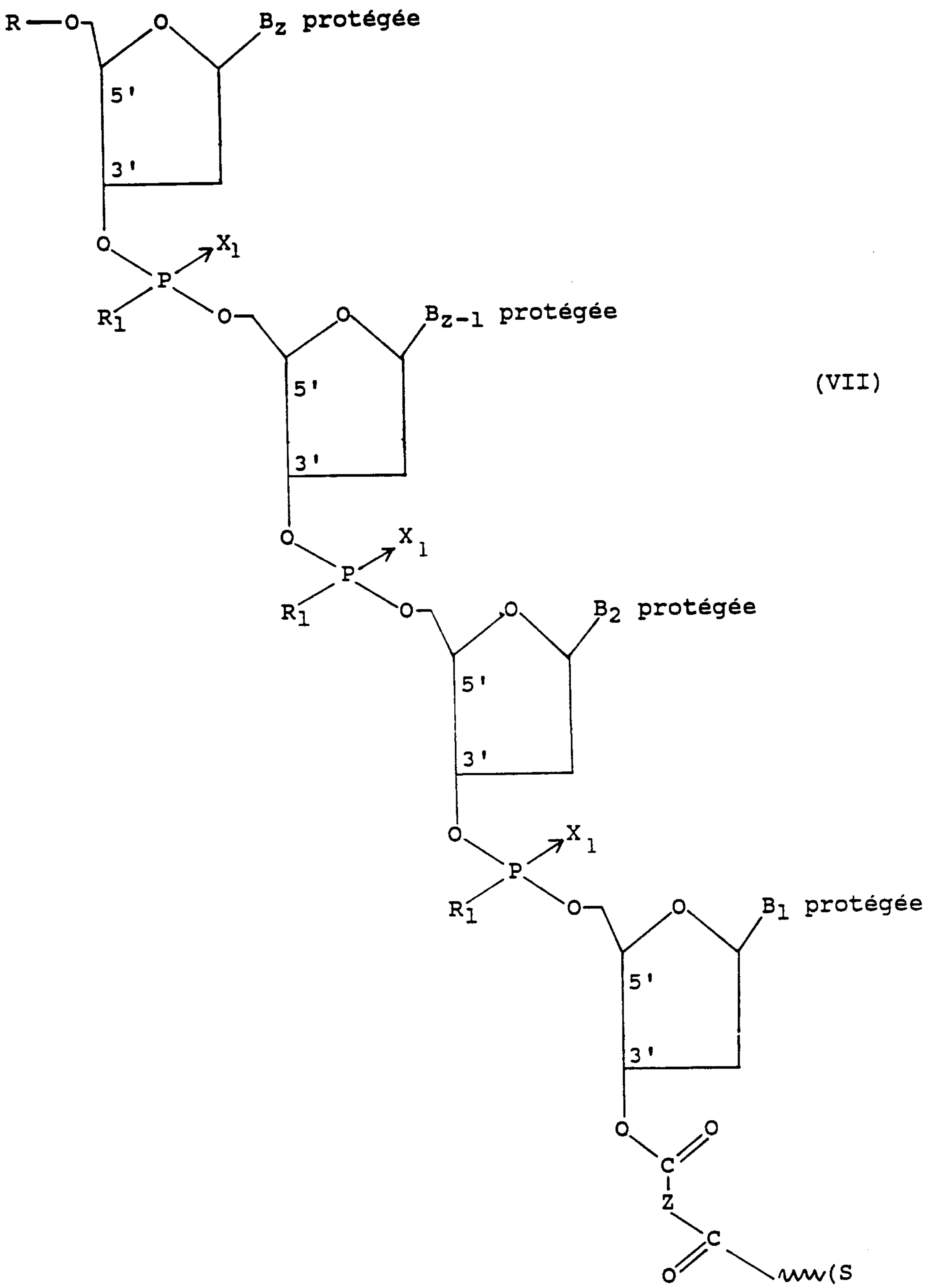

(VII)

séquence, puis déprotège l'oligonucléotide, le sépare du support et purifie le produit de formule (I) ainsi obtenu.

2. Procédé selon la revendication 1, caractérisé en ce que:
   - le déblocage de l'hydroxyle en 5′ du produit de formule (II) est effectué par un réactif acide tel que l'acide acétique, l'acide di ou trichloracétique,

- la réaction de couplage du produit de formule (III) avec le produit de formule (IV) est activée au moyen du tétrazole en opérant dans l'acétonitrile,
- l'oxydation du phosphite de formule (V) en phosphate de formule (VI) est effectuée au moyen de l'iode en solution dans un mélange eau/lutidine/tétrahydrofuranne ou dans un mélange eau/pyridine/tétrahydrofuranne,
- l'oxydation du phosphite de formule (V) pour obtenir la forme sulfurée (VI) est effectuée au moyen du soufre en solution dans un mélange sulfure de carbone, de pyridine et de triéthylamine anhydre,
- en fin de synthèse la déprotection du groupement hydroxyle terminal en 5′ est effectuée au moyen d'un acide tel que l'acide acétique, l'acide di ou trichloracétique,
- la déprotection de l'oligonucléotide de formule (VII) est effectuée au moyen de l'ammoniaque concentré en chauffant modérément le milieu réactionnel.

**3.** Procédé selon la revendicaion 1 pour la préparation des dérivés de la poly L-lysine des séquences d'oligonucléotides telles que définies par la formule (I), procédé caractérisé en ce que l'on fait réagir un produit de formule (VIII) :

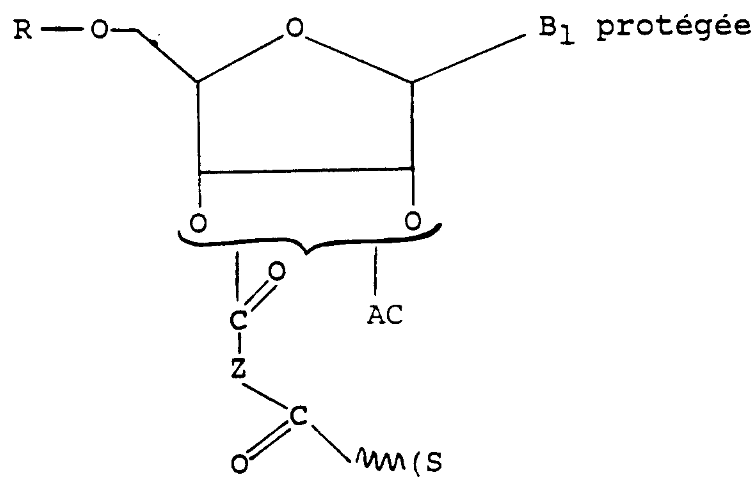

(VIII)

dans laquelle S, Z, R, $B_1$ ont la signification déjà indiquée, avec un produit de formule (IV), pour obtenir un produit de formule (IX) :

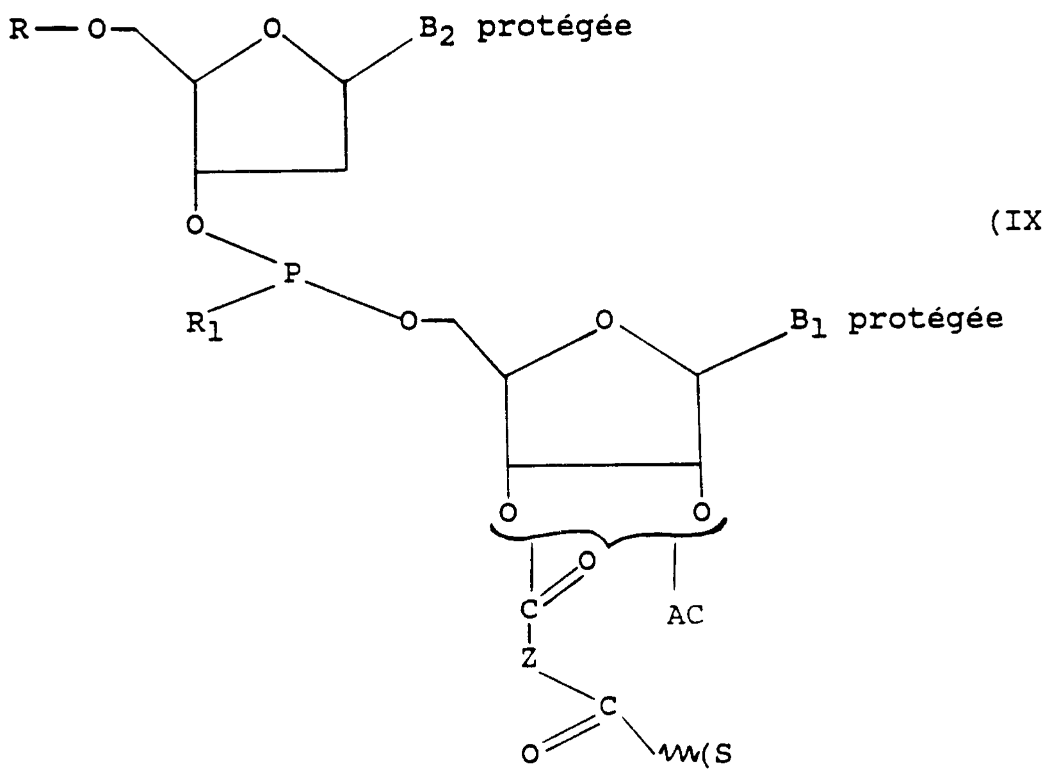

(IX)

dans laquelle S, R, Z, $R_1$, $B_1$ et $B_2$ ont la signification déjà indiquée, oxyde, élimine le support et le groupement activateur pour obtenir un produit de formule (X) :

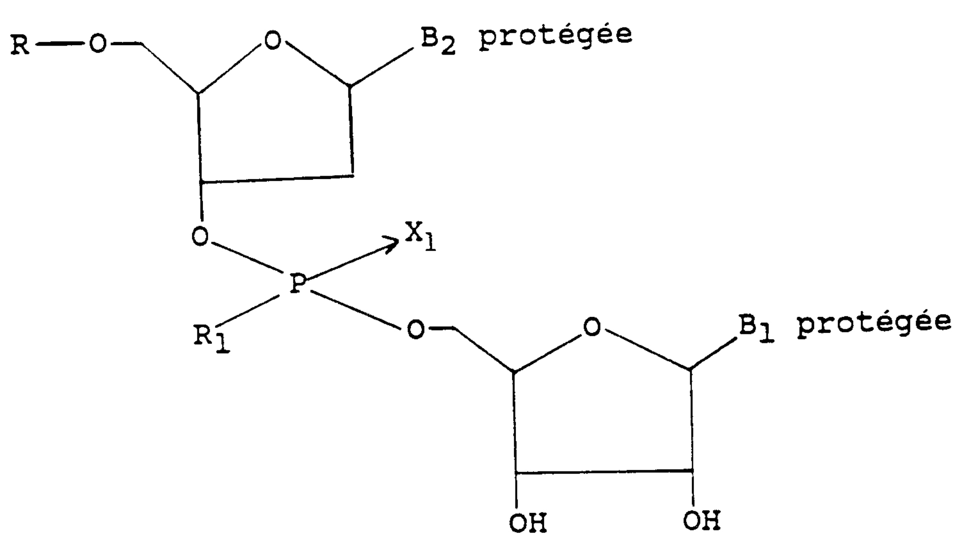

(X)

dans laquelle R, $R_1$, $B_1$ et $B_2$ ont la signification déjà indiquée, $X_1$ représente un atome d'oxygène ou de soufre, oxyde le ribose terminal puis fait réagir avec de la L-lysine en milieu réducteur, pour obtenir le produit de formule (XI) :

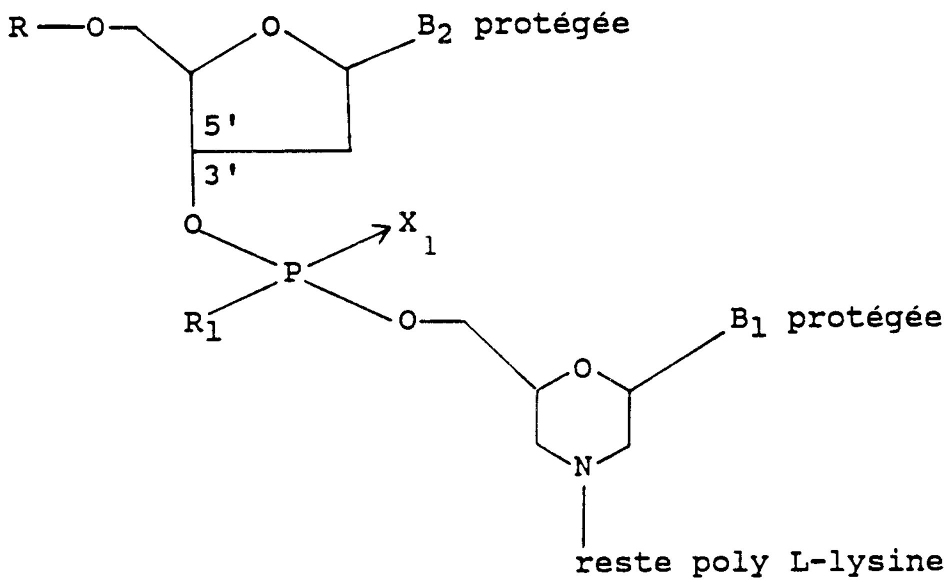

(XI)

dans laquelle R, $R_1$, $B_1$, $B_2$ et $X_1$ ont la signification déjà indiquée, puis poursuit la synthèse comme au départ du produit de formule (VI).

**4.** Procédé selon la revendication 1, 2 ou 3, caractérisé en ce que le premier nucléotide en 3′ de la chaîne à synthétiser est soit le premier nucléotide de la séquence finale

3'

TCA TGG TGT CCT TTG CAG

soit l'un des nucléotides de la séquence de formule ($I_A$) :
- CC TCA TGC TTT CAG TAG

sous forme libre, sous forme alcoylée, sous forme sulfurée ou sous forme de dérivé de la poly L-Lysine.

**5.** Procédé selon l'une quelconque des revendications 1 à 4, caractérisé en ce que le premier nucléotide en 3′ de la chaîne à synthétiser est le premier nucléotide de la séquence - CC ou de la séquence - CC TCA TGC TTT CAG TAG.

**Revendications pour l'Etat contractant suivant : GR**

**1.** Procédé de préparation de la séquence d'oligonucléotides anti-sens, anti-ARN messager du TNF alpha, possèdant la structure de formule (I) suivante :

5' 3'

(d) TCA TGG TGT CCT TTG CAG - X (I)

1 18

dans laquelle X représente un atome d'hydrogène, ou une séquence de 1 à 17 oligonucléotides, sous forme libre, sous forme alcoylée, sous forme sulfurée ou sous forme de dérivé de la poly L-lysine, caractérisé en ce que l'on immobilise sur un support S le premier nucléotide en 3′ de la chaîne à synthétiser de formule :

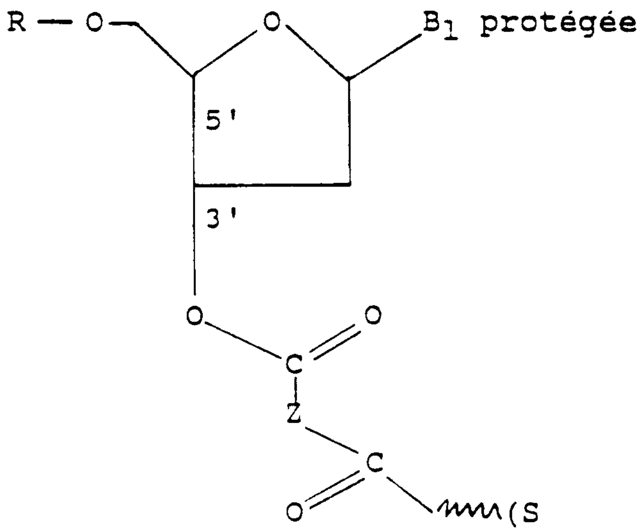

dans laquelle S représente le support, Z représente un groupement hydrocarboné renfermant de 2 à 20 atomes de carbone, $B_1$ est une base purique ou pyrimidique correspondant au premier nucléotide dont la fonction amine est protégée et R est un groupement protecteur,
débloque l'hydroxyle en 5′ par un réactif acide pour obtenir le produit de formule :

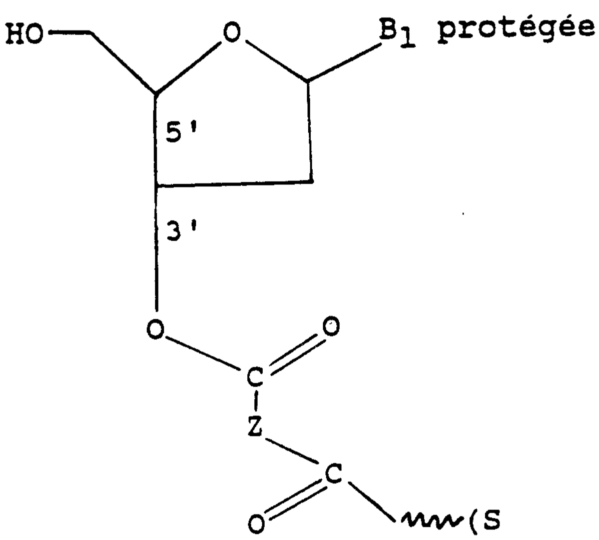

(III)

dans laquelle S, Z et $B_1$ ont la signification déjà indiquée, puis fait réagir ce dernier avec le monomère du second nucléotide de formule :

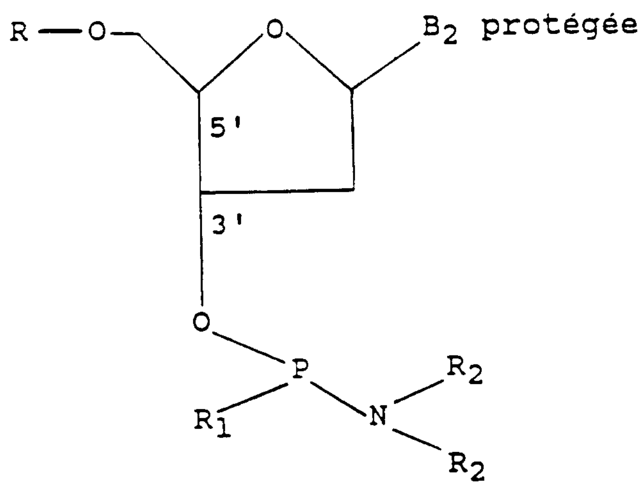

(IV)

dans laquelle R a la signification déjà indiquée, $B_2$ est une base purique ou pyrimidique correspondant au second nucléotide dont la fonction amine est protegée, $R_1$ représente un radical alcoyle ou un groupement -OR'$_1$ ou -SR'$_1$ dans lequel R'$_1$ représente un groupement protecteur, $R_2$ représente un groupement protecteur, pour obtenir un produit de formule :

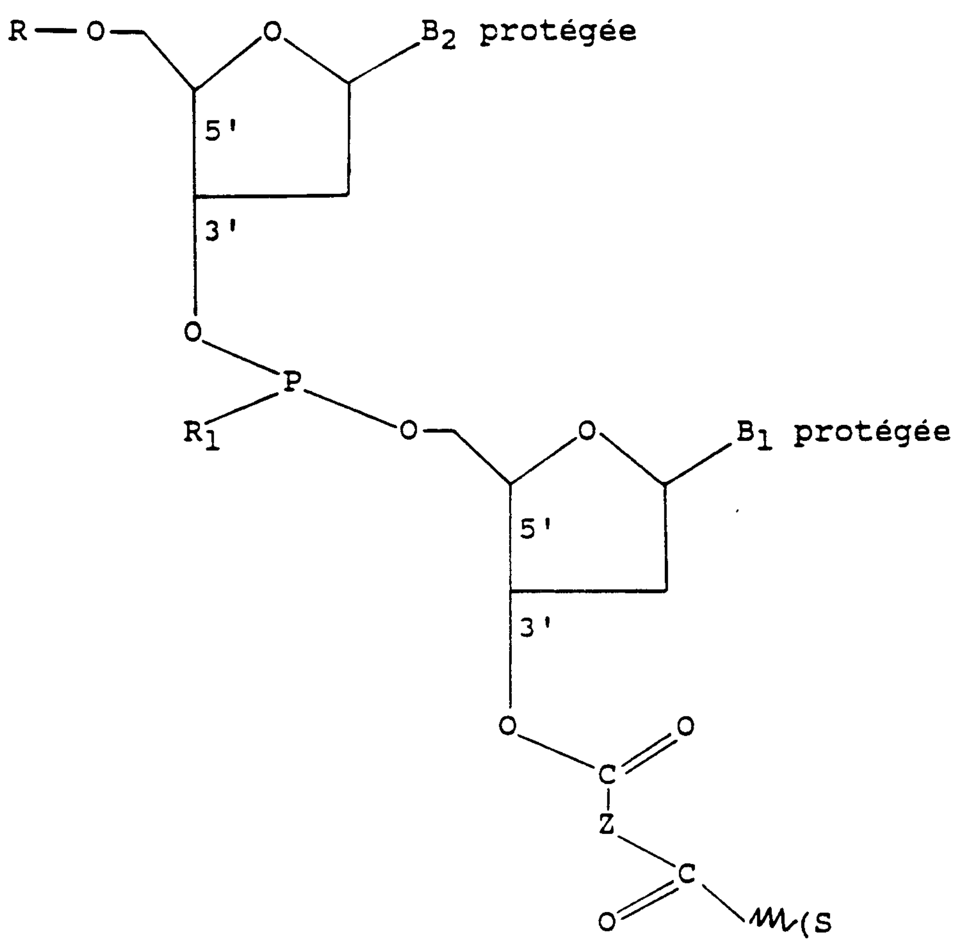

(V)

dans laquelle S, R, $R_1$, $B_1$ et $B_2$ ont la signification déjà indiquée, puis oxyde le produit de formule (V)

obtenu pour obtenir un produit de formule :

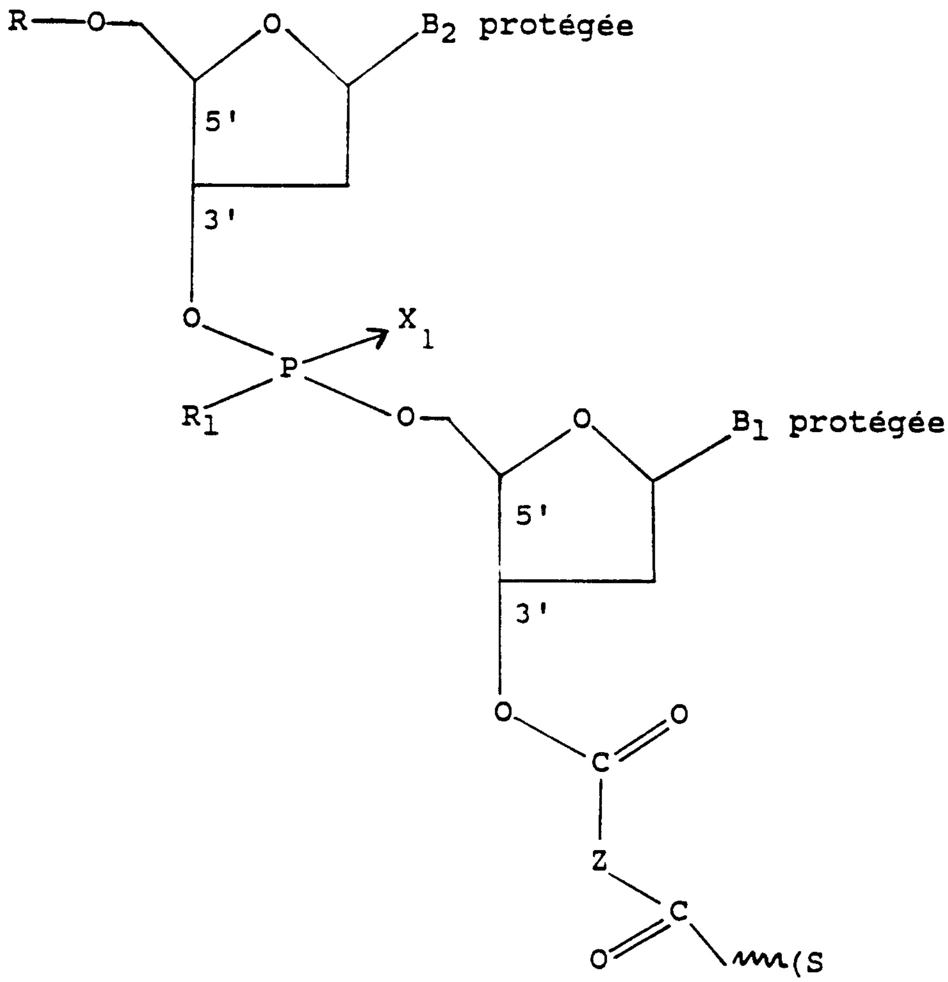

(VI)

dans laquelle S, R, $R_1$, $B_1$, $B_2$ et Z ont la signification déjà indiquée et $X_1$ représente un atome d'oxygène ou de soufre, puis, comme ci-dessus, au départ du produit de formule (II) effectue un nouveau cycle avec ce produit de formule (VI) et le monomère d'un nouveau nucléotide jusqu'à obtention de l'enchaînement désiré pour obtenir finalement un produit de formule :

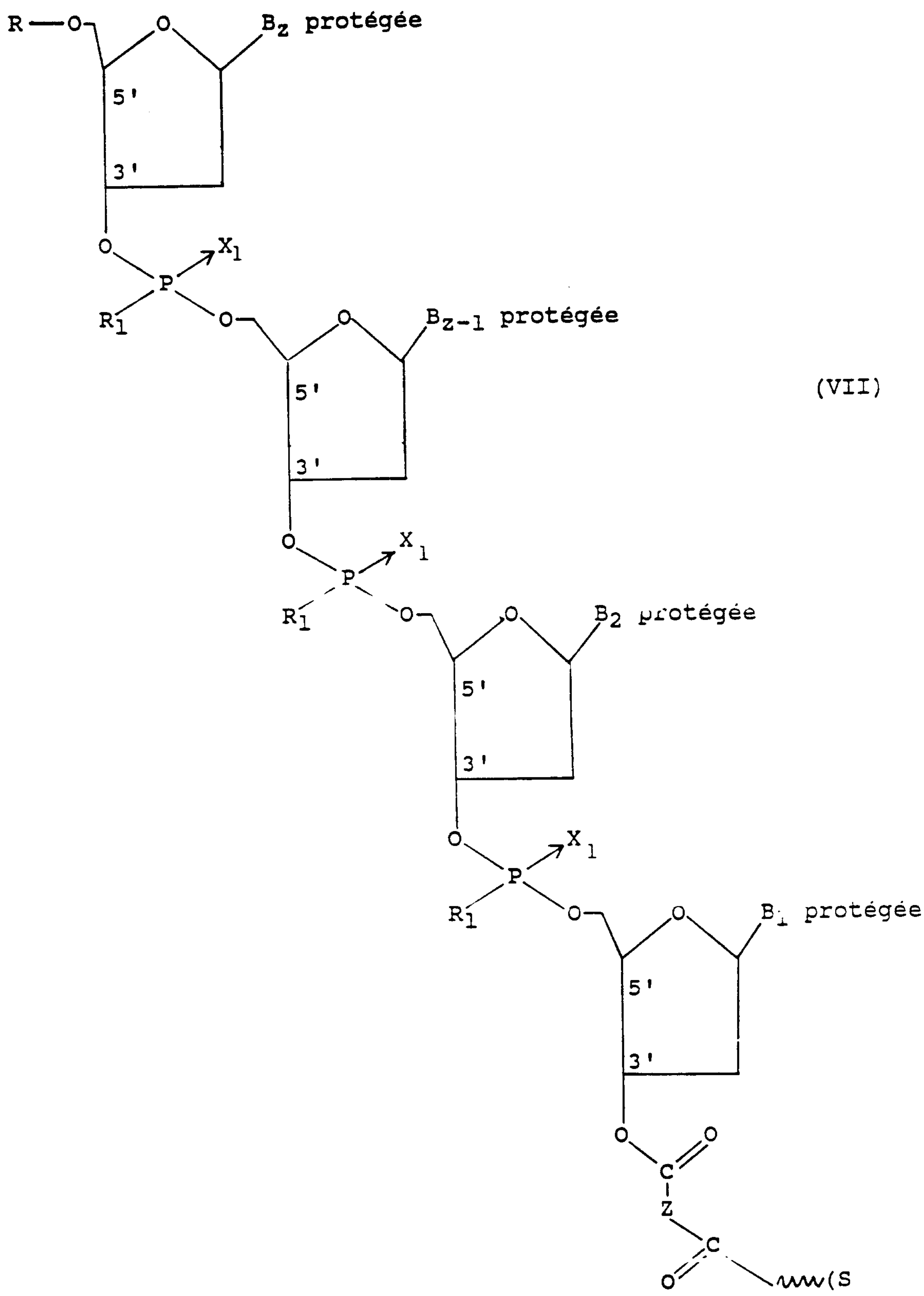

(VII)

dans laquelle S, R, $R_1$, $B_1$, $B_2$, $X_1$ et Z ont la signification dejà indiquée et $B_z$ représente le dernier nucléotide de la séquence, puis déprotège l'oligonucléotide, le sépare du support et purifie le produit de formule (I) ainsi obtenu.

**2.** Procédé selon la revendication 1, caractérise en ce que :

- le déblocage de l'hydroxyle en 5′ du produit de formule (II) est effectué par un réactif acide tel que l'acide acétique, l'acide di ou trichloracétique,
- la réaction de couplage du produit de formule (III) avec le produit de formule (IV) est activée au moyen du tétrazole en opérant dans l'acétonitrile,
- l'oxydation du phosphite de formule (V) en phosphate de formule (VI) est effectuée au moyen de l'iode en solution dans un mélange eau/lutidine/tétrahydrofuranne ou dans un mélange eau/pyridine/tétrahydrofuranne,
- l'oxydation du phosphite de formule (V) pour obtenir la forme sulfurée (VI) est effectuée au moyen du soufre en solution dans un mélange sulfure de carbone, de pyridine et de triéthylamine anhydre,
- en fin de synthèse la déprotection du groupement hydroxyle terminal en 5′ est effectuée au moyen d'un acide tel que l'acide acétique, l'acide di ou trichloracétique,
- la déprotection de l'oligonucléotide de formule (VII) est effectuée au moyen de l'ammoniaque concentré en chauffant modérément le milieu réactionnel.

**3.** Procédé selon la revendicaion 1 pour la préparation des dérivés de la poly L-lysine des séquences d'oligonucléotides telles que définies par la formule (I), procédé caractérisé en ce que l'on fait réagir un produit de formule (VIII) :

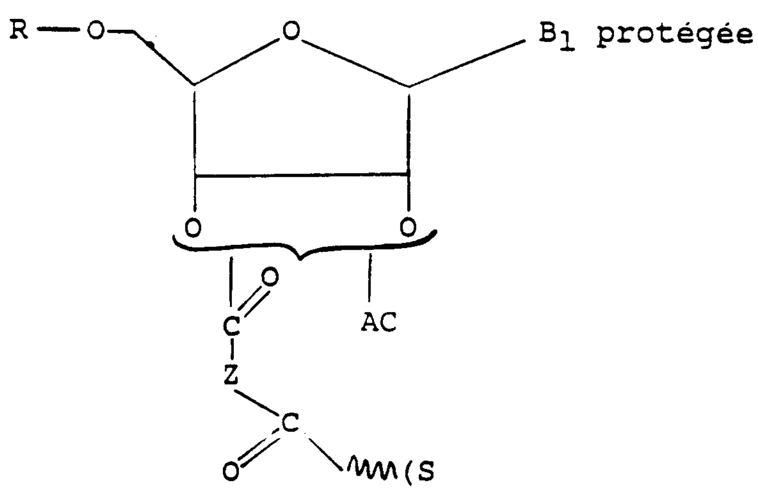

(VIII)

dans laquelle S, Z, R, $B_1$ ont la signification déjà indiquée, avec un produit de formule (IV), pour obtenir un produit de formule (IX) :

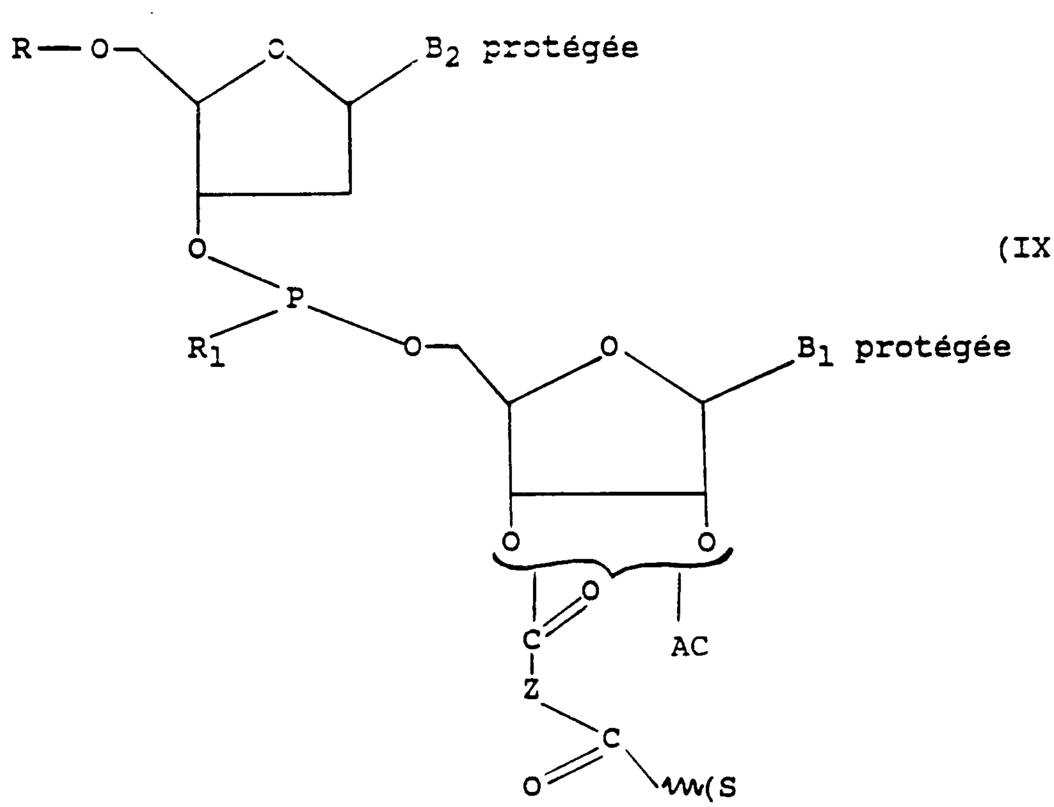

(IX)

dans laquelle S, R, Z, $R_1$, $B_1$ et $B_2$ ont la signification déjà indiquée, oxyde, élimine le support et le groupement activateur pour obtenir un produit de formule (X) :

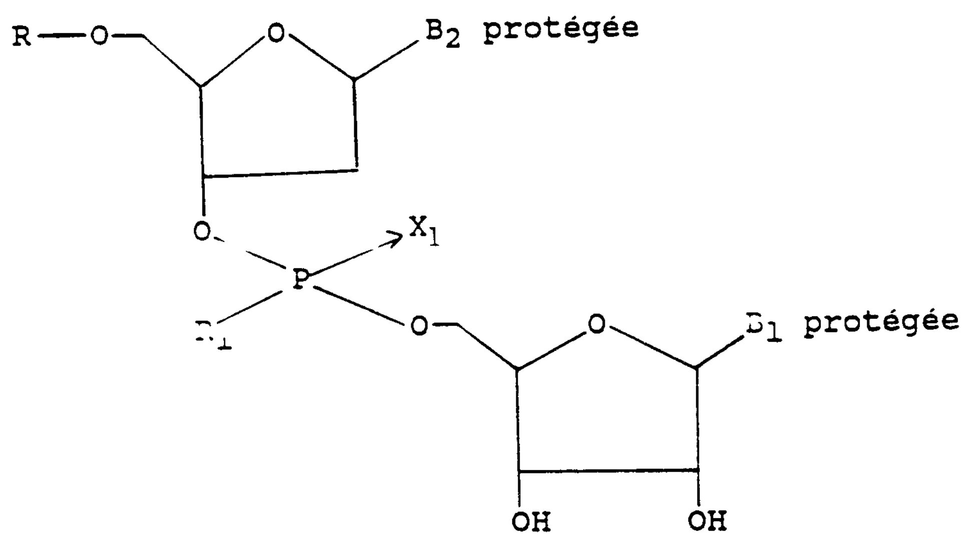

(X)

dans laquelle R, $R_1$, $B_1$ et $B_2$ ont la signification déjà indiquée, $X_1$ représente un atome d'oxygène ou de soufre, oxyde le ribose terminal puis fait réagir avec de la L-lysine en milieu réducteur, pour obtenir le produit de formule (XI) :

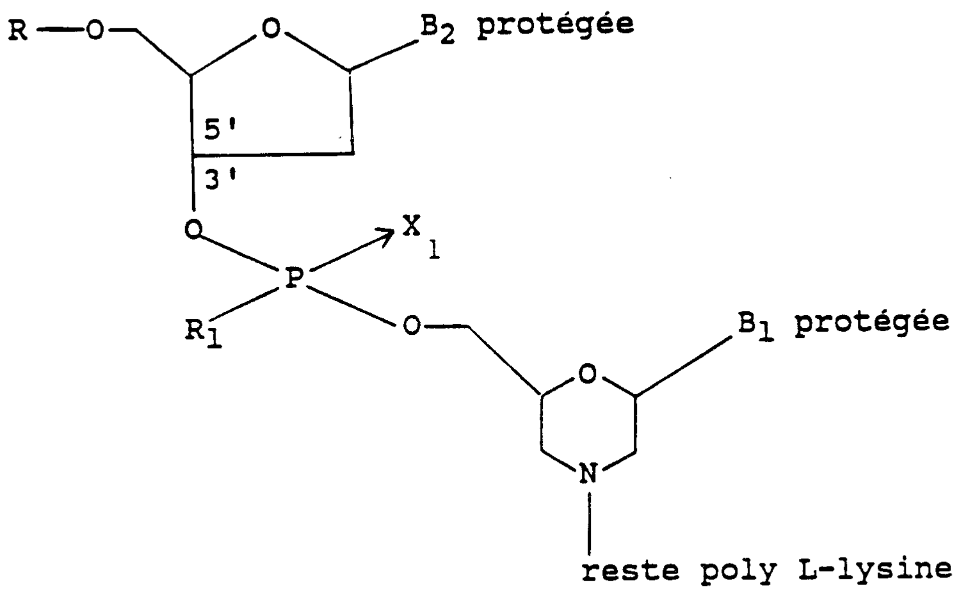

(XI)

dans laquelle R, $R_1$, $B_1$, $B_2$ et $X_1$ ont la signification déjà indiquée, puis poursuit la synthèse comme au départ du produit de formule (VI).

**4.** Procédé selon la revendication 1, 2 ou 3, caractérisé en ce que le premier nucléotide en 3′ de la chaîne à synthétiser est soit le premier nucléotide de la séquence finale

3'
TCA TGG TGT CCT TTG CAG

soit l'un des nucléotides de la séquence de formule ($I_A$) :
- CC TCA TGC TTT CAG TAG

sous forme libre, sous forme alcoylée, sous forme sulfurée ou sous forme de dérivé de la poly L-Lysine.

**5.** Procédé selon l'une quelconque des revendications 1 à 4, caractérisé en ce que le premier nucléotide en 3′ de la chaîne à synthétiser est le premier nucléotide de la séquence - CC ou de la séquence - CC TCA TGC TTT CAG TAG.

**6.** Procédé selon l'une quelconque des revendications 1 à 4, caractérisé en ce que l'on prépare la séquence d'oligonucléotides répondant à la formule :

5' 3'
(d) TCA TGG TGT CCT TTG CAG CC
1 20

sous forme libre, sous forme alcoylée, sous forme sulfurée ou sous forme de dérivé de la poly L-lysine.

**7.** Procédé selon l'une quelconque des revendications 1 à 4, caractérisé en ce que l'on prépare la séquence d'oligonucléotides répondant à la formule :

```
      5'                    3'
(d)   TCA TGG TGT CCT TTG CAG
      1                     18
```

sous forme libre, sous forme alcoylée, sous forme sulfurée ou sous forme de dérivé de la poly L-lysine.

**8.** Procédé selon l'une quelconque des revendications 1 à 4, caractérisé en ce que l'on prépare la séquence d'oligonucléotides répondant à la formule :

```
      5'                                                3'
(d)   TCA TGG TGT CCT TTG CAG CC TCA TGC TTT CAG TAG
      1                                                 35
```

sous forme libre, sous forme alcoylée, sous forme sulfurée ou sous forme de dérivé de la poly L-lysine.

**9.** Procédé de préparation de compositions pharmaceutiques, caractérisé en ce que l'on met à titre de principe actif l'une au moins des nouvelles séquences d'oligonucléotides telles que définies à la revendication 1.

**10.** Procédé de préparation de compositions pharmaceutiques, caractérisé en ce que l'on met à titre de principe actif l'une au moins des nouvelles séquences d'oligonucléotides telles que définies à l'une quelconque des revendications 2 à 8.

**Claims**
**Claims for the following Contracting States : AT, BE, CH, DE, DK, FR, GB, IT, LI, LU, NL, SE**

**1.** Anti-sense oligonucleotide sequence, anti-messenger RNA of alpha TNF, characterized in that it has the following structure of formula (I):

```
      5'                    3'
(d)   TCA TGG TGT CCT TTG CAG - X                    (I)
      1                     18
```

in which X represents a hydrogen atom, or a sequence of 1 to 17 oligonucleotides,
in free form, in alkylated form, in sulphurated form or in the form of a poly L-lysine derivative.

**2.** Oligonucleotide sequence corresponding to formula (I) of claim 1, characterized in that X represents a hydrogen atom or all or part of the sequence of formula ($I_A$):

```
   5'                      3'
   - CC TCA TGC TTT CAG TAG                          (IA)
     19                    35
```

in free form, in alkylated form, in sulphurated form, or in the form of a poly L-lysine derivative.

**3.** Oligonucleotide sequence as defined by formula (I) of claim 1, characterized in that in the said formula (I), X represents a hydrogen atom, a sequence of formula - CC or the sequence of formula ($I_A$), in free form, in alkylated form, in sulphurated form or in the form of a poly L-lysine derivative.

**4.** Oligonucleotide sequence according to any one of claims 1 to 3, characterized in that it corresponds to the formula:

```
    5'                         3'
(d) TCA TGG TGT CCT TTG CAG CC
    1                          20
```

in free form, in alkylated form, in sulphurated form or in the form of a poly L-lysine derivative.

**5.** Oligonucleotide sequence according to any one of claims 1 to 3, characterized in that it corresponds to the formula:

```
    5'                      3'
(d) TCA TGG TGT CCT TTG CAG
    1                       18
```

in free form, in alkylated form, in sulphurated form or in the form of a poly L-lysine derivative.

**6.** Oligonucleotide sequence according to any one of claims 1 to 3, characterized in that it corresponds to the formula:

```
    5'                                             3'
(d) TCA TGG TGT CCT TTG CAG CC TCA TGC TTT CAG TAG
    1                                              35
```

in free form, in alkylated form, in sulphurated form or in the form of a poly L-lysine derivative.

**7.** Preparation process for the oligonucleotide sequence as defined by formula (I) of claim 1, characterized in that the first nucleotide in position 3' of the chain to be synthesized of formula:

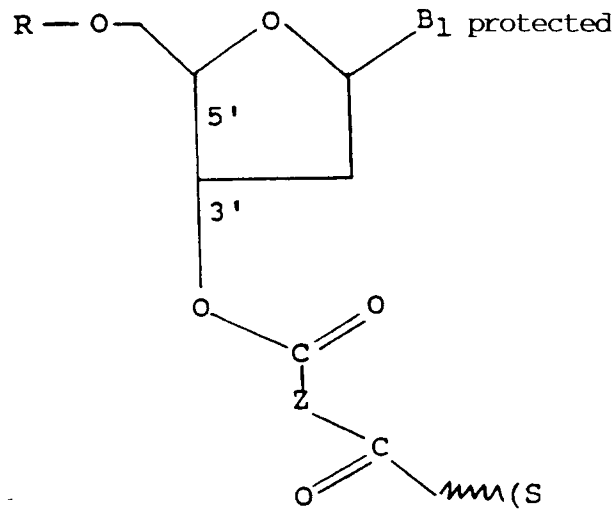

(II)

in which S represents the support, Z represents a hydrocarbon group containing 2 to 20 carbon atoms, $B_1$ is a purine or pyrimidine base corresponding to the first nucleotide the amine function of which is protected and R is a protector group, is immobilized on a support S,
the hydroxyl in position 5' is unblocked by an acid reagent in order to obtain the product of formula:

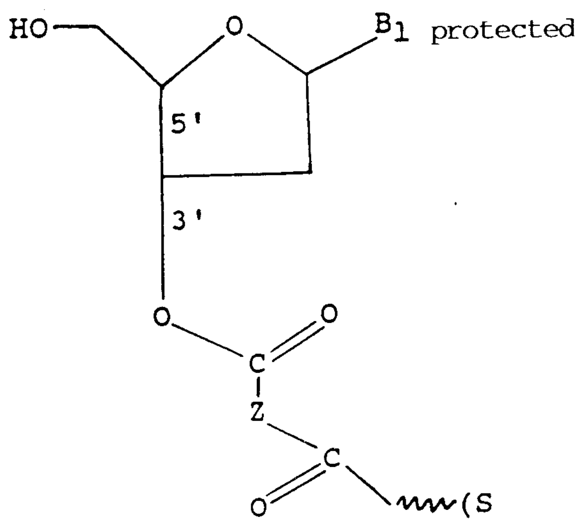

(III)

in which S, Z and $B_1$ have the meaning already indicated, then the latter is reacted with the monomer of the second nucleotide of formula:

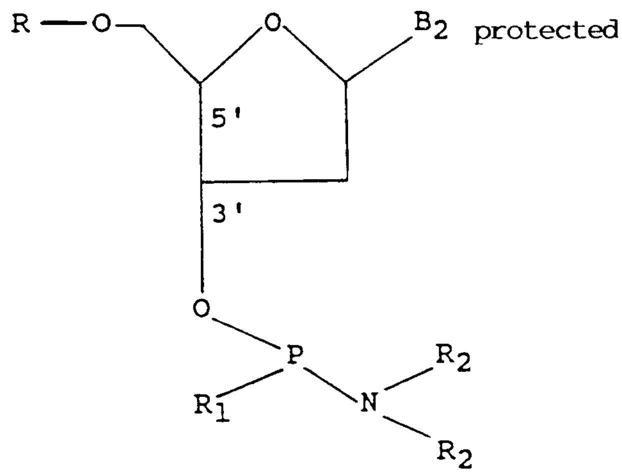

(IV)

in which R has the meaning already indicated, $B_2$ is a purine or pyrimidine base corresponding to the second nucleotide the amine function of which is protected, $R_1$ represents an alkyl radical or an -OR'$_1$ or -SR'$_1$ group in which R'$_1$ represents a protector group, $R_2$ represents a protector group, in order to obtain a product of formula:

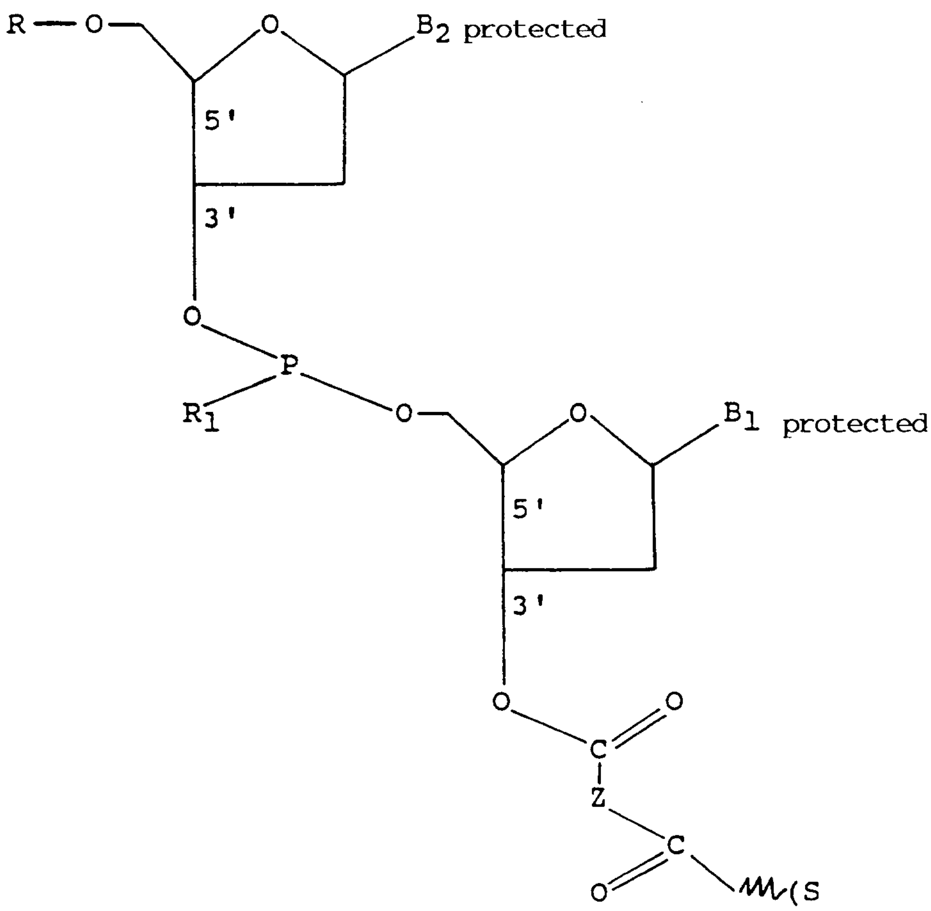

(V)

in which S, R, $R_1$, $B_1$ and $B_2$ have the meaning already indicated, then the product of formula (V) obtained is oxidized in order to obtain a product of formula:

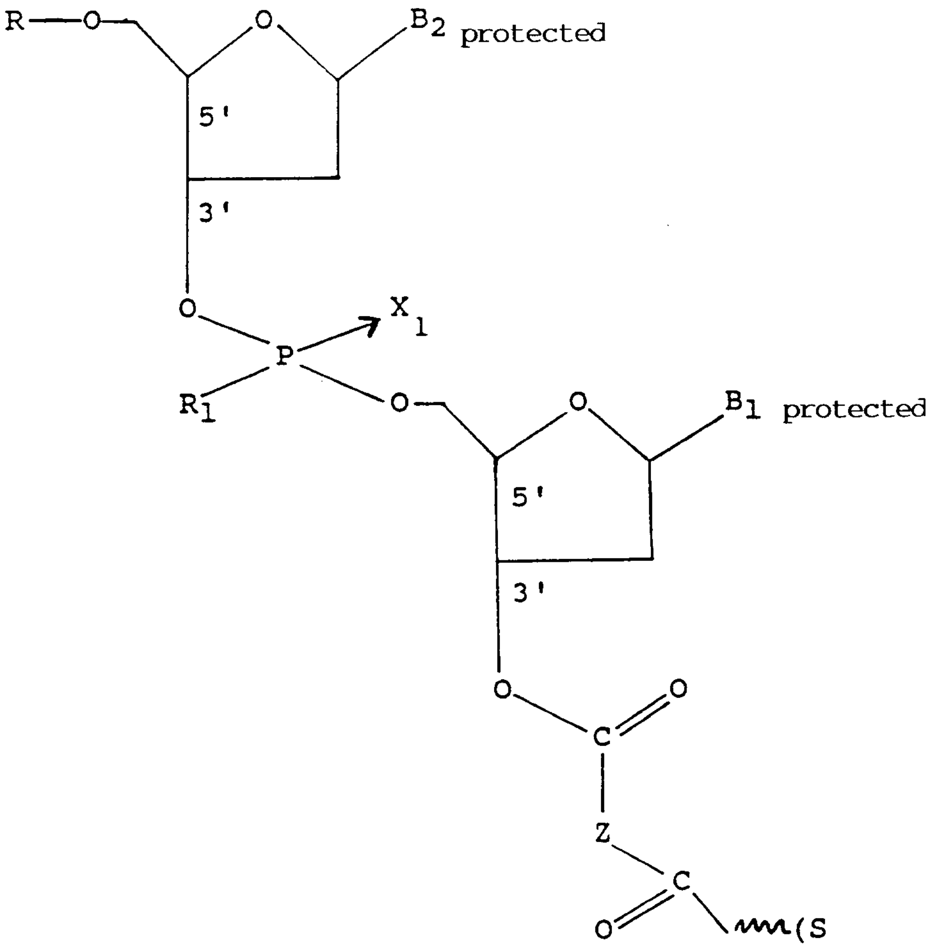

(VI)

in which S, R, $R_1$, $B_1$, $B_2$ and Z have the meaning already indicated and $X_1$ represents an oxygen or sulphur atom, then, as above, starting with the product of formula (II), a new cycle is carried out with this product of formula (VI) and the monomer of a new nucleotide until the desired chain formation is obtained, in order to obtain finally a product of formula:

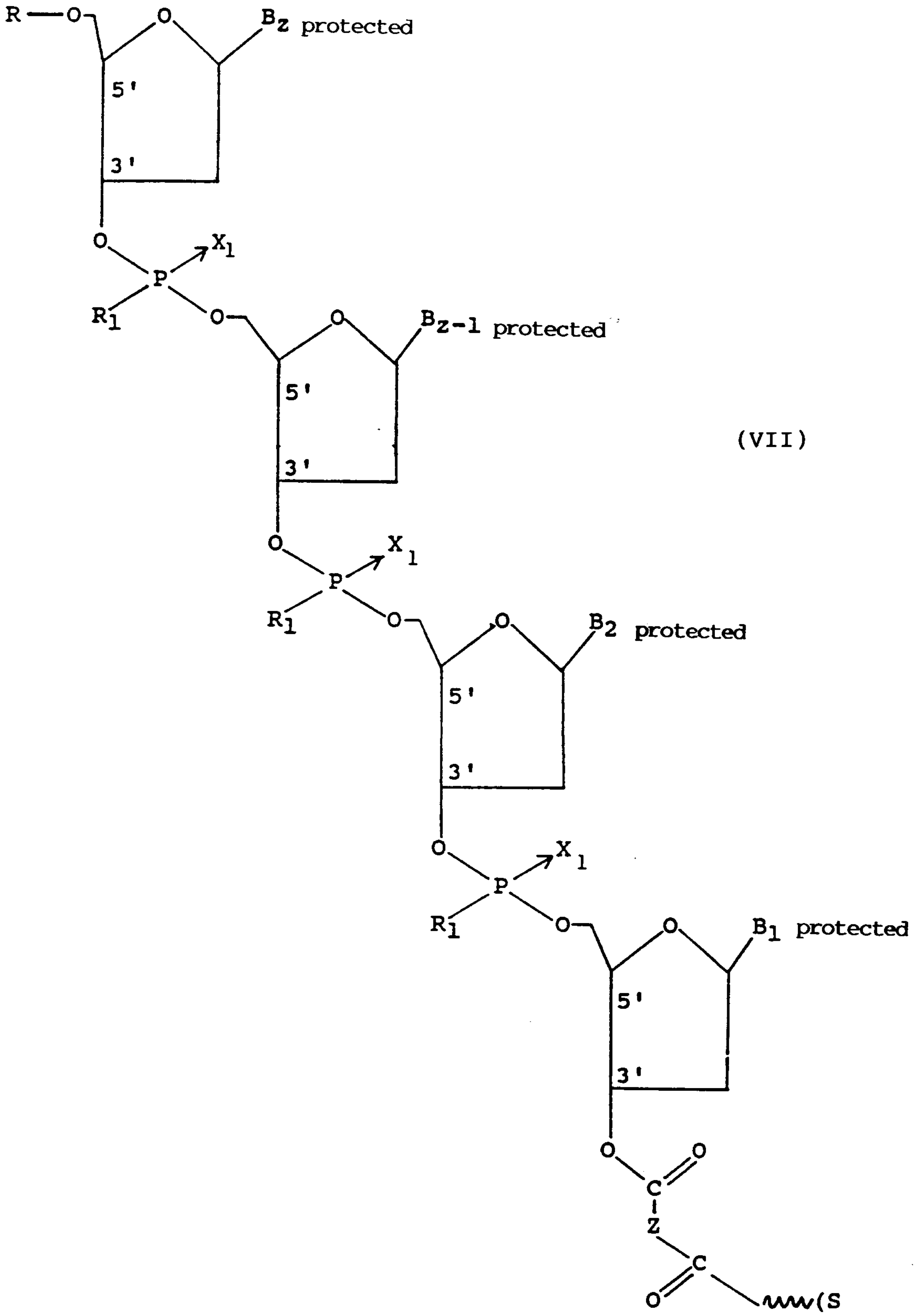

(VII)

in which S, R, $R_1$, $B_1$, $B_2$, $X_1$ and Z have the meaning already indicated and Bz represents the last nucleotide of the sequence, then the oligonucleotide is deprotected, separated from the support and the product of formula (I) thus obtained is purified.

**8.** Process according to claim 7, characterized in that:

- the unblocking of the hydroxyl in position 5' of the product of formula (II) is carried out by an acid reagent such as acetic acid, di- or trichloracetic acid,
- the coupling reaction of the product of formula (III) with the product of formula (IV) is activated using tetrazole operating in acetonitrile,
- the oxidation of the phosphite of formula (V) into the phosphate of formula (VI) is carried out using iodine in solution in a water/lutidine/tetrahydrofuran mixture or in a water/pyridine/tetrahydrofuran mixture,
- the oxidation of the phosphite of formula (V) in order to obtain the sulphurated form (VI) is carried out using sulphur in solution in a mixture of carbon sulphide, pyridine and anhydrous triethylamine,
- at the end of the synthesis the deprotection of the terminal hydroxyl group in position 5' is carried out using an acid such as acetic acid, di- or trichloracetic acid,
- the deprotection of the oligonucleotide of formula (VII) is carried out using concentrated ammonium hydroxide by moderately heating the reaction medium.

**9.** Process according to claim 7 for the preparation of the poly L-lysine derivatives of the oligonucleotide sequences as defined by formula (I), process characterized in that a product of formula (VIII):

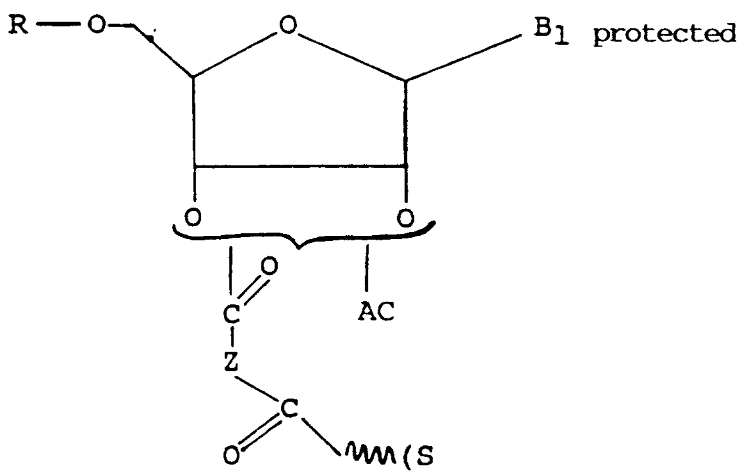

(VIII)

in which S, Z, R, $B_1$ have the meaning already indicated, is reacted with a product of formula (IV), in order to obtain a product of formula (IX):

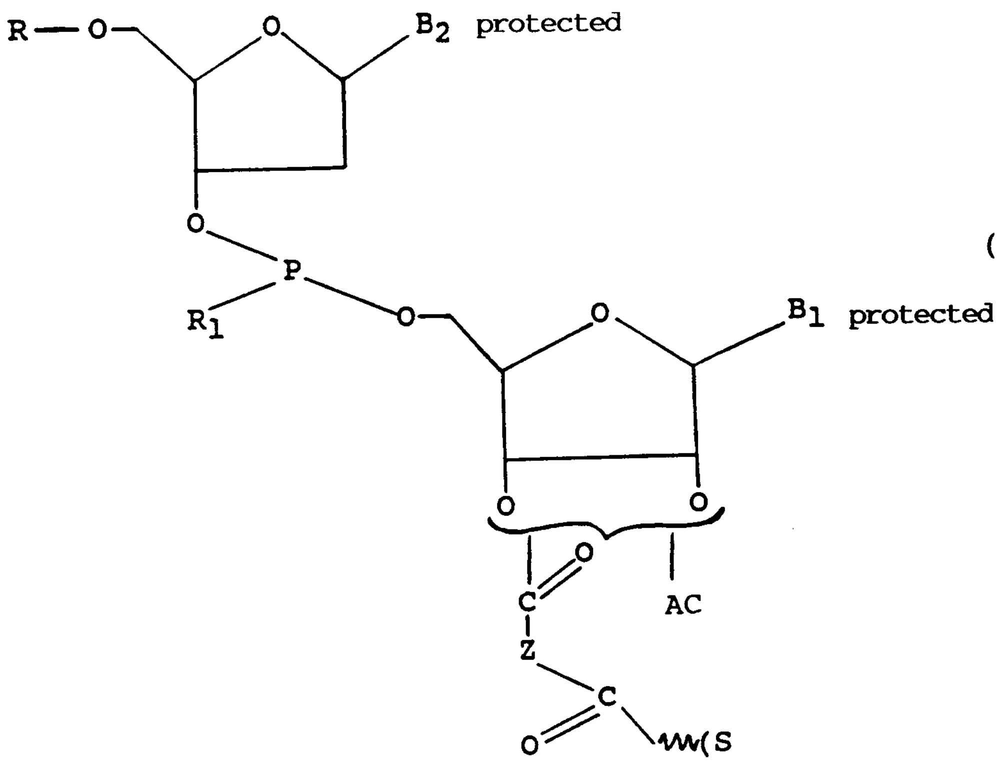

(IX)

in which S, R, Z, $R_1$, $B_1$ and $B_2$ have the meaning already indicated, oxidation is carried out, the support and the activator group are eliminated in order to obtain a product of formula (X):

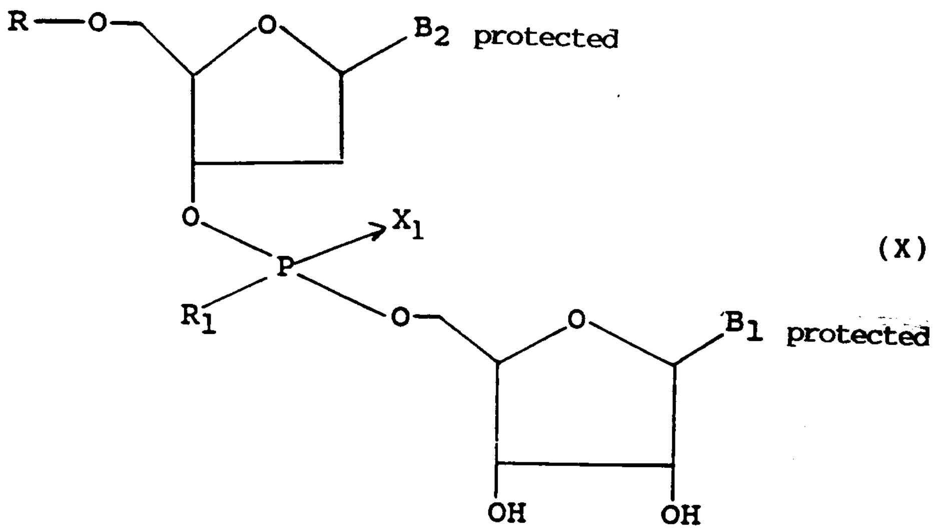

(X)

in which R, $R_1$, $B_1$ and $B_2$ have the meaning already indicated, $X_1$ represents an oxygen or sulphur atom, the terminal ribose is oxidized then reacted with L-lysine in a reducing medium, in order to obtain the product of formula (XI):

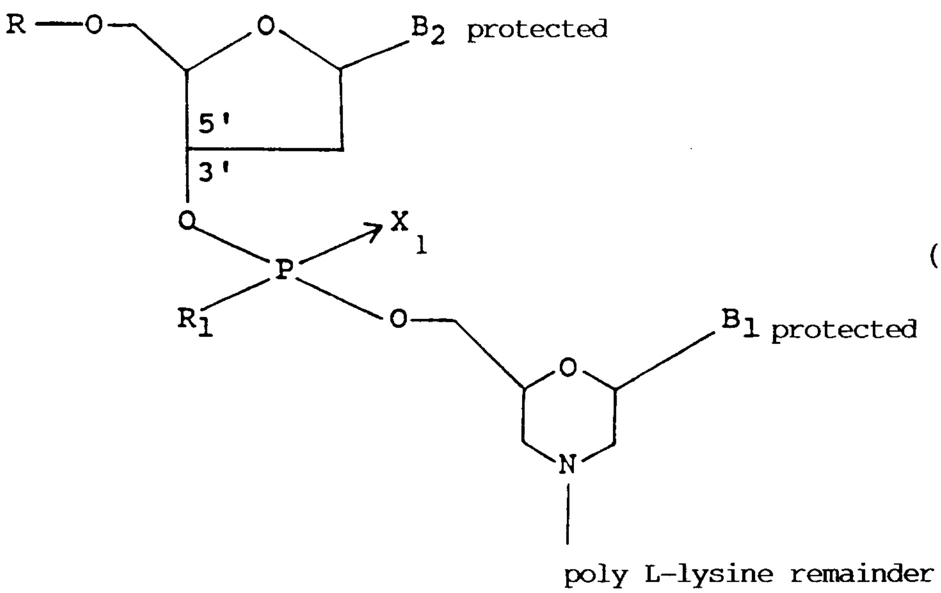

(XI)

in which R, $R_1$, $B_1$, $B_2$ and $X_1$ have the meaning already indicated, then the synthesis is continued as from the product of formula (VI).

**10.** Medicaments, characterized in that they are constituted by new oligonucleotide sequences as defined in claim 1.

**11.** Medicaments, characterized in that they are constituted by new oligonucleotide sequences as defined in any one of claims 2 to 6.

**12.** Pharmaceutical compositions, characterized in that they contain, as active ingredient, at least one of the medicaments as defined in any one of claims 10 or 11.

**Claims for the following Contracting State : ES**

**1.** Preparation process for the anti-sense oligonucleatide sequence, anti-messenger RNA of alpha TNF, having the following structure of formula (I):

```
     5'                         3'
(d)  TCA  TGG  TGT  CCT  TTG  CAG - X          (I)
     1                          18
```

in which X represents a hydrogen atom, or a sequence of 1 to 17 oligonucleotides,
in free form, in alkylated form, in sulphurated form or in the form of a derivative of poly L-lysine, characterized in that the first nucleotide in position 3' of the chain to be synthesized of formula:

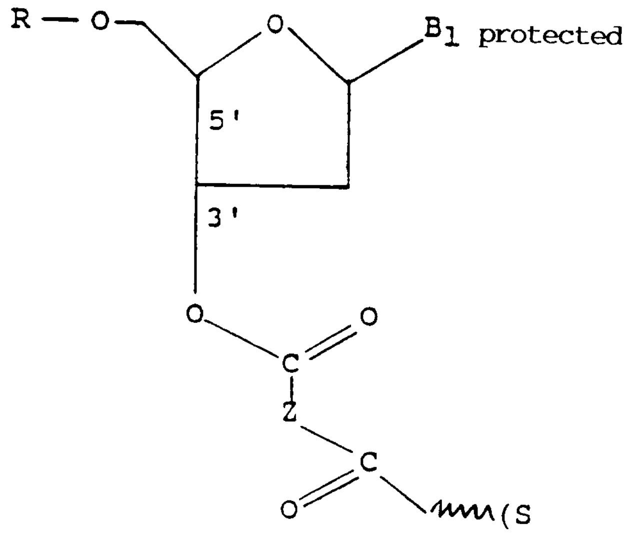

(II)

in which S represents the support, Z represents a hydrocarbon group containing 2 to 20 carbon atoms, $B_1$ is a purine or pyrimidine base corresponding to the first nucleotide the amine function of which is protected and R is a protector group, is immobilized on a support S,
the hydroxyl in position 5' is unblocked by an acid reagent in order to obtain the product of formula:

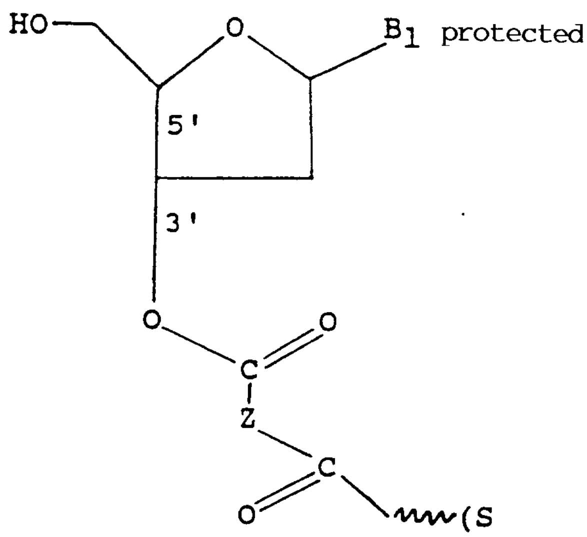

(III)

in which S, Z and $B_1$ have the meaning already indicated, then the latter is reacted with the monomer of the second nucleotide of formula:

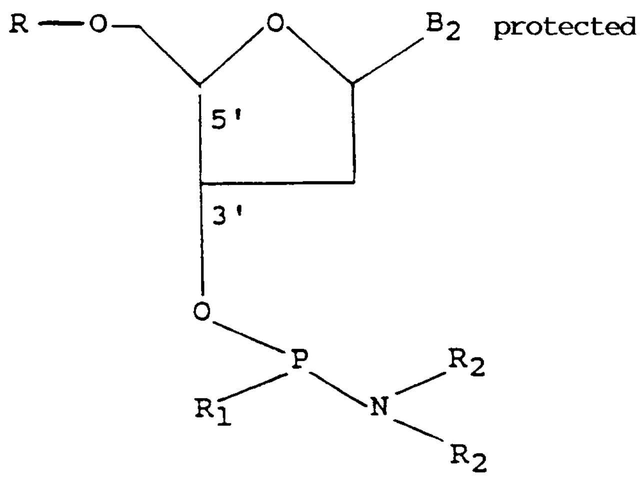

(IV)

in which R has the meaning already indicated, $B_2$ is a purine or pyrimidine base corresponding to the second nucleotide the amine function of which is protected, $R_1$ represents an alkyl radical or an $-OR'_1$ or $-SR'_1$ group in which $R'_1$ represents a protector group, $R_2$ represents a protector group, in order to obtain a product of formula:

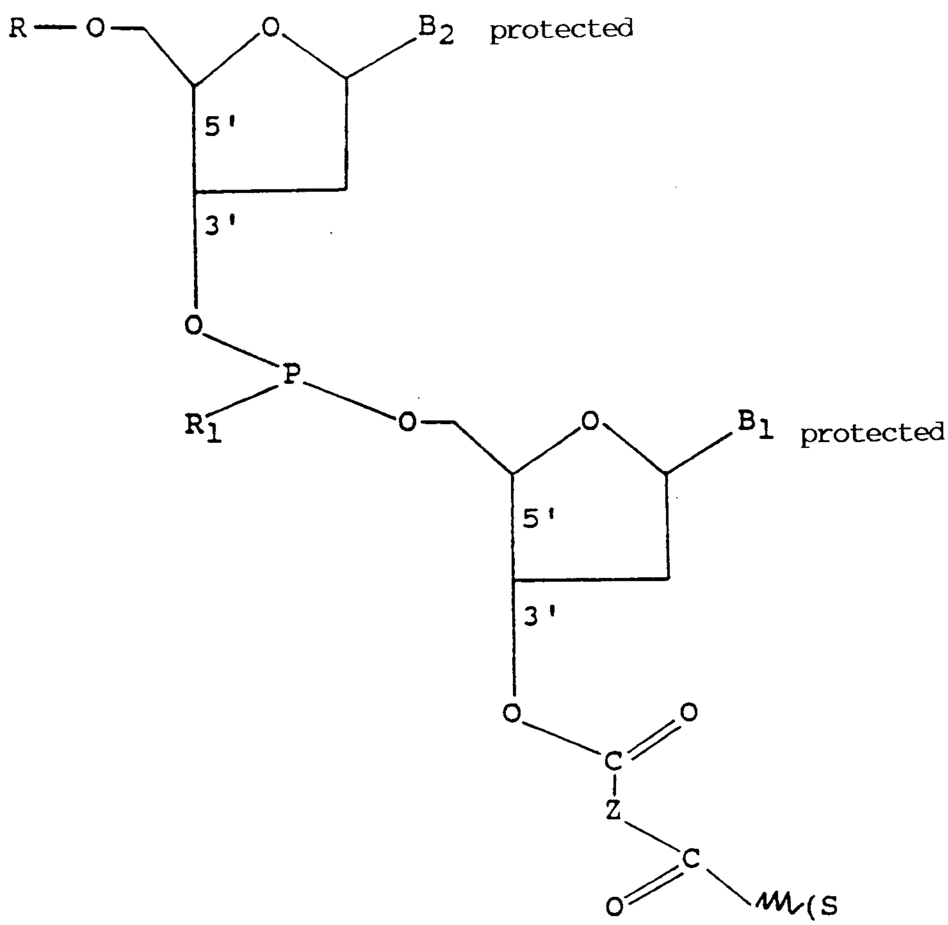

(V)

in which S, R, $R_1$, $B_1$ and $B_2$ have the meaning already indicated, then the product of formula (V) obtained is oxidized in order to obtain a product of formula:

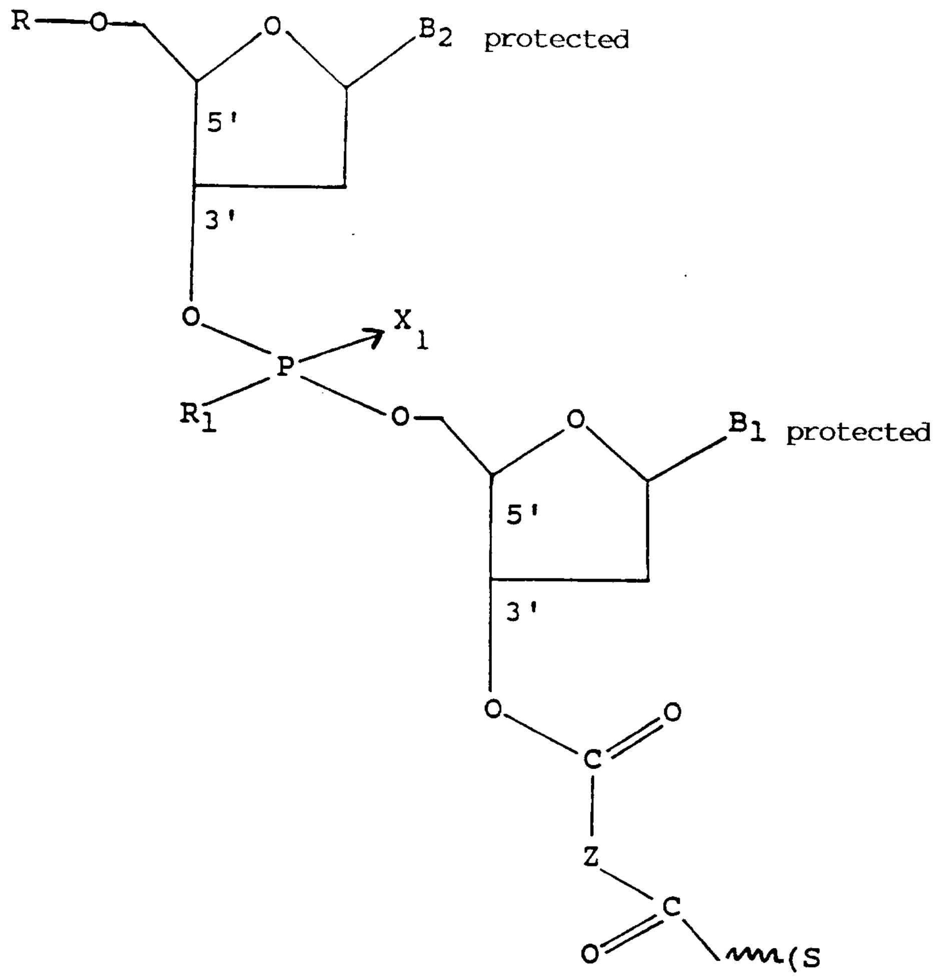

(VI)

in which S, R, $R_1$, $B_1$, $B_2$ and Z have the meaning already indicated and $X_1$ represents an oxygen or sulphur atom, then, as above, starting with the product of formula (II), a new cycle is carried out with this product of formula (VI) and the monomer of a new nucleotide until the desired chain formation is obtained, in order to obtain finally a product of formula:

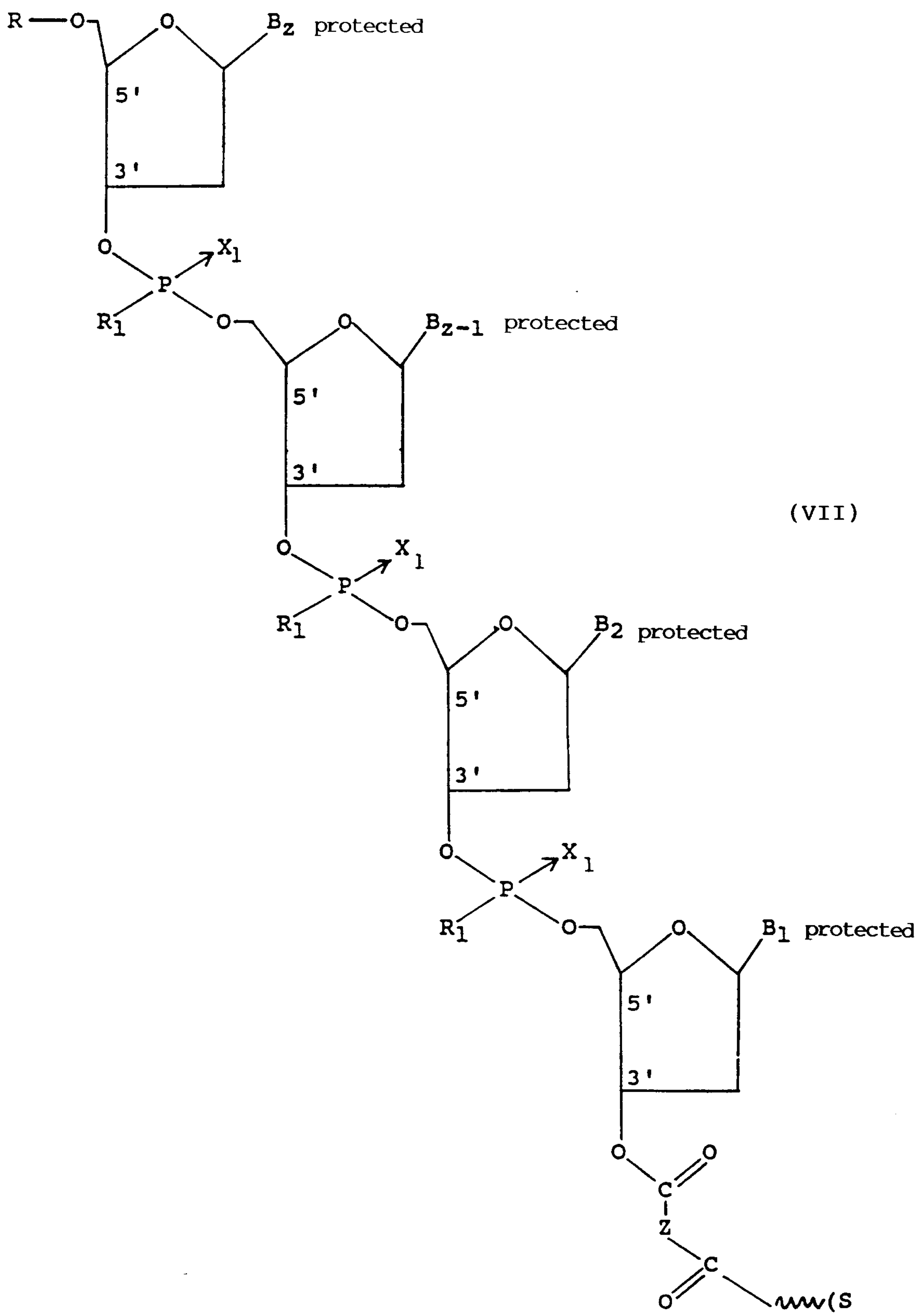

(VII)

in which S, R, $R_1$, $B_1$, $B_2$, $X_1$ and Z have the meaning already indicated and Bz represents the last nucleotide of the sequence, then the oligonucleotide is deprotected, separated from the support and the product of formula (I) thus obtained is purified.

**2.** Process according to claim 1, characterized in that:

- the unblocking of the hydroxyl in position 5' of the product of formula (II) is carried out by an acid reagent such as acetic acid, di- or trichloracetic acid,
- the coupling reaction of the product of formula (III) with the product of formula (IV) is activated using tetrazole operating in acetonitrile,
- the oxidation of the phosphite of formula (V) into the phosphate of formula (VI) is carried out using iodine in solution in a water/lutidine/tetrahydrofuran mixture or in a water/pyridine/tetrahydrofuran mixture,
- the oxidation of the phosphite of formula (V) in order to obtain the sulphurated form (VI) is carried out using sulphur in solution in a mixture of carbon sulphide, pyridine and anhydrous triethylamine,
- at the end of the synthesis the deprotection of the terminal hydroxyl group in position 5' is carried out using an acid such as acetic acid, di- or trichloracetic acid,
- the deprotection of the oligonucleotide of formula (VII) is carried out using concentrated ammonium hydroxide by moderately heating the reaction medium.

**3.** Process according to claim 1 for the preparation of the poly L-lysine derivatives of the oligonucleotide sequences as defined by formula (I), process characterized in that a product of formula (VIII):

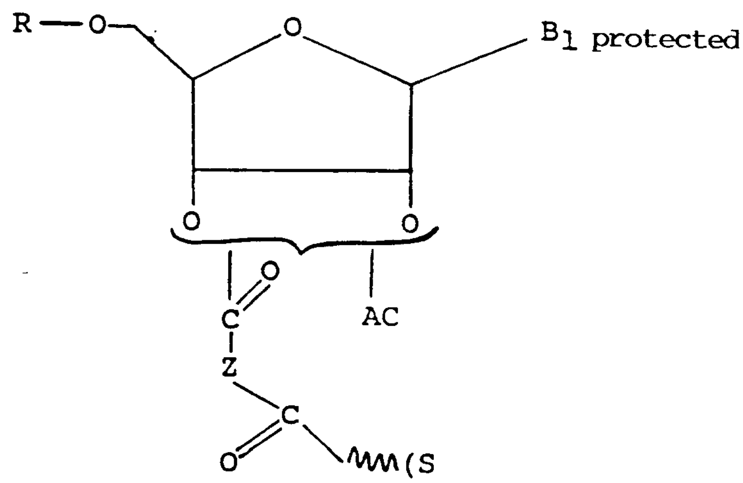

(VIII)

in which S, Z, R, $B_1$ have the meaning already indicated, is reacted with a product of formula (IV), in order to obtain a product of formula (IX):

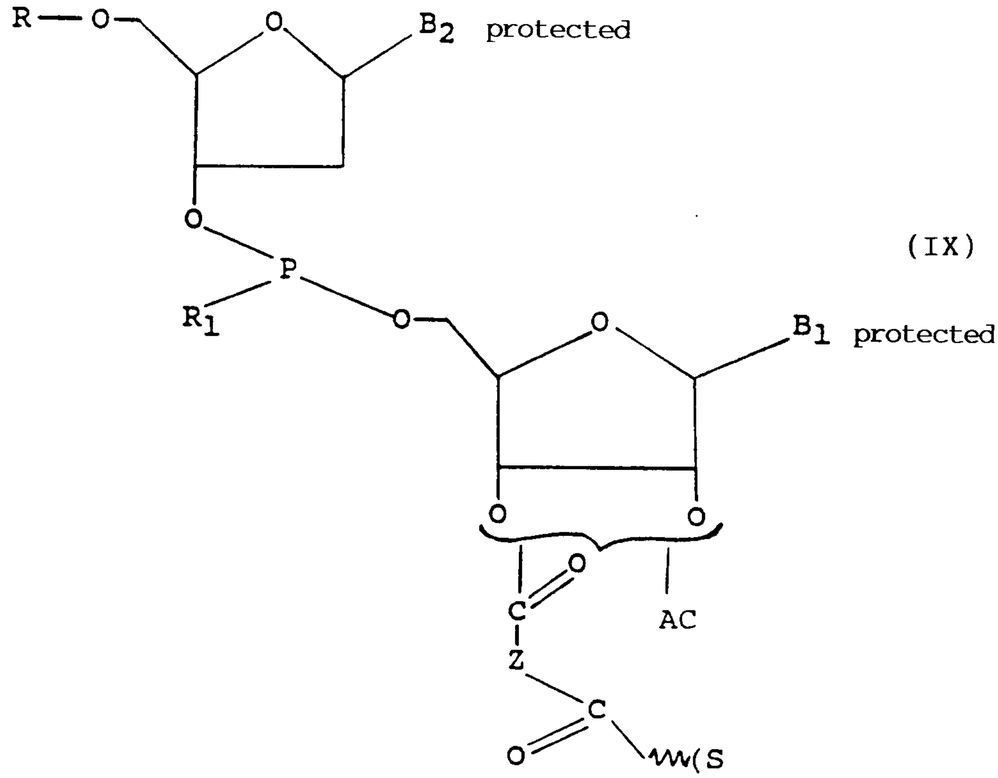

(IX)

in which S, R, Z, $R_1$, $B_1$ and $B_2$ have the meaning already indicated, oxidation is carried out, the support and the activator group are eliminated in order to obtain a product of formula (X):

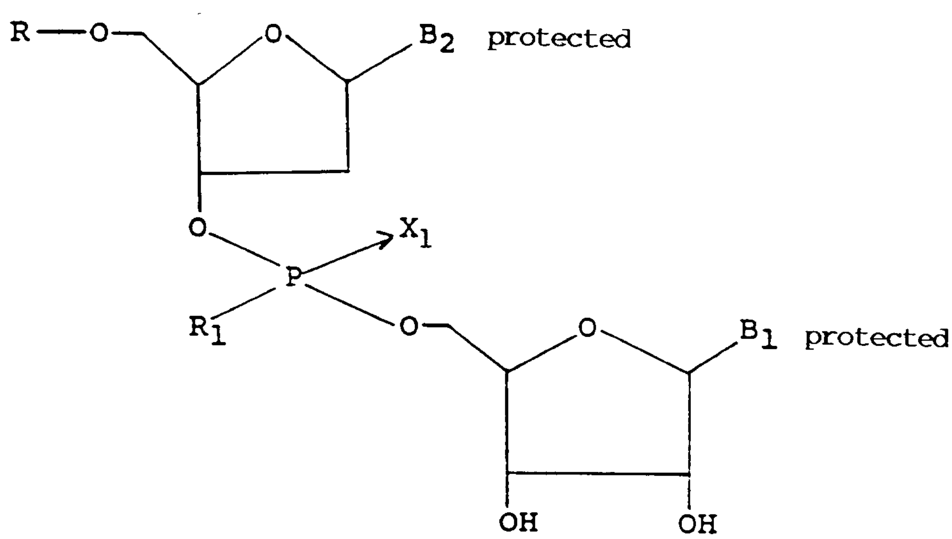

(X)

in which R, $R_1$, $B_1$ and $B_2$ have the meaning already indicated, $X_1$ represents an oxygen or sulphur atom, the terminal ribose is oxidized then reacted with L-lysine in a reducing medium, in order to obtain the product of formula (XI):

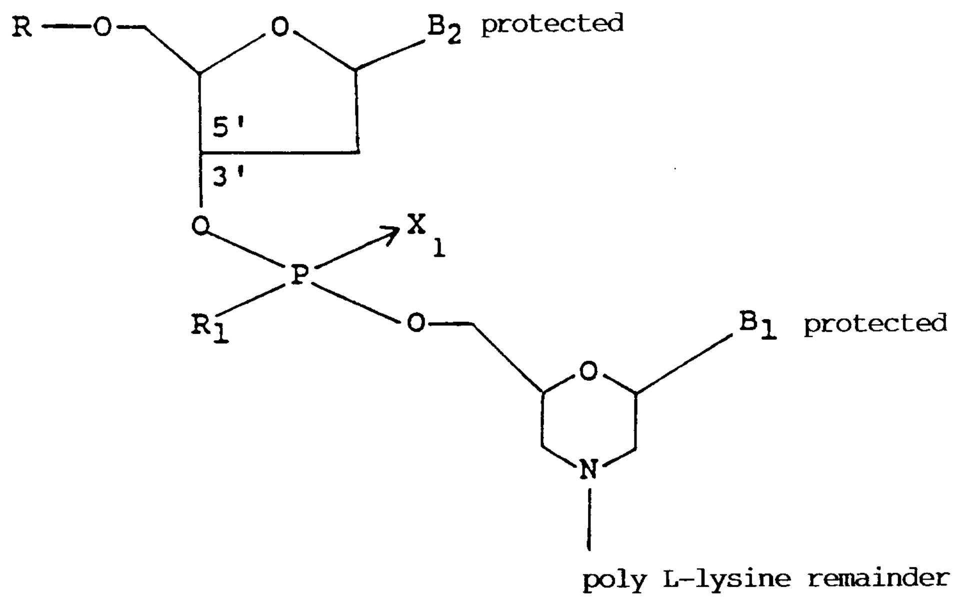

(XI)

in which R, $R_1$, $B_1$, $B_2$ and $X_1$ have the meaning already indicated, then the synthesis is continued as from the product of formula (VI).

**4.** Process according to claim 1, 2 or 3, characterized in that the first nucleotide in position 3' of the chain to be synthesized is either the first nucleotide of the final sequence

3′
TCA TGG TGT CCT TTG CAG

or one of the nucleotides of the sequence of formula ($I_A$):
- CC TCA TGC TTT CAG TAG
in free form, in alkylated form, in sulphurated form or in the form of a poly L-lysine derivative.

**5.** Process according to any one of claims 1 to 4, characterized in that the first nucleotide in position 3' of the chain to be synthesized is the first nucleotide of the - CC sequence or the - CC TCA TGC TTT CAG TAG sequence.

**Claims for the following Contracting State : GR**

**1.** Preparation process for the anti-sense oligonucleatide sequence, anti-messenger RNA of alpha TNF, having the following structure of formula (I):

```
     5′                    3′
(d)  TCA  TGG  TGT  CCT  TTG  CAG  -  X        (I)
     1                     18
```

in which X represents a hydrogen atom, or a sequence of 1 to 17 oligonucleotides,
in free form, in alkylated form, in sulphurated form or in the form of a derivative of poly L-lysine, characterized in that the first nucleotide in position 3' of the chain to be synthesized of formula:

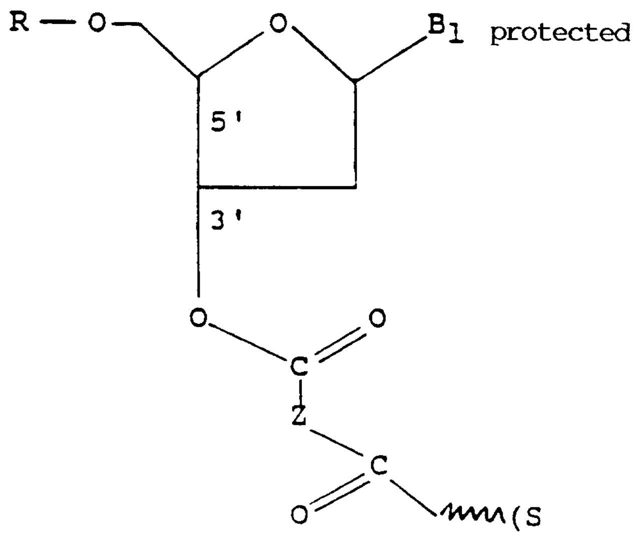

(II)

in which S represents the support, Z represents a hydrocarbon group containing 2 to 20 carbon atoms, $B_1$ is a purine or pyrimidine base corresponding to the first nucleotide the amine function of which is protected and R is a protector group, is immobilized on a support S,
the hydroxyl in position 5' is unblocked by an acid reagent in order to obtain the product of formula:

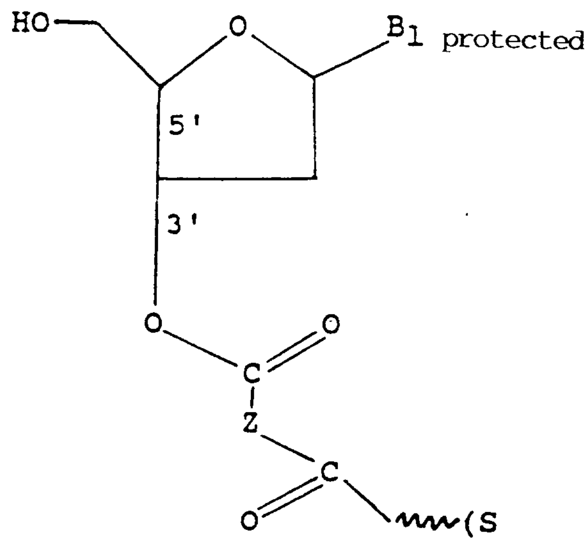

(III)

in which S, Z and $B_1$ have the meaning already indicated, then the latter is reacted with the monomer of the second nucleotide of formula:

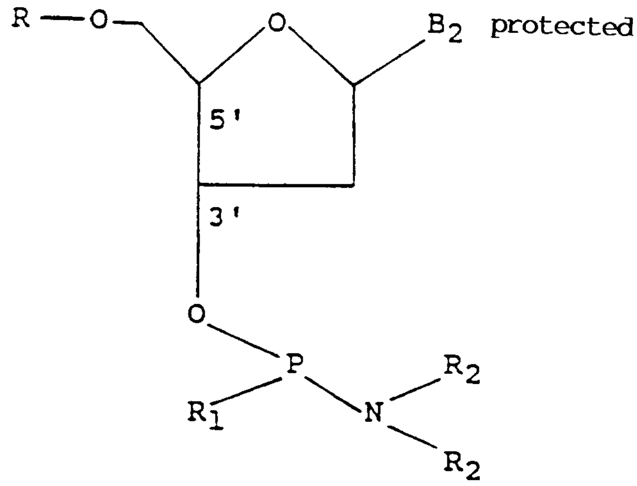

(IV)

in which R has the meaning already indicated, $B_2$ is a purine or pyrimidine base corresponding to the second nucleotide the amine function of which is protected, $R_1$ represents an alkyl radical or an $-OR'_1$ or $-SR'_1$ group in which $R'_1$ represents a protector group, $R_2$ represents a protector group, in order to obtain a product of formula:

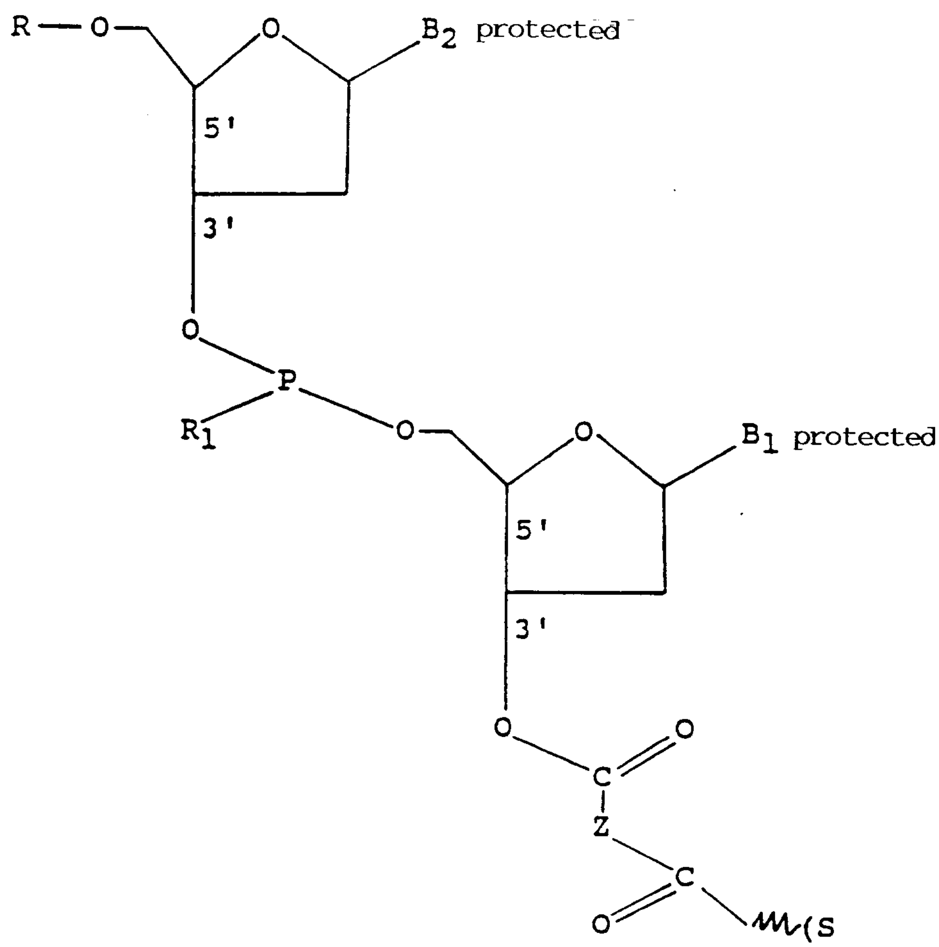

(V)

in which S, R, $R_1$, $B_1$ and $B_2$ have the meaning already indicated, then the product of formula (V) obtained is oxidized in order to obtain a product of formula:

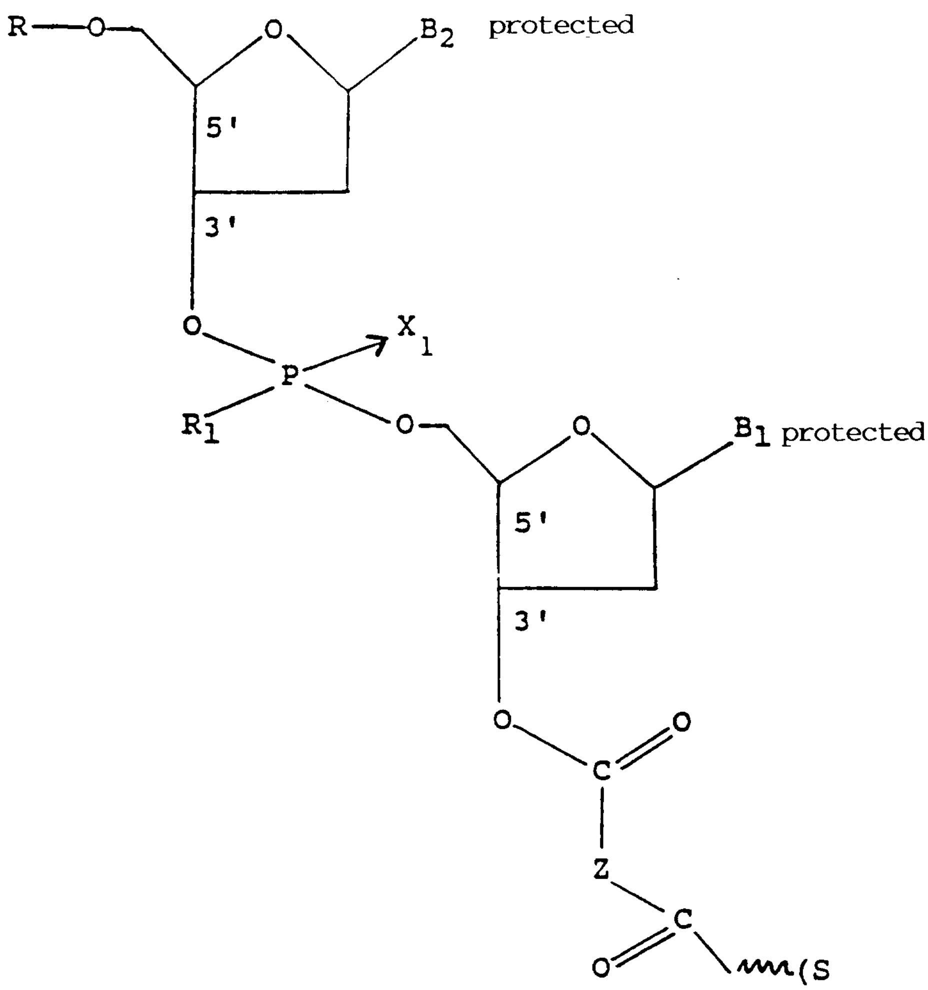

(VI)

in which S, R, $R_1$, $B_1$, $B_2$ and Z have the meaning already indicated and $X_1$ represents an oxygen or sulphur atom, then, as above, starting with the product of formula (II), a new cycle is carried out with this product of formula (VI) and the monomer of a new nucleotide until the desired chain formation is obtained, in order to obtain finally a product of formula:

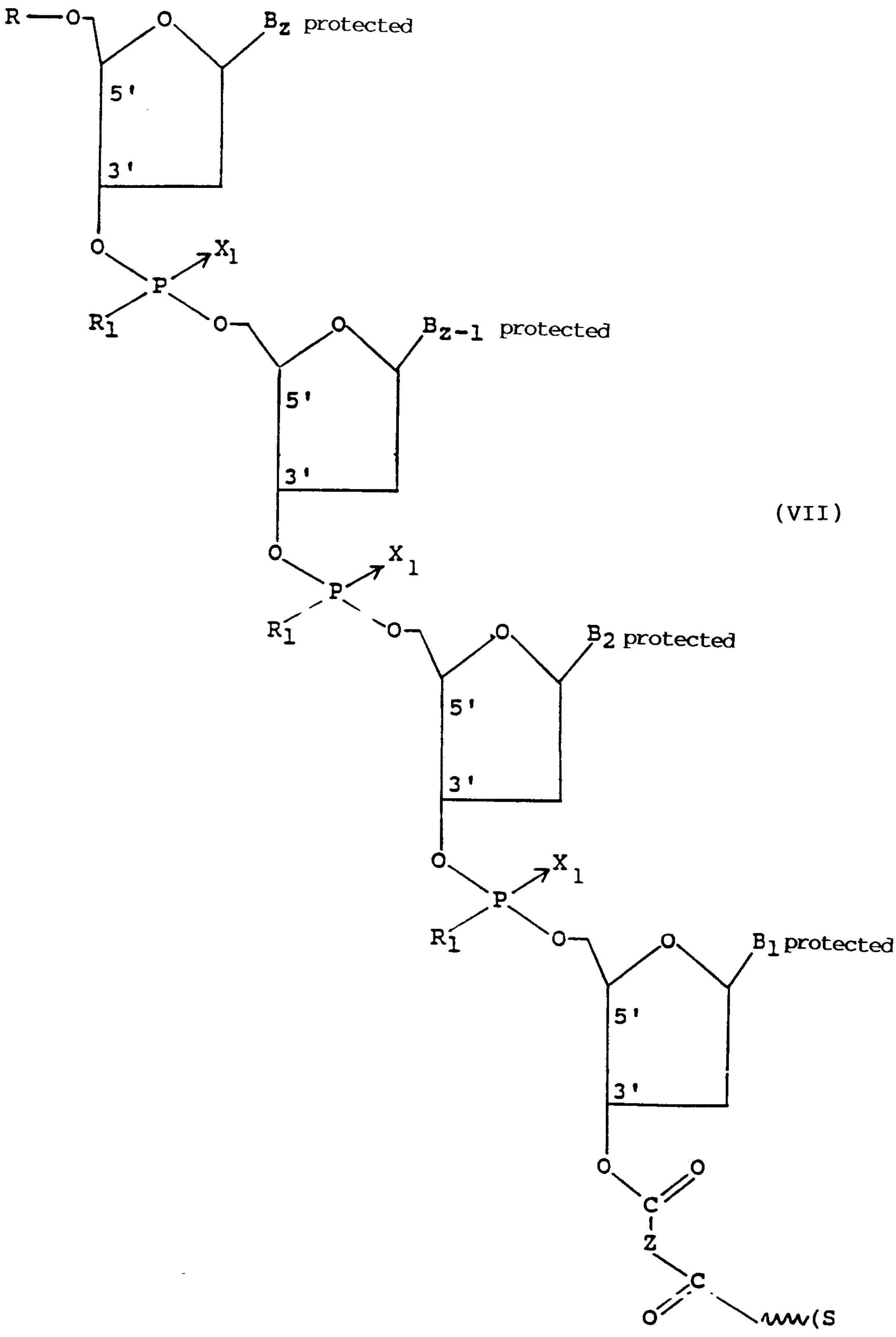

(VII)

in which S, R, $R_1$, $B_1$, $B_2$, $X_1$ and Z have the meaning already indicated and Bz represents the last nucleotide of the sequence, then the oligonucleotide is deprotected, separated from the support and the product of formula (I) thus obtained is purified.

**2.** Process according to claim 1, characterized in that:

- the unblocking of the hydroxyl in position 5' of the product of formula (II) is carried out by an acid reagent such as acetic acid, di- or trichloracetic acid,
- the coupling reaction of the product of formula (III) with the product of formula (IV) is activated using tetrazole operating in acetonitrile,
- the oxidation of the phosphite of formula (V) into the phosphate of formula (VI) is carried out using iodine in solution in a water/lutidine/tetrahydrofuran mixture or in a water/pyridine/tetrahydrofuran mixture,
- the oxidation of the phosphite of formula (V) in order to obtain the sulphurated form (VI) is carried out using sulphur in solution in a mixture of carbon sulphide, pyridine and anhydrous triethylamine,
- at the end of the synthesis the deprotection of the terminal hydroxyl group in position 5' is carried out using an acid such as acetic acid, di- or trichloracetic acid,
- the deprotection of the oligonucleotide of formula (VII) is carried out using concentrated ammonium hydroxide by moderately heating the reaction medium.

**3.** Process according to claim 1 for the preparation of the poly L-lysine derivatives of the oligonucleotide sequences as defined by formula (I), process characterized in that a product of formula (VIII):

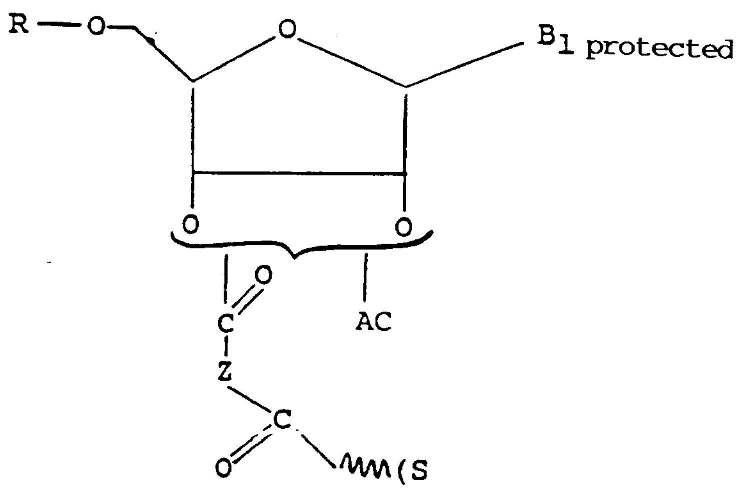

(VIII)

in which S, Z, R, $B_1$ have the meaning already indicated, is reacted with a product of formula (IV), in order to obtain a product of formula (IX):

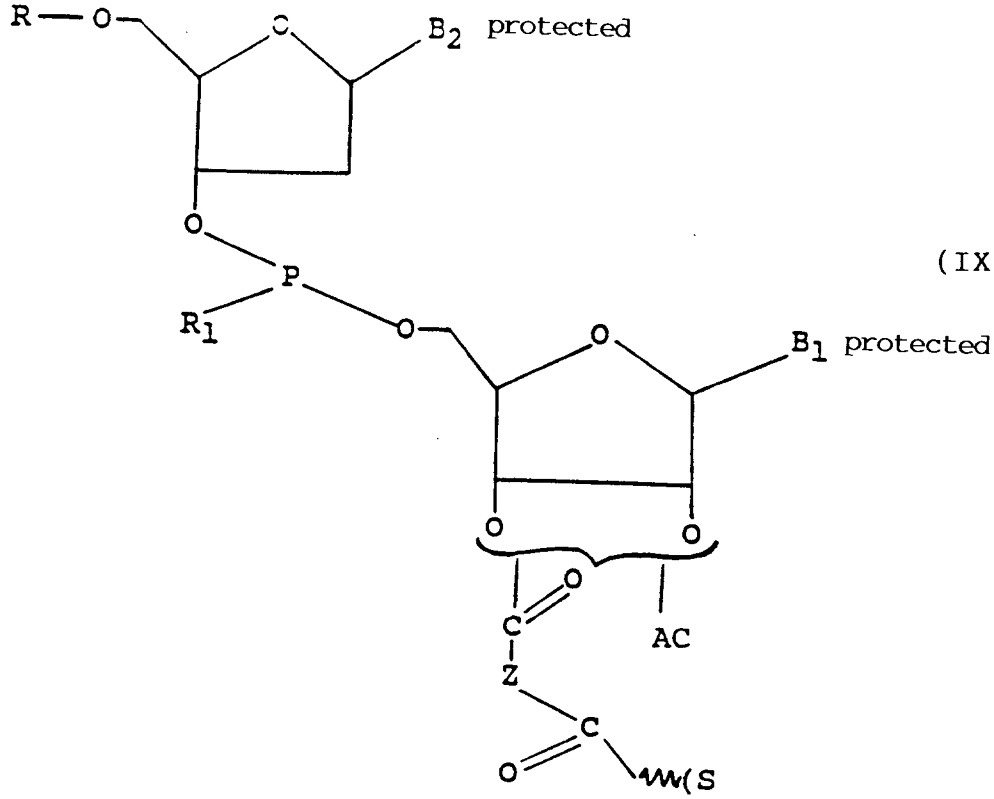

(IX)

in which S, R, Z, $R_1$, $B_1$ and $B_2$ have the meaning already indicated, oxidation is carried out, the support and the activator group are eliminated in order to obtain a product of formula (X):

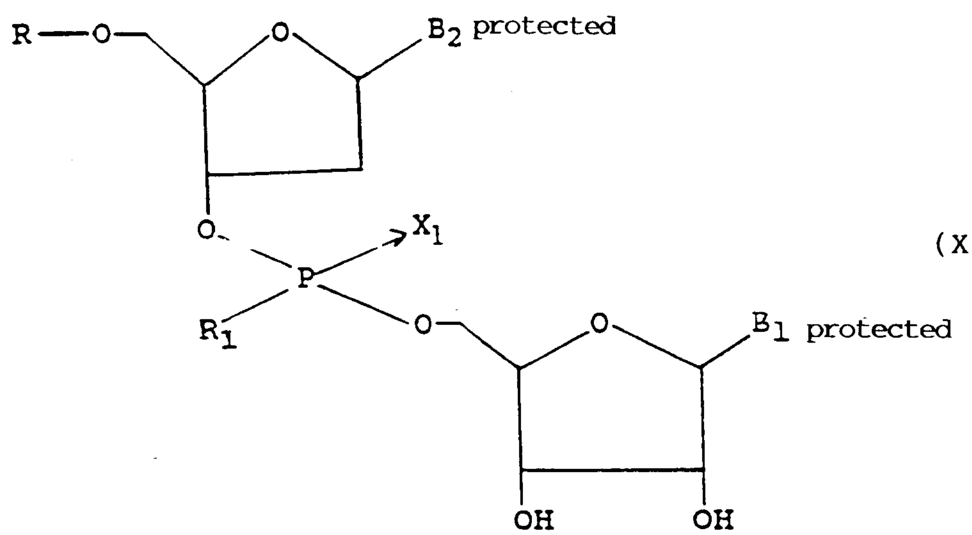

(X)

in which R, $R_1$, $B_1$ and $B_2$ have the meaning already indicated, $X_1$ represents an oxygen or sulphur atom, the terminal ribose is oxidized then reacted with L-lysine in a reducing medium, in order to obtain the product of formula (XI):

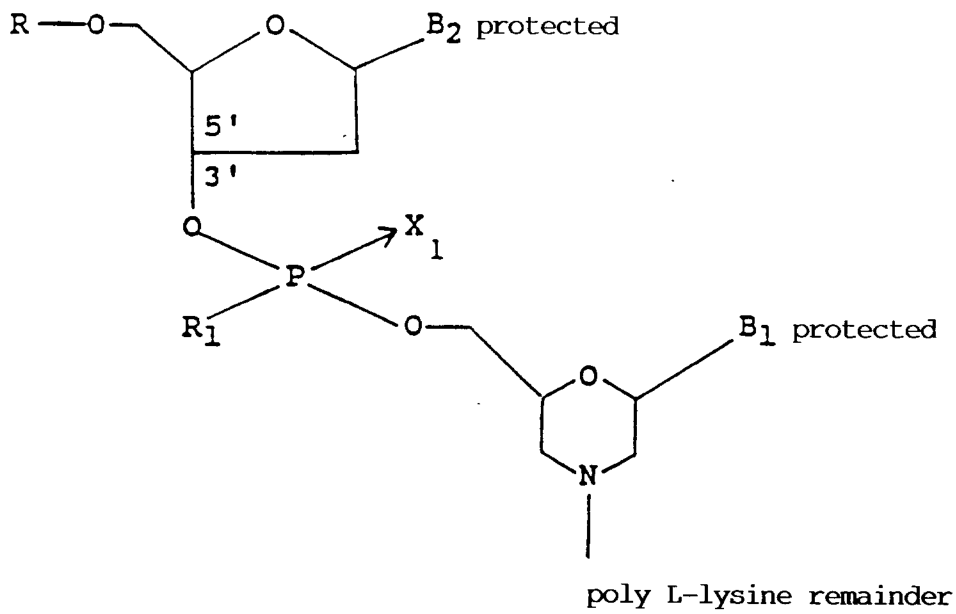

(XI)

in which R, $R_1$, $B_1$, $B_2$ and $X_1$ have the meaning already indicated, then the synthesis is continued as from the product of formula (VI).

**4.** Process according to claim 1, 2 or 3, characterized in that the first nucleotide in position 3' of the chain to be synthesized is either the first nucleotide of the final sequence

3′
TCA TGG TGT CCT TTG CAG

or one of the nucleotides of the sequence of formula ($I_A$):
- CC TCA TGC TTT CAG TAG
in free form, in alkylated form, in sulphurated form or in the form of a poly L-lysine derivative.

**5.** Process according to any one of claims 1 to 4, characterized in that the first nucleotide in position 3' of the chain to be synthesized is the first nucleotide of the - CC sequence or the - CC TCA TGC TTT CAG TAG sequence.

**6.** Process according to any one of claims 1 to 4, characterized in that the oligonucleotide sequence corresponding to the formula:

5′ 3′
(d) TCA TGG TGT CCT TTG CAG CC
1 20

is prepared in free form, in alkylated form, in sulphurated form or in the form of a poly L-lysine derivative.

**7.** Process according to any one of claims 1 to 4, characterized in that the oligonucleotide sequence corresponding to the formula:

5′ 3′
(d) TCA TGG TGT CCT TTG CAG
1 18

is prepared in free form, in alkylated form, in sulphurated form or in the form of a poly L-lysine derivative.

**8.** Process according to any one of claims 1 to 4, characterized in that the oligonucleotide sequence corresponding to the formula:

5′ 3′
(d) TCA TGG TGT CCT TTG CAG CC TCA TGC TTT CAG TAG
1 35

is prepared in free form, in alkylated form, in sulphurated form or in the form of a poly L-lysine derivative.

**9.** Preparation process for pharmaceutical compositions, characterized in that at least one of the new oligonucleotide sequences as defined in claim 1 is used as active ingredient.

**10.** Preparation process for pharmaceutical compositions, characterized in that at least one of the new oligonucleotide sequences as defined in any one of claims 2 to 8 is used as active ingredient.

**Patentansprüche**
**Patentansprüche für folgende Vertragsstaaten : AT, BE, CH, DE, DK, FR, GB, IT, LI, LU, NL, SE**

**1.** Antisense-Oligonucleotidsequenz, Antisense-RNA des alpha-TNF, dadurch gekennzeichnet, daß sie die Struktur der folgenden Formel (I) besitzt:

5' 3'
(d) TCA TGG TGT CCT TTG CAG - X (I)
1 18

worin X ein Wasserstoffatom oder eine Sequenz von 1 bis 17 Oligonucleotiden bedeutet, in freier Form, in alkylierter Form, in sulfurierter Form oder in Form des Poly-L-lysinderivats.

**2.** Oligonucleotidsequenz entsprechend der obigen Formel (I) gemäß Anspruch 1, dadurch gekennzeichnet, daß X ein Wasserstoffatom oder insgesamt oder einen Teil der Sequenz der Formel ($I_A$)

5' 3'
(d) - CC TCA TGC TTT CAG TAG ($I_A$)
19 35

in freier Form, in alkylierter Form, in sulfurierter Form oder in Form des Poly-L-lysinderivat bedeutet.

**3.** Oligonucleotidsequenzen wie in Formel (I) von Anspruch 1 definiert, dadurch gekennzeichnet, daß in der Formel (I) X ein Wasserstoffatom, eine Sequenz der Formel - CC oder die Sequenz der Formel ($I_A$) bedeutet, in freier Form, in alkylierter Form, in sulfurierter Form oder in Form des Poly-L-lysinderivats.

**4.** Oligonucleotidsequenz gemäß einem der Ansprüche 1 bis 3, dadurch gekennzeichnet, daß sie der Formel

```
     5'                        3'
(d)  TCA TGG TGT CCT TTG CAG CC
     1                         20
```

entspricht, in freier Form, in alkylierter Form, in sulfurierter Form oder in Form des Poly-L-lysinderivats.

**5.** Oligonucleotidsequenz gemäß einem der Ansprüche 1 bis 3, dadurch gekennzeichnet, daß sie der Formel

```
     5'                     3'
(d)  TCA TGG TGT CCT TTG CAG
     1                      18
```

entspricht, in freier Form, in alkylierter Form, in sulfurierter Form oder in Form des Poly-L-lysinderivats.

**6.** Oligonucleotidsequenz gemäß einem der Ansprüche 1 bis 3, dadurch gekennzeichnet, daß sie der Formel

```
     5'                                                3'
(d)  TCA TGG TGT CCT TTG CAG CC TCA TGC TTT CAG TAG
     1                                                 35
```

entspricht, in freier Form, in alkylierter Form, in sulfurierter Form oder in Form des Poly-L-lysinderivats.

**7.** Verfahren zur Herstellung der Oligonucleotidsequenz, wie sie durch Formel (I) von Anspruch 1 definiert ist, dadurch gekennzeichnet, daß man auf einem Träger S das erste Nucleotid in 3'-Stellung der zu synthetisierenden Kette der Formel

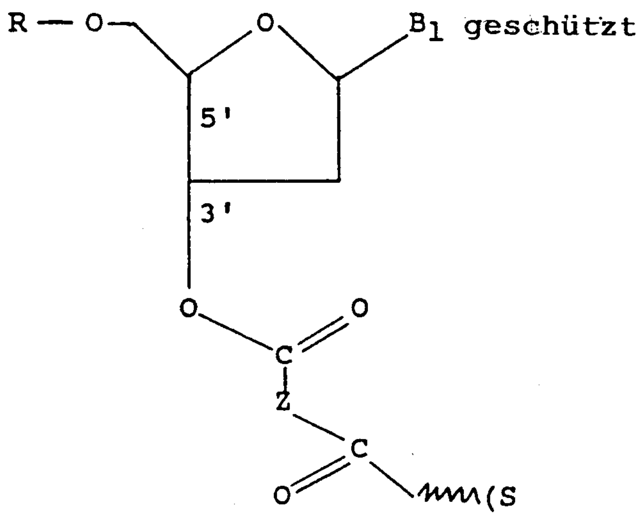

(II)

immobilisiert, worin S den Träger bedeutet, Z bedeutet eine Kohlenwasserstoffgruppe mit 2 bis 20 Kohlenstoffatomen, $B_1$ ist eine Purin- oder Pyrimidinbase entsprechend dem ersten Nucleotid, dessen Aminfunktion geschützt ist, und R ist eine Schutzgruppe,
das Hydroxyl in 5'-Stellung durch ein saures Reagens entblockiert, um das Produkt der Formel

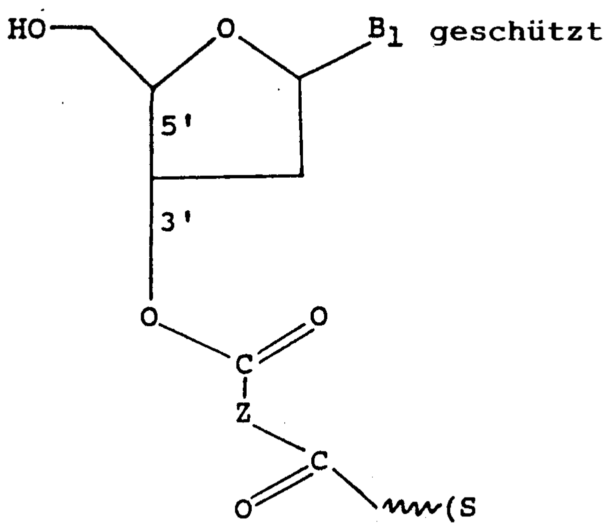

(III)

zu erhalten, worin S, Z und $B_1$ die angegebene Bedeutung haben, dann dieses letztere zur Reaktion mit dem Monomeren des zweiten Nucleotids der Formel

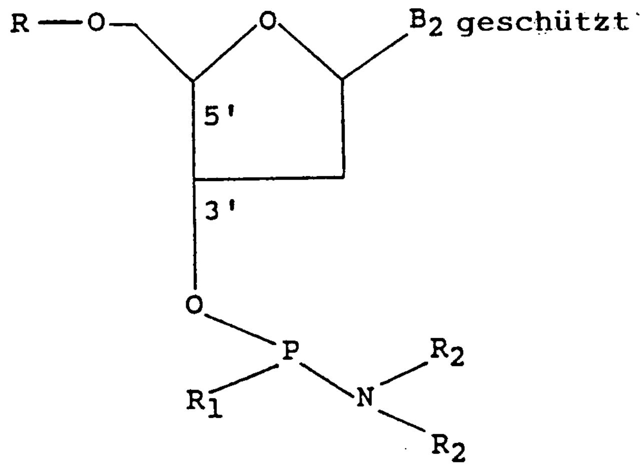

(IV)

reagieren läßt, worin R die bereits angegebene Bedeutung hat, $B_2$ ist eine Purin- oder Pyrimidinbase entsprechend dem zweiten Nucleotid, dessen Aminfunktion geschützt ist, $R_1$ bedeutet einen Alkylrest oder eine Gruppe -OR'$_1$ oder -SR'$_1$, worin R'$_1$ eine Schutzgruppe bedeutet, $R_2$ bedeutet eine Schutzgruppe, um ein Produkt der Formel

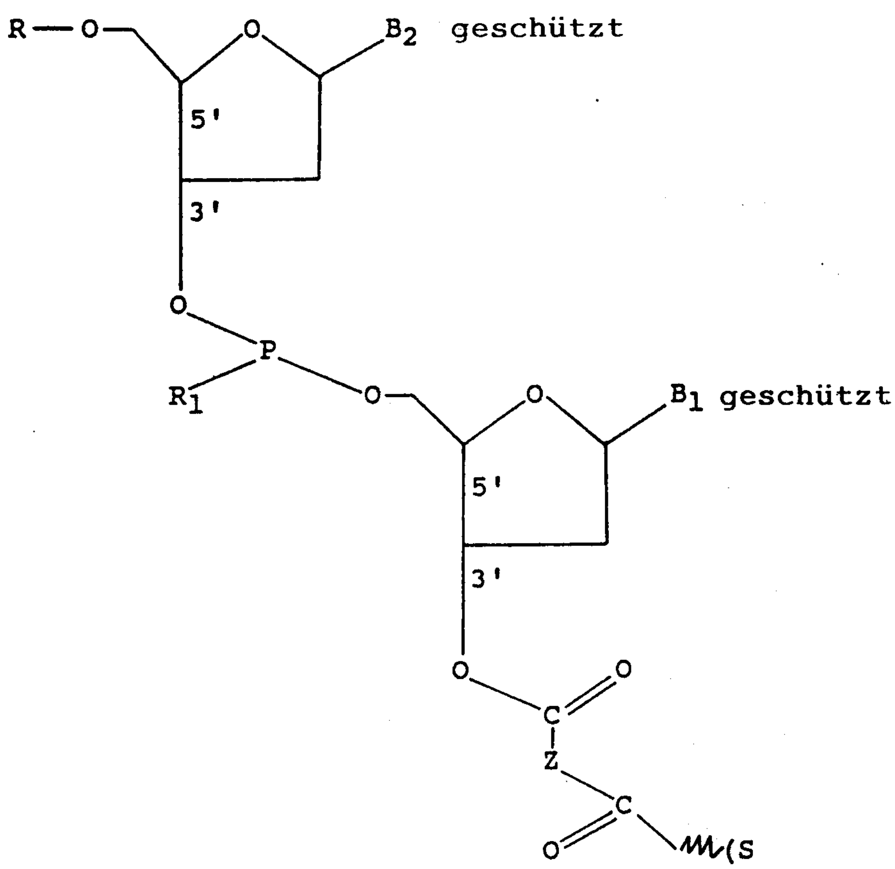

(V)

zu erhalten, worin S, R, $R_1$, $B_1$ und $B_2$ die vorstehend angegebene Bedeutung haben, dann oxidiert man das erhaltene Produkt der Formel (V), um ein Produkt der Formel

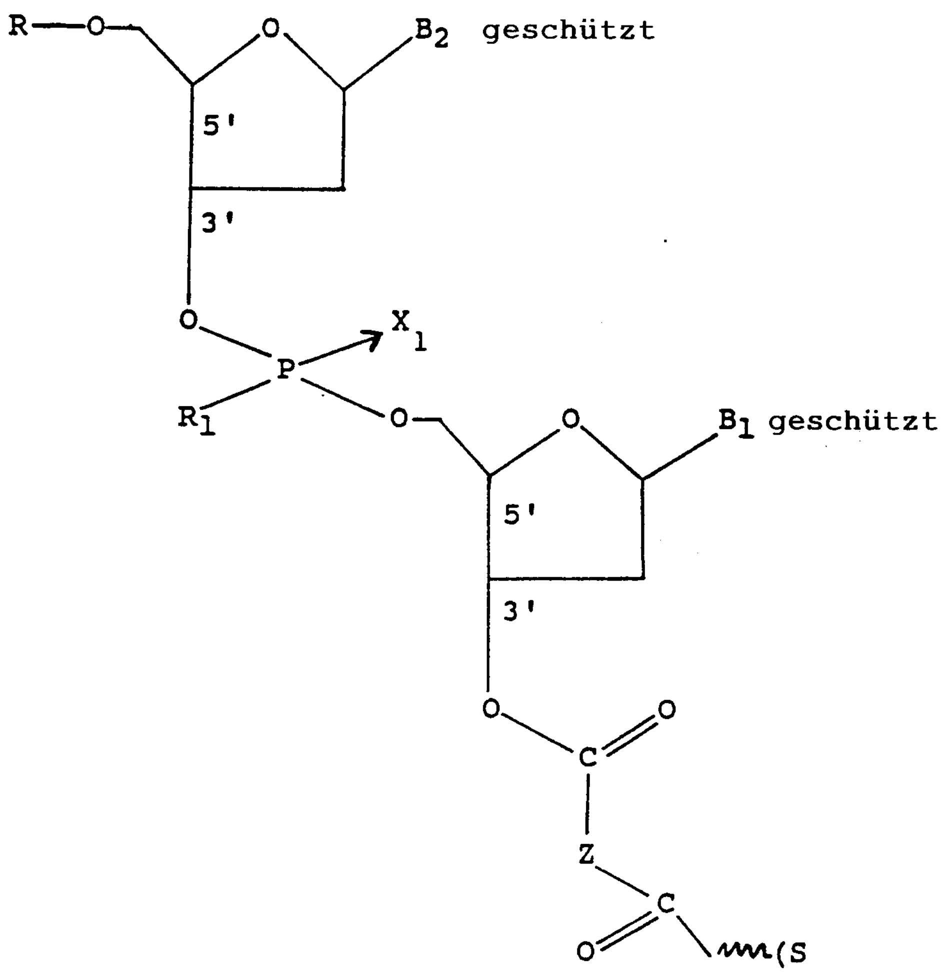

(VI)

zu erhalten, worin S, R, $R_1$, $B_1$, $B_2$ und Z die vorstehend genannte Bedeutung haben, und $X_1$ bedeutet ein Sauerstoff- oder Schwefelatom, dann bewirkt man, wie vorstehend beschrieben ausgehend vom Produkt der Formel (II), einen neuen Zyklus mit diesem Produkt der Formel (VI) und dem Monomeren eines neuen Nucleotids bis zu Erzielung der gewünschten Verkettung, um schließlich ein Produkt der Formel

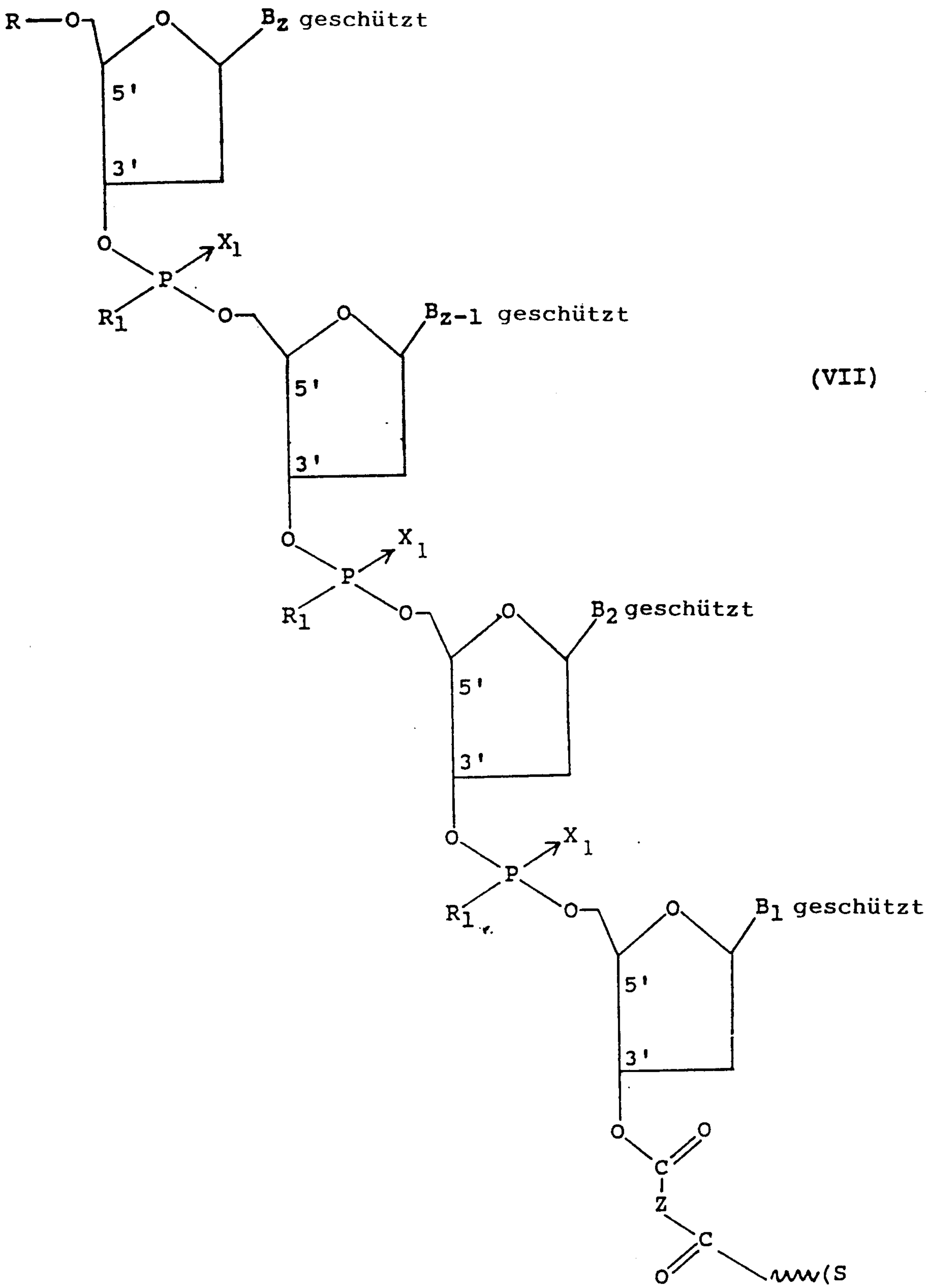

(VII)

zu erhalten, worin S, R, $R_1$, $B_1$, $B_2$, $X_1$ und Z die vorstehend angegebenen Bedeutungen haben, und $B_z$ das letzte Nucleotid der Sequenz bedeutet, dann spaltet man die Schutzgruppen des Oligonucleotids ab, trennt es vom Träger und reinigt das so erhaltene Produkt der Formel (I).

**8.** Verfahren gemäß Anspruch 7, dadurch gekennzeichnet, daß

- die Entblockierung des Hydroxyls in 5'-Stellung des Produkts der Formel (II) durch ein saures Reagens wie Essigsäure, Di- oder Trichloressigsäure bewirkt wird;
- die Kupplungsreaktion des Produkts der Formel (III) mit dem Produkt der Formel (IV) mittels Tetrazol aktiviert wird, wobei in Acetonitril gearbeitet wird;
- die Oxidation des Phosphits der Formel (V) zum Phosphat der Formel (VI) mittels Jod in Lösung in einem Gemisch Wasser/Lutidin/Tetrahydrofuran oder in einem Gemisch Wasser/Pyridin/Tetrahydrofuran bewirkt wird;
- die Oxidation des Phosphits der Formel (V) zur Erzielung der sulfurierten Form (VI) mittels Schwefel in Lösung in einem wasserfreien Gemisch von Schwefelkohlenstoff, Pyridin und Triethylamin bewirkt wird;
- am Schluß der Synthese die Schutzgruppenabspaltung der endständigen Hydroxylgruppe in 5'-Stellung mittels einer Säure wie Essigsäure, Di- oder Trichloressigsäure bewirkt wird;
- die Schutzgruppenabspaltung des Oligonucleotids der Formel (VII) mittels konzentriertem Ammoniak bewirkt wird, wobei das Reaktionsmilieu mäßig erhitzt wird.

**9.** Verfahren gemäß Anspruch 7 zur Herstellung der Derivate des Poly-L-lysins der Oligonucleotidsequenzen wie sie durch Formel (I) definiert sind, wobei dieses Verfahren dadurch gekennzeichnet ist, daß man ein Produkt der Formel (VIII):

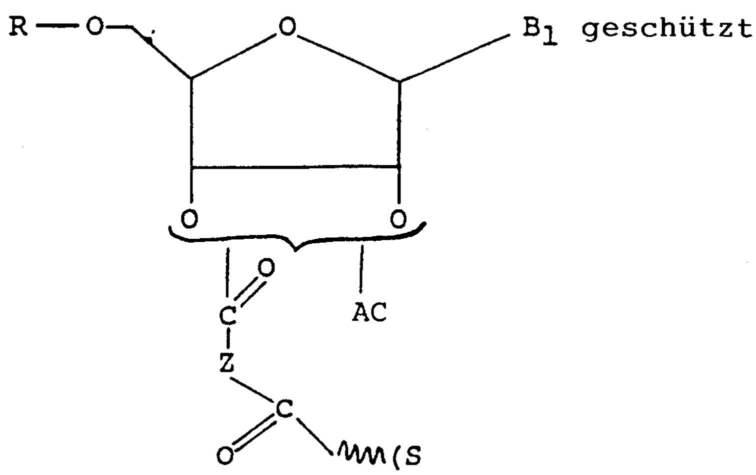

(VIII)

worin S, Z, R, $B_1$ die vorstehend angegebene Bedeutung haben, mit einem Produkt der Formel (IV) zur Reaktion bringt, um ein Produkt der Formel (IX):

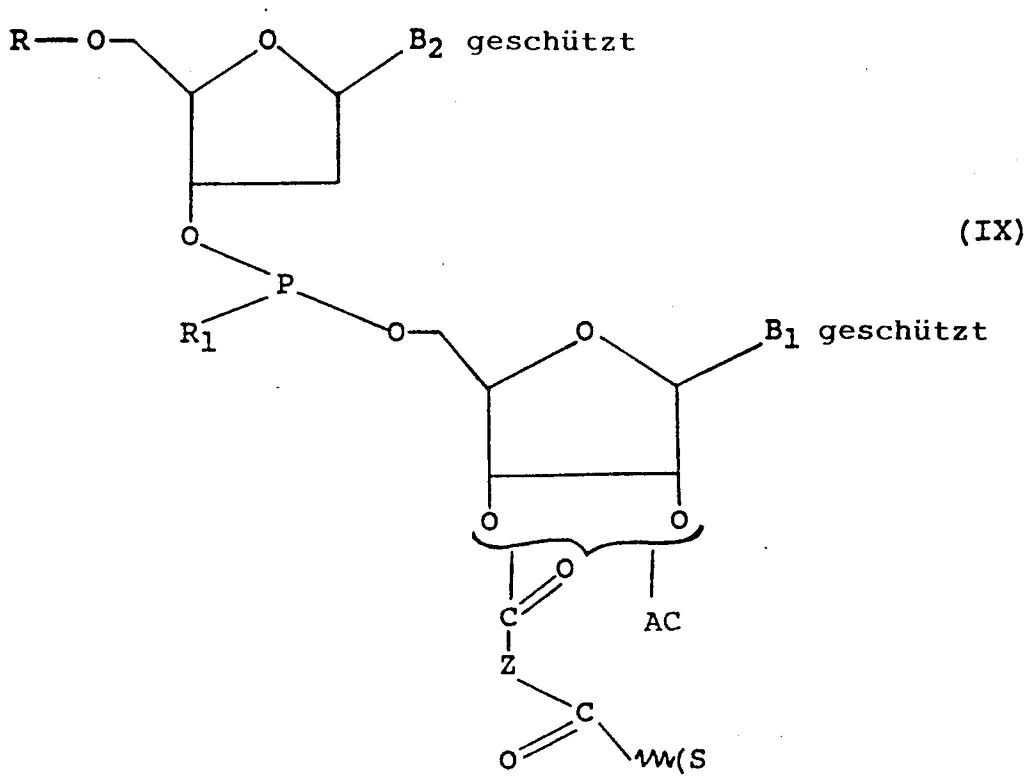

(IX)

zu erhalten, worin S, R, Z, $R_1$, $B_1$ und $B_2$ die vorstehend angegebene Bedeutung haben, man oxidiert, entfernt den Träger und die aktivierende Gruppe, um ein Produkt der Formel (X)

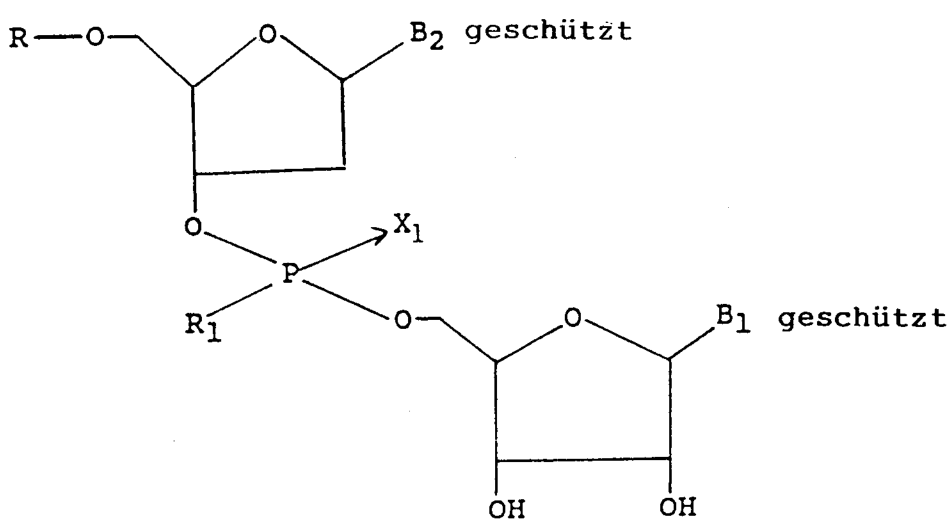

(X)

zu erhalten, worin R, $R_1$, $B_1$ und $B_2$ die vorstehend angegebene Bedeutung haben, $X_1$ bedeutet ein Sauerstoff- oder Schwefelatom, man oxidiert die endständige Ribose, dann setzt man mit L-Lysin in reduzierendem Milieu um, um das Produkt der Formel (XI)

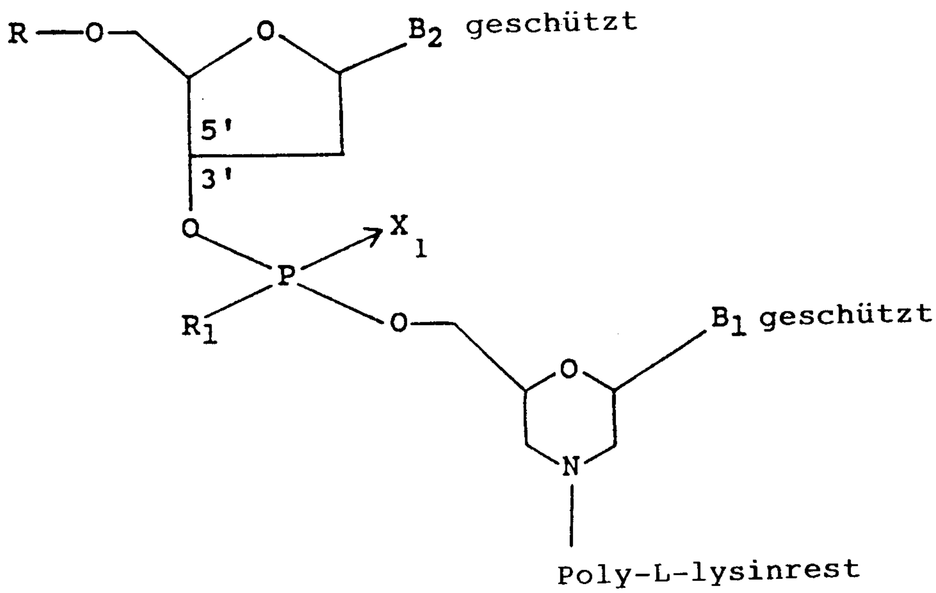

(XI)

zu erhalten, worin R, $R_1$, $B_1$, $B_2$ und $X_1$ die vorstehend angegebene Bedeutung haben, dann setzt man die Synthese wie ausgehend vom Produkt der Formel (VI) fort.

**10.** Arzneimittel, dadurch gekennzeichnet, daß sie aus den neuen Oligonucleotidsequenzen, wie sie in Anspruch 1 definiert sind, bestehen.

**11.** Arzneimittel, dadurch gekennzeichnet, daß sie aus den neuen Oligonucleotidsequenzen, wie sie in einem der Ansprüche 2 bis 6 definiert sind, bestehen.

**12.** Pharmazeutische Zusammensetzungen, dadurch gekennzeichnet, daß sie als Wirkstoff wenigstens eines der Arzneimittel wie sie in einem der Ansprüche 10 oder 11 definiert sind, enthalten.

**Patentansprüche für folgenden Vertragsstaat : ES**

**1.** Verfahren zur Herstellung der Anti-sense-Oligonucleotidsequenz, Antisense-RNA des alpha-TNF, mit der Struktur der folgenden Formel (I):

```
      5'                      3'
(d)   TCA TGG TGT CCT TTG CAG - X          (I)
      1                       18
```

worin X ein Wasserstoffatom oder eine Sequenz von 1 bis 17 Oligonucleotiden bedeutet, in freier Form, in alkylierter Form, in sulfurierter Form oder in Form des Poly-L-lysinderivats, dadurch gekennzeichnet, daß man auf einem Träger S das erste Nucleotid in 3'-Stellung der zu synthetisierenden Kette der Formel

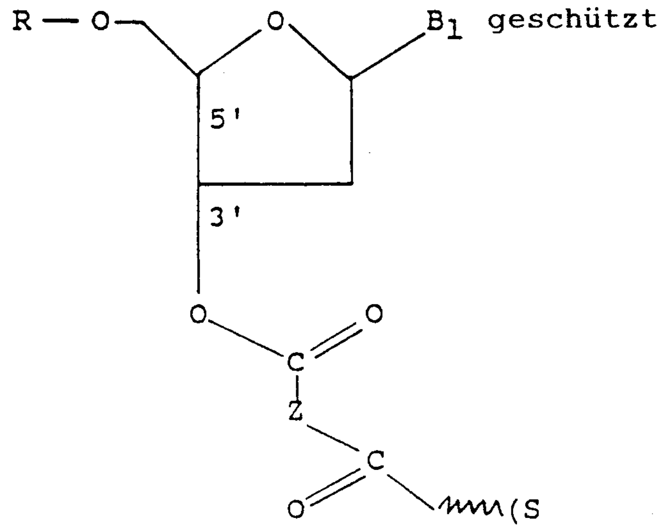

immobilisiert, worin S den Träger bedeutet, Z bedeutet eine Kohlenwasserstoffgruppe mit 2 bis 20 Kohlenstoffatomen, $B_1$ ist eine Purin- oder Pyrimidinbase entsprechend dem ersten Nukleotid, dessen Aminfunktion geschützt ist, und R ist eine Schutzgruppe,
das Hydroxyl in 5'-Stellung durch ein saures Reagens entblockiert, um das Produkt der Formel

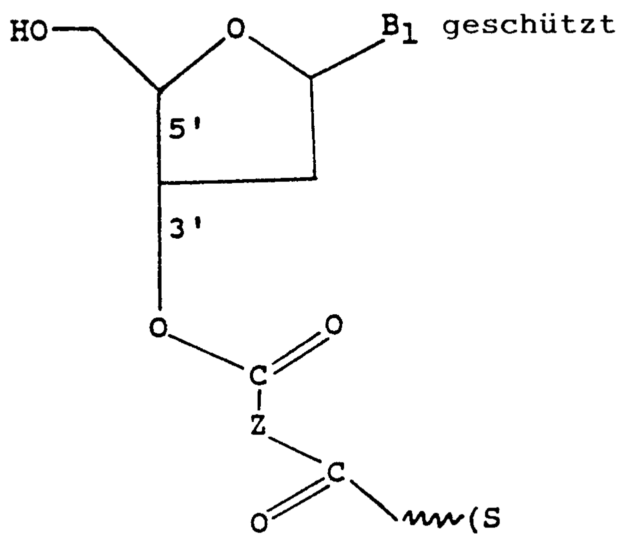

(III)

zu erhalten, worin S, Z und $B_1$ die bereits angegebene Bedeutung haben, dann dieses letztere mit dem Monomeren des zweiten Nucleotids der Formel (IV):

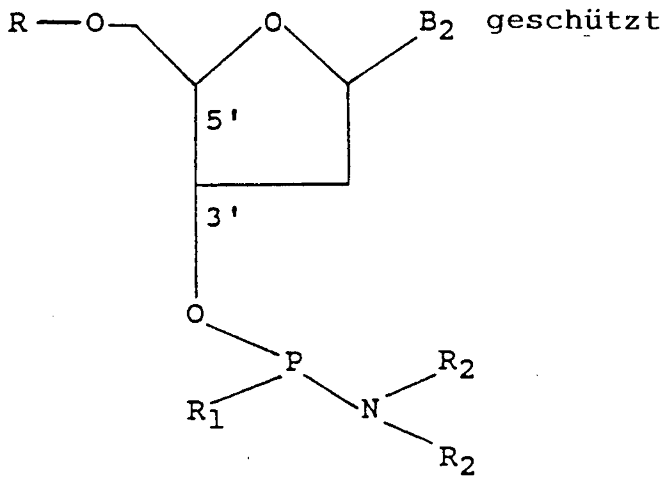

(IV)

reagieren läßt, worin R die bereits angegebene Bedeutung hat, $B_2$ ist eine Purin- oder Pyrimidinbase entsprechend dem zweiten Nucleotid, dessen Aminfunktion geschützt ist, $R_1$ bedeutet einen Alkylrest oder eine Gruppe -OR'$_1$ oder -SR'$_1$, worin R'$_1$ eine Schutzgruppe bedeutet, $R_2$ bedeutet eine Schutzgruppe, um ein Produkt der Formel

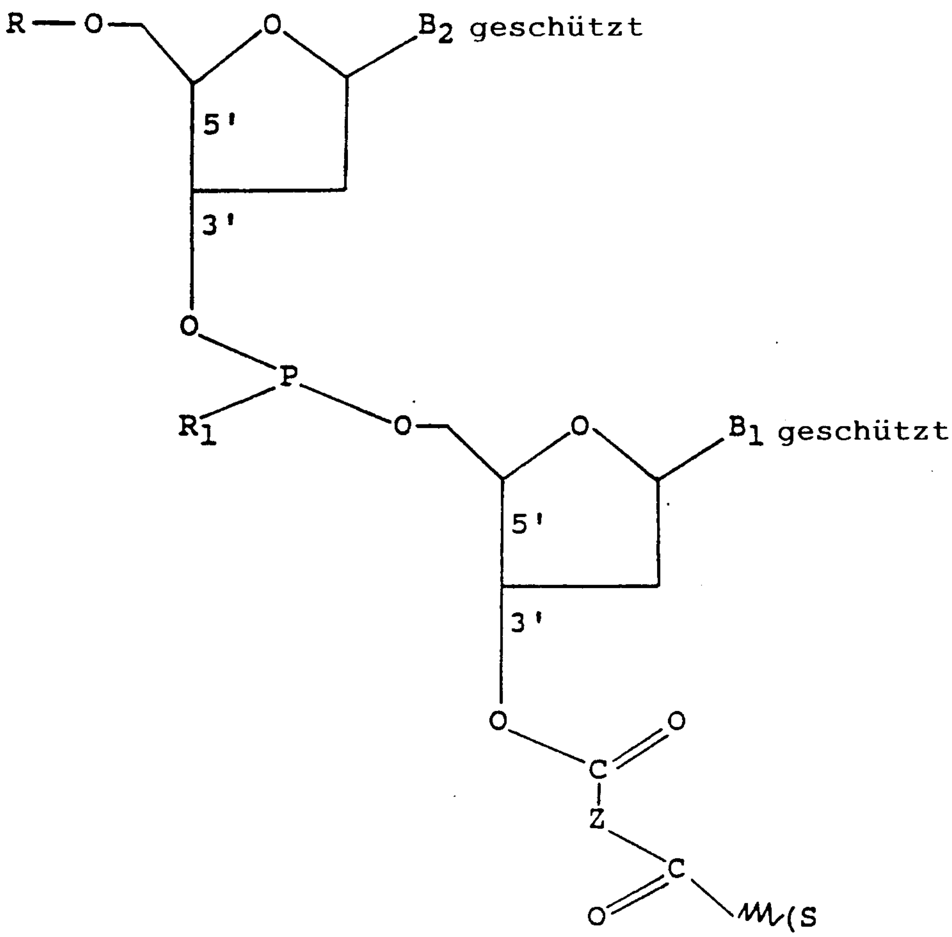

(V)

zu erhalten, worin S, R, $R_1$, $B_1$ und $B_2$ die vorstehend angegebene Bedeutung haben, dann oxidiert man das erhaltene Produkt der Formel (V), um ein Produkt der Formel

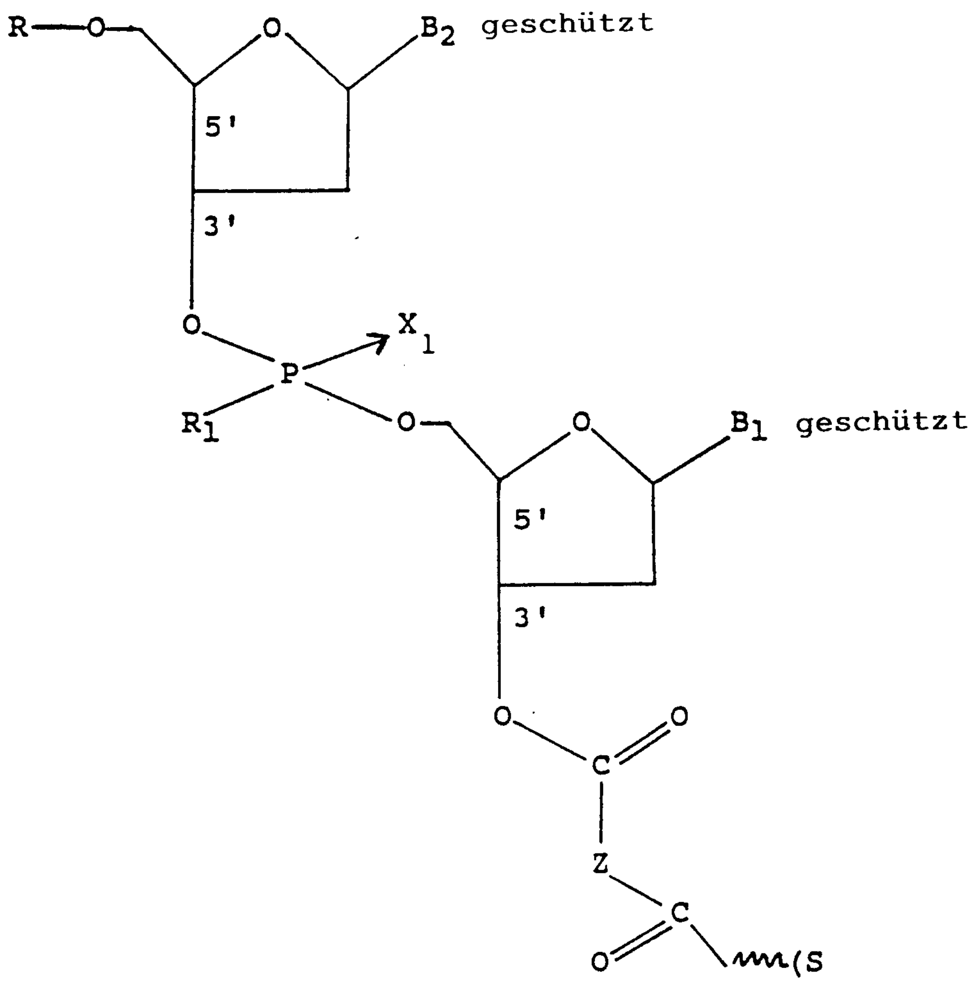

(VI)

zu erhalten, worin S, R, $R_1$, $B_1$, $B_2$ und Z die vorstehend genannte Bedeutung haben, und $X_1$ bedeutet ein Sauerstoff- oder Schwefelatom, dann bewirkt man, wie vorstehend beschrieben ausgehend vom Produkt der Formel (II), einen neuen Zyklus mit diesem Produkt der Formel (VI) und dem Monomeren eines neuen Nucleotids bis zu Erzielung der gewünschten Verkettung, um schließlich ein Produkt der Formel

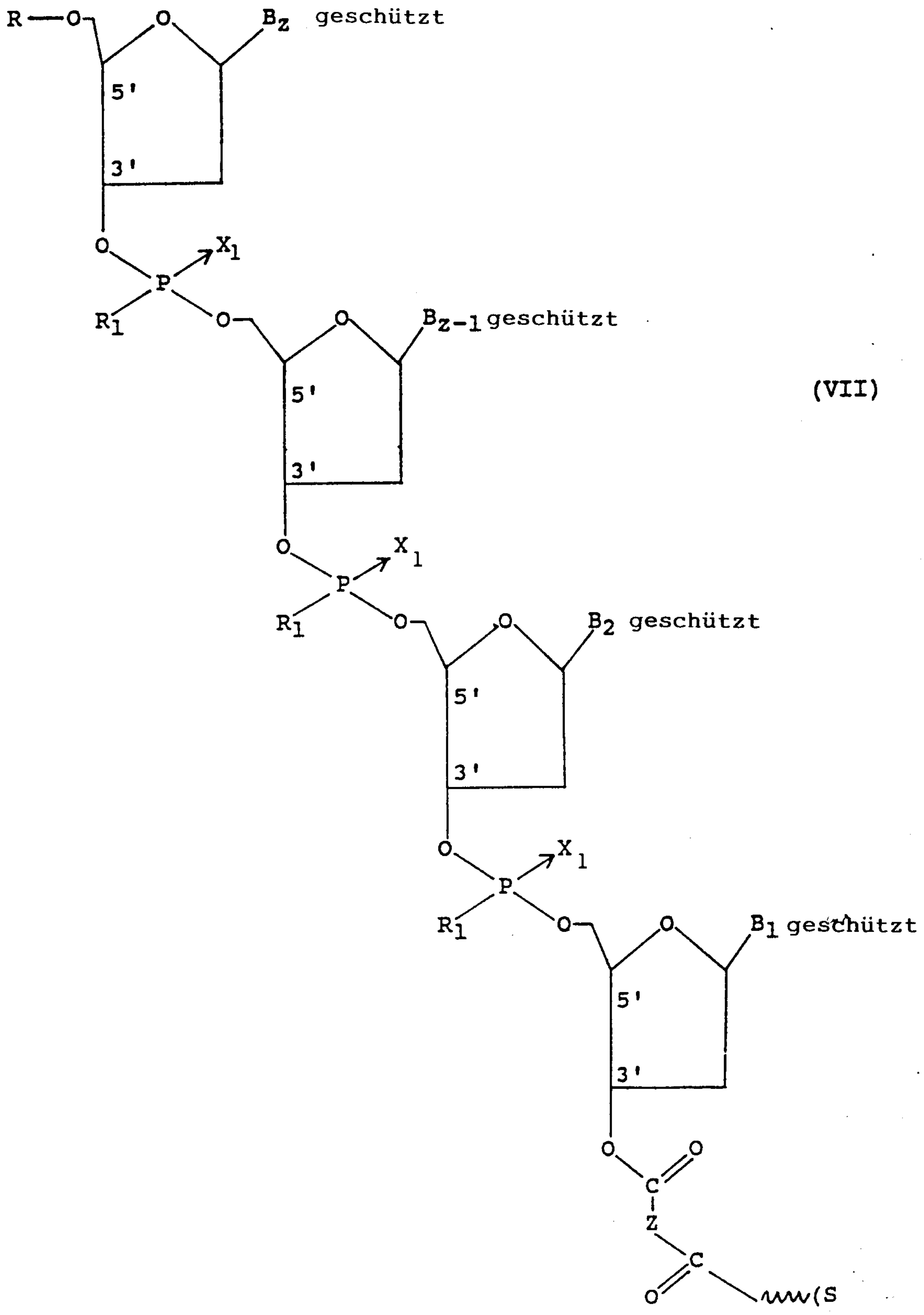

(VII)

zu erhalten, worin S, R, $R_1$, $B_1$, $B_2$, $X_1$ und Z die vorstehend angegebenen Bedeutungen haben, und $B_z$ das letzte Nucleotid der Sequenz bedeutet, dann spaltet man die Schutzgruppen des Oligonucleotids ab, trennt es vom Träger und reinigt das so erhaltene Produkt der Formel (I).

**2.** Verfahren gemäß Anspruch 1, dadurch gekennzeichnet, daß

- die Entblockierung des Hydroxyls in 5'-Stellung des Produkts der Formel (II) durch ein saures Reagens wie Essigsäure, Di- oder Trichloressigsäure bewirkt wird;
- die Kupplungsreaktion des Produkts der Formel (III) mit dem Produkt der Formel (IV) mittels Tetrazol aktiviert wird, wobei in Acetonitril gearbeitet wird;
- die Oxidation des Phosphits der Formel (V) zum Phosphat der Formel (VI) mittels Jod in Lösung in einem Gemisch Wasser/Lutidin/Tetrahydrofuran oder in einem Gemisch Wasser/Pyridin/Tetrahydrofuran bewirkt wird;
- die Oxidation des Phosphits der Formel (V) zur Erzielung der sulfurierten Form (VI) mittels Schwefel in Lösung in einem wasserfreien Gemisch von Schwefelkohlenstoff, Pyridin und Triethylamin bewirkt wird;
- am Schluß der Synthese die Schutzgruppenabspaltung der endständigen Hydroxylgruppe in 5'-Stellung mittels einer Säure wie Essigsäure, Di- oder Trichloressigsäure bewirkt wird;
- die Schutzgruppenabspaltung des Oligonucleotids der Formel (VII) mittels konzentriertem Ammoniak bewirkt wird, wobei das Reaktionsmilieu mäßig erhitzt wird.

**3.** Verfahren gemäß Anspruch 1 zur Herstellung der Derivate des Poly-L-lysins der Oligonucleotidsequenzen wie sie durch Formel (I) definiert sind, wobei das Verfahren dadurch gekennzeichnet ist, daß man ein Produkt der Formel (VIII):

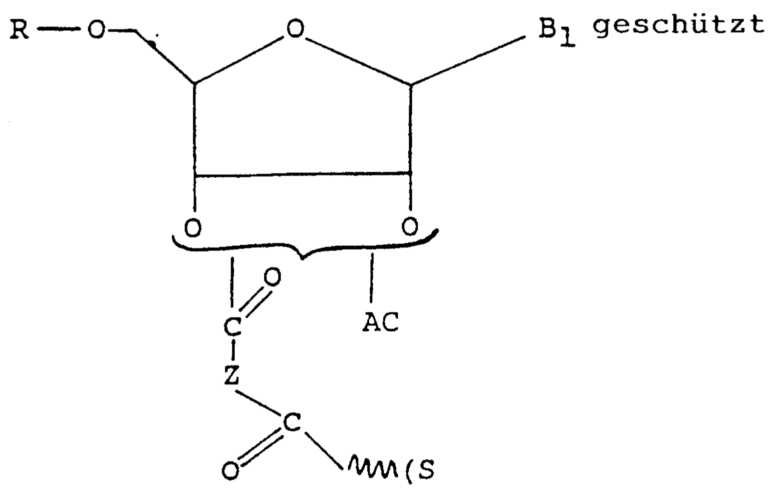

(VIII)

worin S, Z, R, $B_1$ die vorstehend angegebene Bedeutung haben, mit einem Produkt der Formel (IV) zur Reaktion bringt, um ein Produkt der Formel (IX):

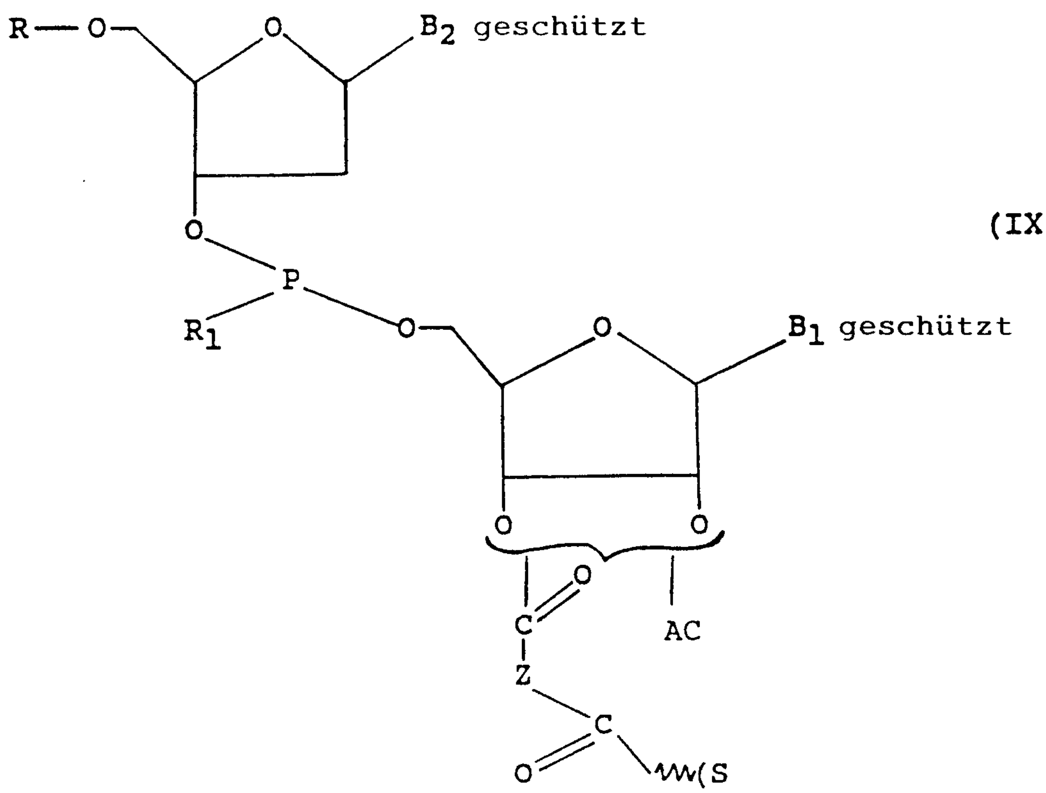

(IX)

zu erhalten, worin S, R, Z, $R_1$, $B_1$ und $B_2$ die vorstehend angegebene Bedeutung haben, man oxidiert, entfernt den Träger und die aktivierende Gruppe, um ein Produkt der Formel (X)

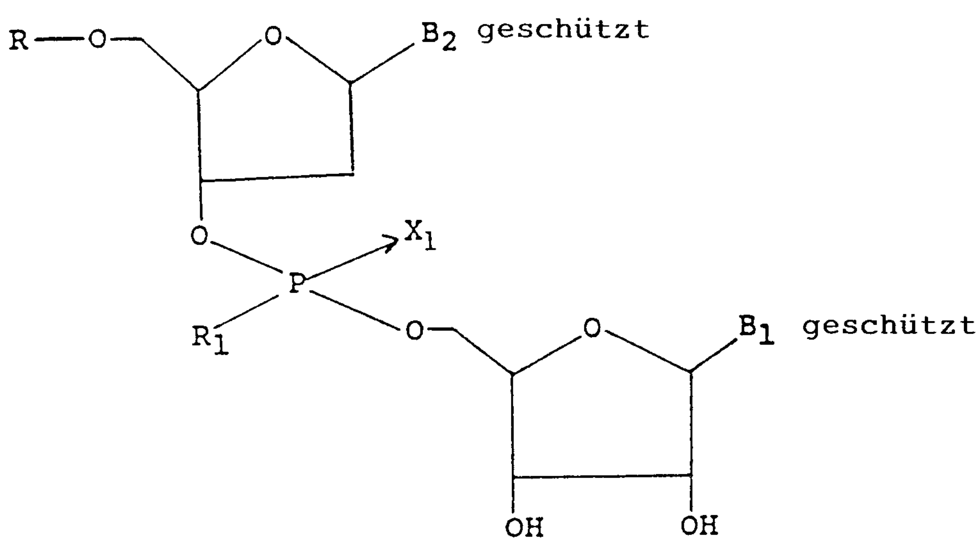

(X)

zu erhalten, worin R, $R_1$, $B_1$ und $B_2$ die vorstehend angegebene Bedeutung haben, $X_1$ bedeutet ein Sauerstoff- oder Schwefelatom, man oxidiert die endständige Ribose, dann setzt man mit L-Lysin in reduzierendem Milieu um, um das Produkt der Formel (XI)

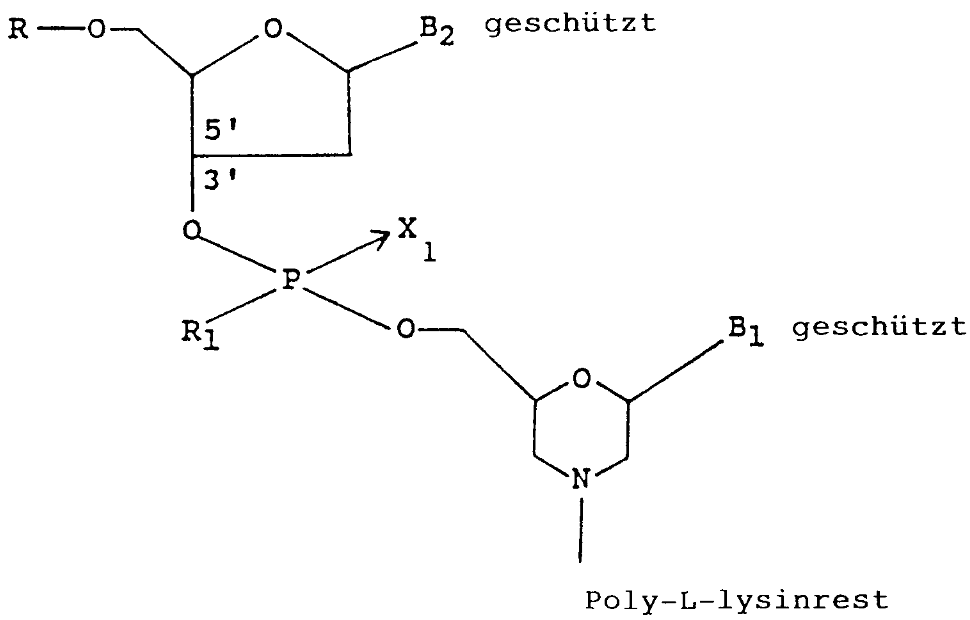

(XI)

zu erhalten, worin R, $R_1$, $B_1$, $B_2$ und $X_1$ die vorstehend angegebene Bedeutung haben, dann setzt man die Synthese wie ausgehend vom Produkt der Formel (VI) fort.

4. Verfahren gemäß Anspruch 1, 2 oder 3, dadurch gekennzeichnet, daß das erste Nukleotid in 3'-Stellung der zu synthetisierenden Kette entweder das erste Nucleotid der Endsequenz

```
                                3'
TCA  TGG  TGT  CCT  TTG  CAG
```

ist oder eines der Nukleotide der Sequenz der Formel ($I_A$):
- CC TCA TGC TTT CAG TAG
ist, in freier Form, in alkylierter Form, in sulfurierter Form oder in Form des Poly-L-lysinderivats.

5. Verfahren gemäß einem der Ansprüche 1 bis 4, dadurch gekennzeichnet, daß das erste Nukleotid in 3'-Stellung der zu synthetisierenden Kette, das erste Nucleotid der Sequenz - CC oder der Sequenz - CC TCA TGC TTT CAG TAG ist.

**Patentansprüche für folgenden Vertragsstaat : GR**

1. Verfahren zur Herstellung der Antisense-Oligonucleotidsequenz, Antisense-RNA des alpha-TNF, mit der Struktur der folgenden Formel (I):

```
     5'                       3'
(d)  TCA  TGG  TGT  CCT  TTG  CAG  -  X          (I)
     1                        18
```

worin X ein Wasserstoffatom oder eine Sequenz von 1 bis 17 Oligonucleotiden bedeutet, in freier Form, in alkylierter Form, in sulfurierter Form oder in Form des Poly-L-lysinderivats, dadurch gekennzeichnet, daß man auf einem Träger S das erste Nucleotid in 3'-Stellung der zu synthetisierenden Kette der Formel

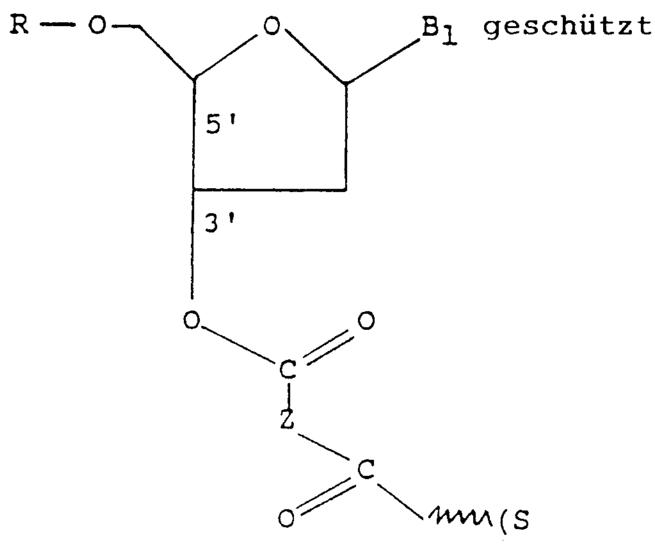

immobilisiert, worin S den Träger bedeutet, Z bedeutet eine Kohlenwasserstoffgruppe mit 2 bis 20 Kohlenstoffatomen, $B_1$ ist eine Purin- oder Pyrimidinbase entsprechend dem ersten Nucleotid, dessen Aminfunktion geschützt ist, und R ist eine Schutzgruppe, das Hydroxyl in 5'-Stellung durch ein saures Reagens entblockiert, um das Produkt der Formel

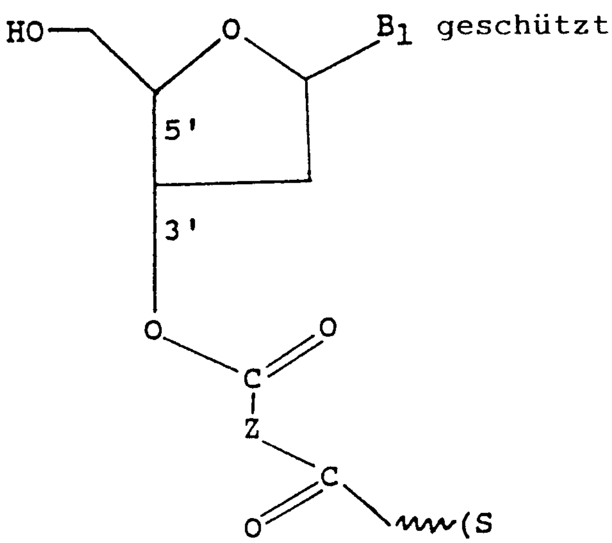

(III)

zu erhalten, worin S, Z und $B_1$ die bereits angegebene Bedeutung haben, dann dieses letztere mit dem Monomeren des zweiten Nucleotids der Formel (IV):

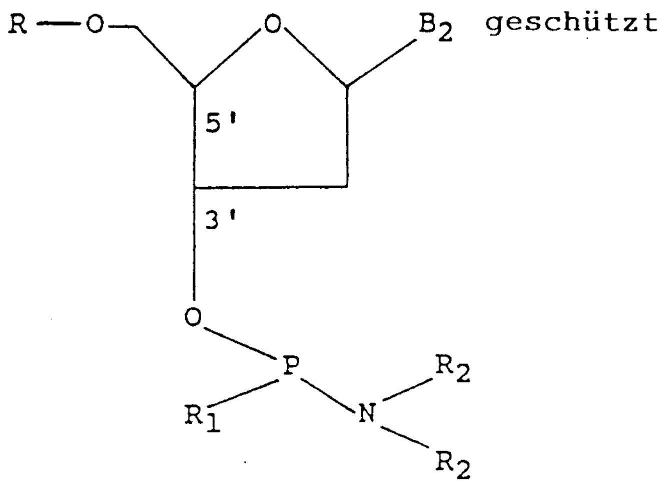

(IV)

reagieren laßt, worin R die bereits angegebene Bedeutung hat, $B_2$ ist eine Purin- oder Pyrimidinbase entsprechend dem zweiten Nucleotid, dessen Aminfunktion geschützt ist, $R_1$ bedeutet einen Alkylrest oder eine Gruppe -OR'$_1$ oder -SR'$_1$, worin R'$_1$ eine Schutzgruppe bedeutet, $R_2$ bedeutet eine Schutzgruppe, um ein Produkt der Formel

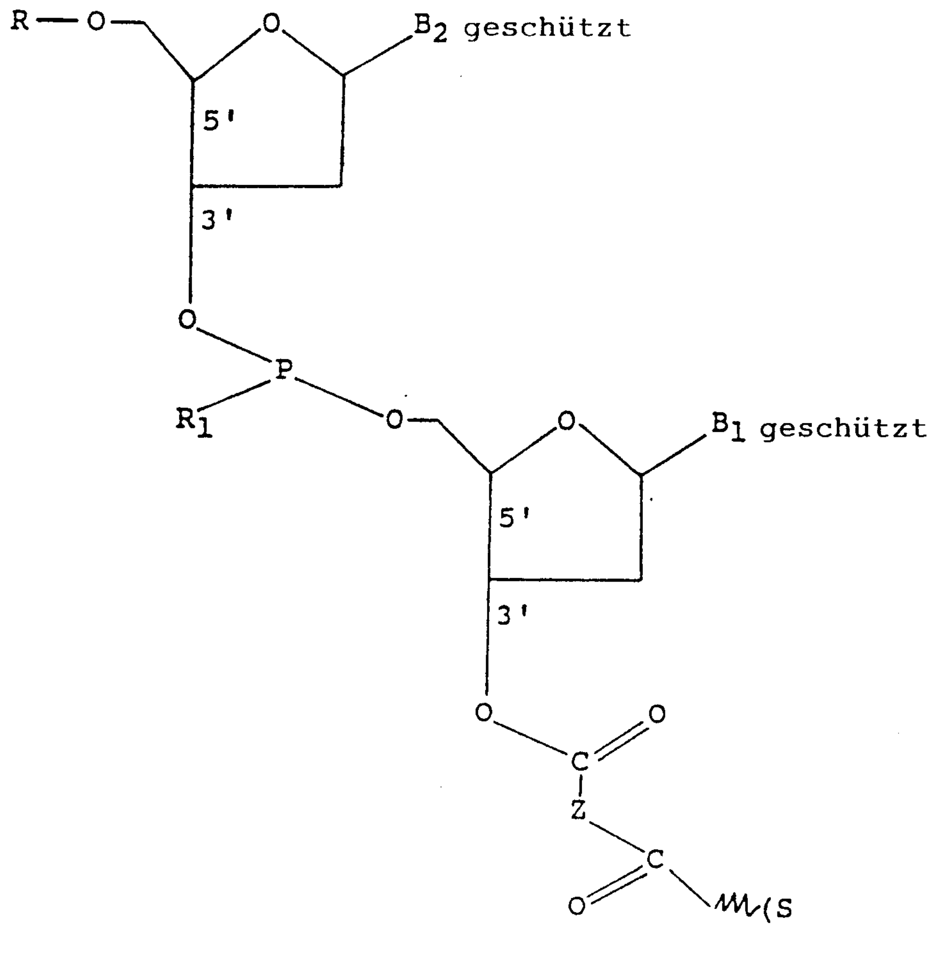

(V)

zu erhalten, worin S, R, $R_1$, $B_1$ und $B_2$ die vorstehend angegebene Bedeutung haben, dann oxidiert man das erhaltene Produkt der Formel (V), um ein Produkt der Formel

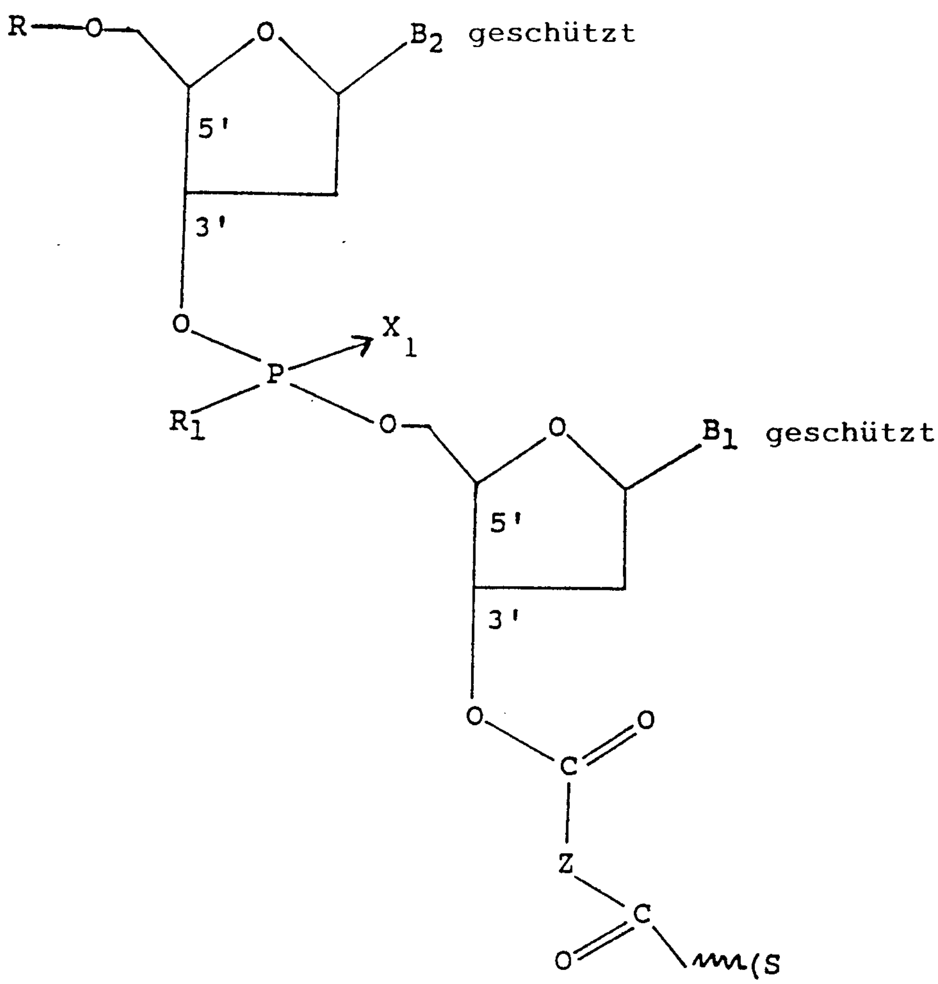

(VI)

zu erhalten, worin S, R, $R_1$, $B_1$, $B_2$ und Z die vorstehend genannte Bedeutung haben, und $X_1$ bedeutet ein Sauerstoff- oder Schwefelatom, dann bewirkt man, wie vorstehend beschrieben ausgehend vom Produkt der Formel (II), einen neuen Zyklus mit diesem Produkt der Formel (VI) und dem Monomeren eines neuen Nucleotids bis zu Erzielung der gewünschten Verkettung, um schließlich ein Produkt der Formel

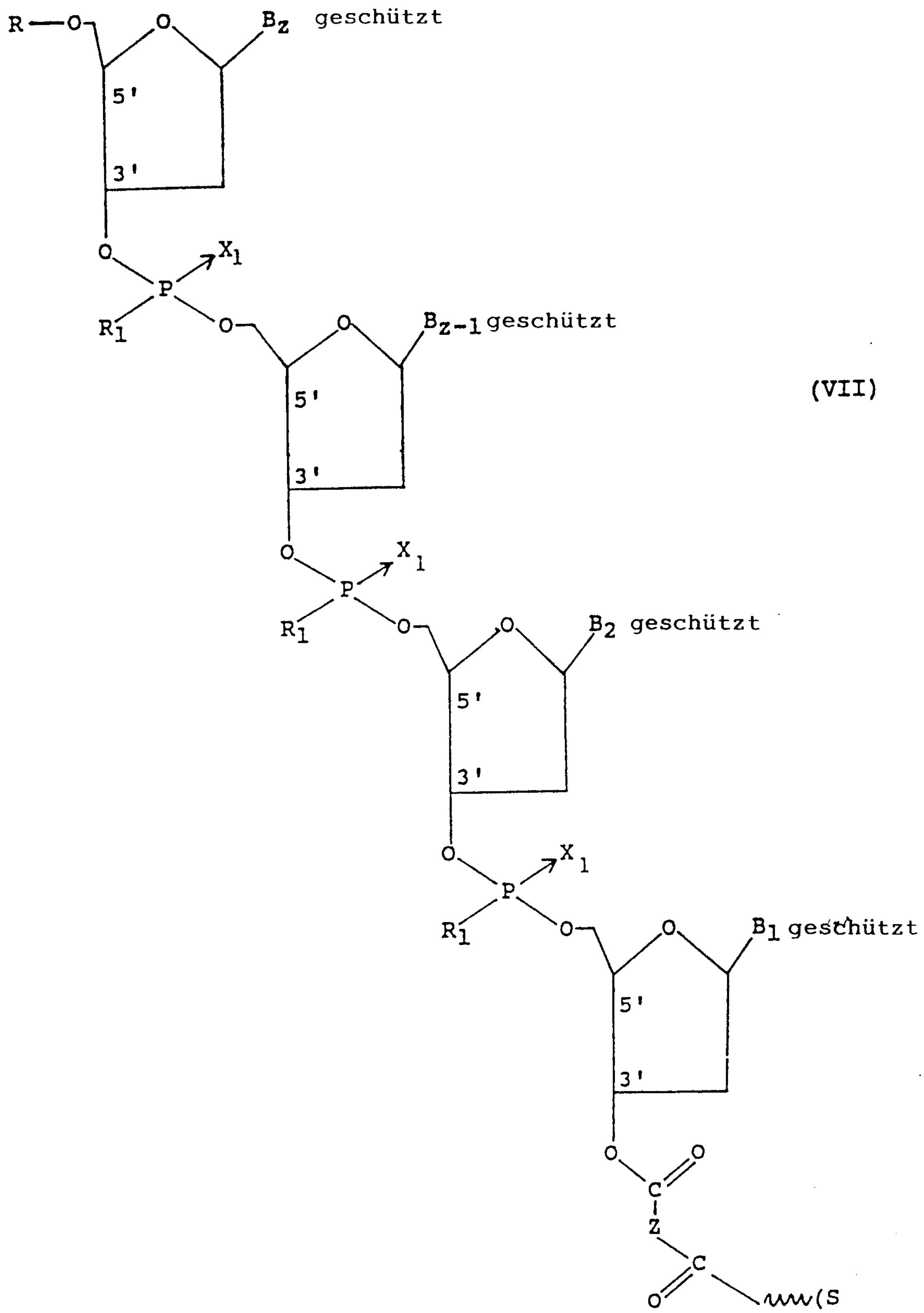

(VII)

zu erhalten, worin S, R, $R_1$, $B_1$, $B_2$, $X_1$ und Z die vorstehend angegebenen Bedeutungen haben, und $B_z$ das letzte Nucleotid der Sequenz bedeutet, dann spaltet man die Schutzgruppen des Oligonucleotids ab, trennt es vom Träger und reinigt das so erhaltene Produkt der Formel (I).

**2.** Verfahren gemäß Anspruch 1 dadurch gekennzeichnet, daß

- die Entblockierung des Hydroxyls in 5'-Stellung des Produkts der Formel (II) durch ein saures Reagens wie Essigsäure, Di- oder Trichloressigsäure bewirkt wird;
- die Kupplungsreaktion des Produkts der Formel (III) mit dem Produkt der Formel (IV) mittels Tetrazol aktiviert wird, wobei in Acetonitril gearbeitet wird;
- die Oxidation des Phosphits der Formel (V) zum Phosphat der Formel (VI) mittels Jod in Lösung in einem Gemisch Wasser/Lutidin/Tetrahydrofuran oder in einem Gemisch Wasser/Pyridin/Tetrahydrofuran bewirkt wird;
- die Oxidation des Phosphits der Formel (V) zur Erzielung der sulfurierten Form (VI) mittels Schwefel in Lösung in einem wasserfreien Gemisch von Schwefelkohlenstoff, Pyridin und Triethylamin bewirkt wird;
- am Schluß der Synthese die Schutzgruppenabspaltung der endständigen Hydroxylgruppe in 5'-Stellung mittels einer Säure wie Essigsäure, Di- oder Trichloressigsäure bewirkt wird;
- die Schutzgruppenabspaltung des Oligonucleotids der Formel (VII) mittels konzentriertem Ammoniak bewirkt wird, wobei das Reaktionsmilieu mäßig erhitzt wird.

**3.** Verfahren gemäß Anspruch 1 zur Herstellung der Derivate des Poly-L-lysins der Oligonucleotidsequenzen, wie sie durch Formel (I) definiert sind, wobei das Verfahren dadurch gekennzeichnet ist, daß man ein Produkt der Formel (VIII):

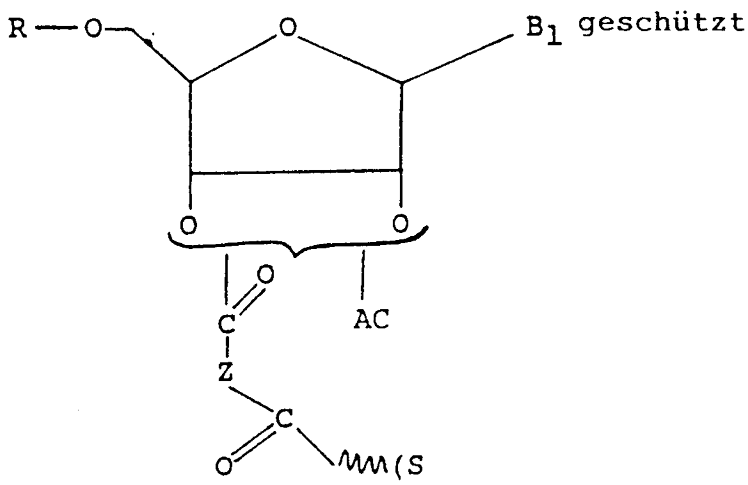

(VIII)

worin S, Z, R, $B_1$ die vorstehend angegebene Bedeutung haben, mit einem Produkt der Formel (IV) zur Reaktion bringt, um ein Produkt der Formel (IX):

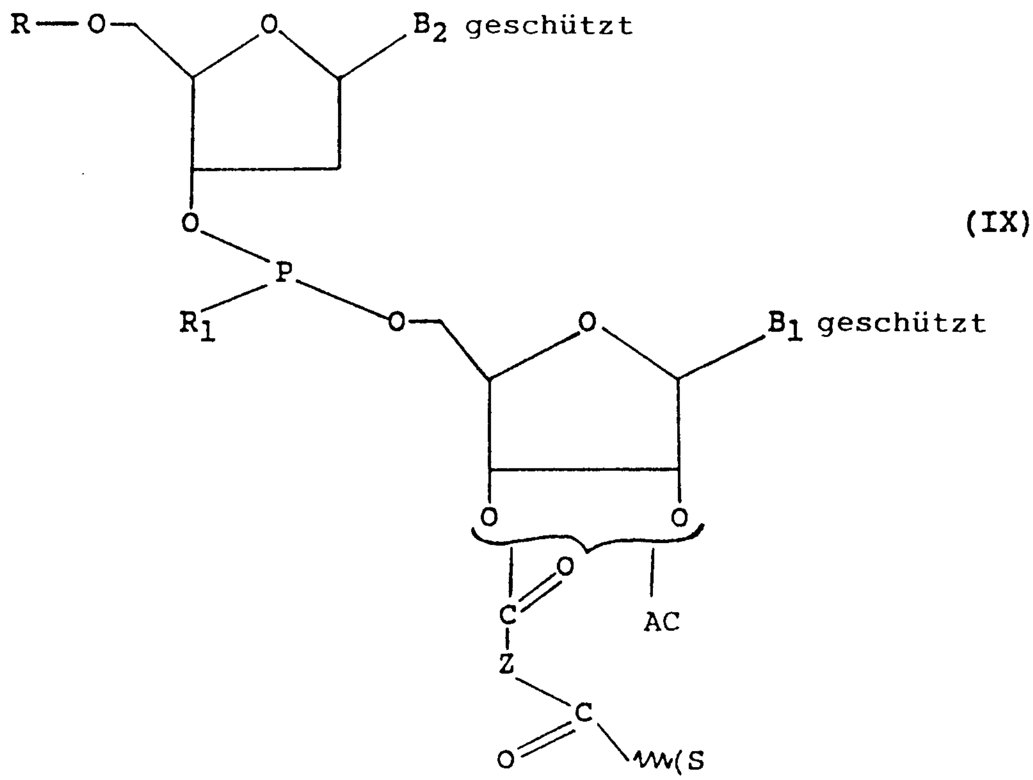

(IX)

zu erhalten, worin S, R, Z, $R_1$, $B_1$ und $B_2$ die vorstehend angegebene Bedeutung haben, man oxidiert, entfernt den Träger und die aktivierende Gruppe, um ein Produkt der Formel (X)

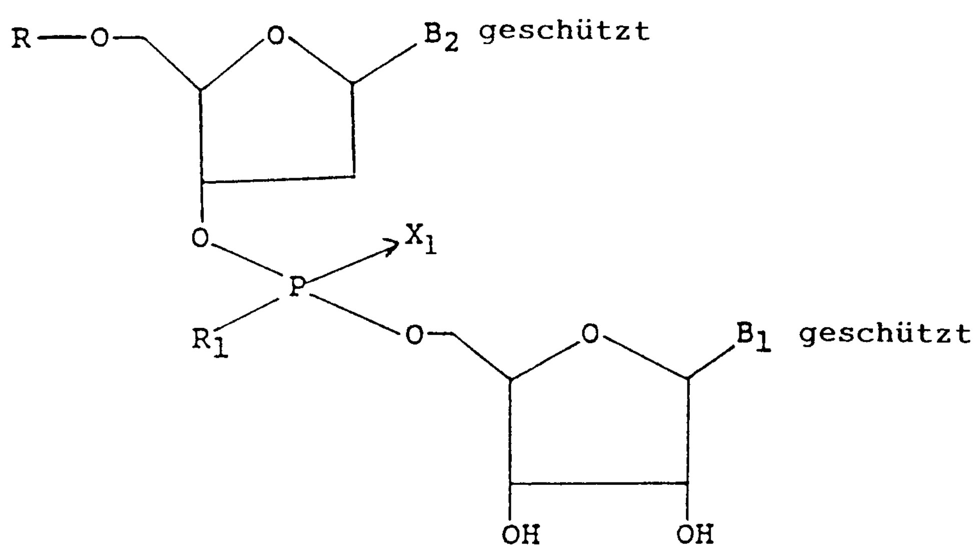

(X)

zu erhalten, worin R, $R_1$, $B_1$ und $B_2$ die vorstehend angegebene Bedeutung haben, $X_1$ bedeutet ein Sauerstoff- oder Schwefelatom, man oxidiert die endständige Ribose, dann setzt man mit L-Lysin in reduzierendem Milieu um, um das Produkt der Formel (XI)

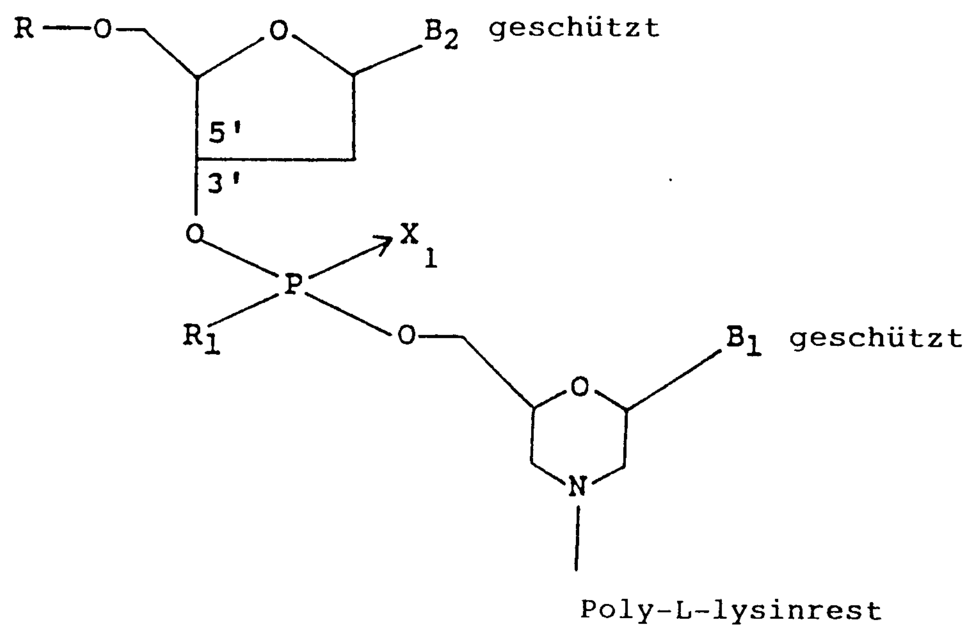

(XI)

zu erhalten, worin R, $R_1$, $B_1$, $B_2$ und $X_1$ die vorstehend angegebene Bedeutung haben, dann setzt man die Synthese wie ausgehend vom Produkt der Formel (VI) fort.

**4.** Verfahren gemäß Anspruch 1, 2 oder 3, dadurch gekennzeichnet, daß das erste Nucleotid in 3'-Stellung der zu synthetisierenden Kette entweder das erste Nukleotid der Endsequenz

3'
TCA TGG TGT CCT TTG CAG

ist oder eines der Nukleotide der Sequenz der Formel ($I_A$):
- CC TCA TGC TTT CAG TAG
ist, in freier Form, in alkylierter Form, in sulfurierter Form oder in Form des Poly-L-lysinderivats.

**5.** Verfahren gemäß einem der Ansprüche 1 bis 4, dadurch gekennzeichnet, daß das erste Nucleotid in 3'-Stellung der zu synthetisierenden Kette, das erste Nukleotid der Sequenz - CC oder der Sequenz - CC TCA TGC TTT CAG TAG ist.

**6.** Verfahren gemäß einem der Ansprüche 1 bis 4, dadurch gekennzeichnet, daß man die Oligonucleotidsequenz entsprechend der Formel

5' 3'
(d) TCA TGG TGT CCT TTG CAG CC
1 20

in freier Form, in alkylierter Form, in sulfurierter Form oder in Form des Poly-L-lysinderivats herstellt.

**7.** Verfahren gemäß einem der Ansprüche 1 bis 4, dadurch gekennzeichnet, daß man die Oligonucleotidsequenz entsprechend der Formel

```
     5'                   3'
(d)  TCA TGG TGT CCT TTG CAG
     1                    18
```

in freier Form, in alkylierter Form, in sulfurierter Form oder in Form des Poly-L-lysinderivats herstellt.

**8.** Verfahren gemäß einem der Ansprüche 1 bis 4, dadurch gekennzeichnet, daß man die Oligonucleotidseguenz entsprechend der Formel

```
     5'                                                 3'
(d)  TCA TGG TGT CCT TTG CAG CC TCA TGC TTT CAG TAG
     1                                                  35
```

in freier Form, in alkylierter Form, in sulfurierter Form oder in Form des Poly-L-lysinderivats herstellt.

**9.** Verfahren zur Herstellung von pharmazeutischen Zusammensetzungen, dadurch gekennzeichnet, daß man als Wirkstoff mindestens eine der neuen Oligonucleotidsequenzen, wie sie in Anspruch 1 definiert sind, einsetzt.

**10.** Verfahren zur Herstellung von pharmazeutischen Zusammensetzungen, dadurch gekennzeichnet, daß man als Wirkstoff mindestens eine der neuen Oligonucleotidseguenzen, wie sie in einem der Ansprüche 2 bis 8 definiert sind, einsetzt.